# EUROPÄISCHE PATENTSCHRIFT

(11) **EP 3 760 990 B1**
(45) Veröffentlichungstag und Bekanntmachung des Hinweises auf die Patenterteilung: **01.11.2023**
(21) Anmeldenummer: 20167240.9
(22) Anmeldetag: 31.03.2020
(51) Int. Cl.: G01J 3/02, G01J 3/06, G01J 3/10, G01J 3/28, G01J 3/453, A61B 5/00, G01B 9/02

(54) **VERFAHREN UND SHEAR-INVARIANTES MICHELSONTYP-INTERFEROMETER ZUR SINGLE-SHOT-IMAGING-FT-SPEKTROSKOPIE**
METHOD AND SHEAR-INVARIANT MICHELSON TYPE INTERFEROMETER FOR SINGLE-SHOT-IMAGING FT-SPECTROSCOPY
PROCÉDÉ ET INTERFÉROMÈTRE DE TYPE MICHELSON INVARIANT AU CISAILLEMENT DESTINÉS À LA SPECTROSCOPIE PAR TRANSFORMÉE DE FOURIER À L'IMAGERIE À PRISE UNIQUE

(43) Veröffentlichungstag der Anmeldung: 06.01.2021
(73) Patentinhaber: Universität Stuttgart, 70174 Stuttgart (DE)
(72) Erfinder: KÖRNER, Klaus, 10367 Berlin (DE); HERKOMMER, Alois M., 73431 Aalen (DE)
(74) Vertreter: Müller-Boré & Partner Patentanwälte PartG mbB

(56) Entgegenhaltungen:
- DE-B3-102010 006 239
- US-A- 5 131 747
- AKIAKO HIRAI ET AL: "Application of Measurement multiple-image fourier of fast phenomena transform spectral imaging to measurement of fast phenomena", OPTICAL REVIEW, SPRINGER VERLAG, TOKYO, JP , Bd. 1, Nr. 2 1. Januar 1994 (1994-01-01), Seiten 205-207, XP009523497, ISSN: 1340-6000, DOI: 10.1007/BF03254863 Gefunden im Internet: URL:http://link.springer.com/article/10.10 07/BF03254863/fulltext.html
- KUDENOV MICHAEL W ET AL: "Compact snapshot real-time imaging spectrometer", ELECTRO-OPTICAL REMOTE SENSING, PHOTONIC TECHNOLOGIES, AND APPLICATIONS V, SPIE, 1000 20TH ST. BELLINGHAM WA 98225-6705 USA, Bd. 8186, Nr. 1, 6. Oktober 2011 (2011-10-06), Seiten 1-12, XP060010218, DOI: 10.1117/12.902722 [gefunden am 1901-01-01]

## Beschreibung

Die Erfindung betrifft ein Fourier-Transformations-(FT-)Spektrometer zur Gewinnung hyperspektraler Teilbilder mittels Single-Shot Verfahren mit Michelsontyp-Interferometer und ein Verfahren zur interferometrischen Messung insbesondere für das Messen bei chaotischen Bewegungen des Objekts oder des Messgerätes und für turbulente Szenen auf Basis zumindest einer "(2n+1)"-Winkelspiegelgruppe, die in mindestens einem Arm des Michelsontyp-Interferometers angeordnet ist.

Insbesondere kann die Single-Shot-Fourier-Transformations-Spektroskopie mit einer vergleichsweise niedrigen bis zu einer moderaten spektralen Auflösung verwendet werden. Bevorzugt liegt die spektrale Auflösung bei dieser Spektroskopie in einem Bereich delta_sigma von etwa 4cm⁻¹ bis etwa 1000cm⁻¹ (1cm⁻¹ = 1/cm = reciprocal centimeters, Einheit der Wellenzahl), wobei der Spektralbereich bevorzugt vom Ultravioletten bis zum Terahertz-Bereich der Wellenlänge elektromagnetischer Strahlung adressiert wird, jedoch üblicherweise nicht in einem einzigen Instrument. Bevorzugt kann die Erfindung im visuellen Spektralbereich (VIS), im nahen Infrarot (NIR), im mittleren Infrarot (MIR), im fernen Infrarot-Bereich (FIR), im Terahertz-Bereich oder auch in kombinierten Spektralbereichen angewendet werden.

Ein Anwendungsgebiet der Erfindung kann die Untersuchung der Haut am Menschen durch einen Arzt, beispielsweise beim Hautkrebs-Screening sein. Ein weiteres Anwendungsgebiet kann bei einer chirurgischen Operation am lebenden Menschen die Untersuchung von zumindest teilweise freigelegten Organgeweben darstellen. Ein weiteres Anwendungsgebiet kann die Inspektion des Augeninneren, beispielsweise die Untersuchung der Netzhaut darstellen.

Ein anderes Anwendungsgebiet kann beispielsweise die Analyse von Lebensmitteln sein, diese insbesondere auf einem unruhig und/oder ruckelnd laufenden Transportband bewegt werden. Dazu zählt auch die Prüfung landwirtschaftlich erzeugter Lebensmittel wie beispielsweise Schüttgut-Produkte wie Getreide und Hülsenfrüchte, wo eine 100%-Prüfung am laufenden Band erforderlich ist und/oder zumindest eine hohe Stichprobenzahl genommen werden muss. Ferner kann die Erfindung bei der Fluoreszenzlicht-Analyse von Objekten und Szenen in UV-Auflicht mit Auswertung des Fluoreszenzlichts als Informationsträger angewandt werden.

Darüber hinaus kann ist das Messen in sich bewegter Objekte, wie ausbrechende Vulkane und/oder Feuerstürme bei Waldbränden, mit einer relativ gleichmäßigen Bewegung des Spektrometers beim Überflug mittels eines Helikopters und/oder eines Flugzeugs für die Applikation der Erfindung zugänglich sein.

Auch wirbelnde Partikel, beispielsweise in einer Strömung, sind mit dem erfindungsgemäßen Ansatz spektral und zumindest mit Einschränkungen ortsaufgelöst erfassbar.

Es ist auch möglich, verschiedenartige Kunststoffe im Hausmüll und/oder in Industrieabfällen anhand des Spektrums selbst in der Bewegung zu identifizieren und der Sortierung zuzuführen. Es ist auch möglich, bei der Müllsortierung an Land und/oder auch im Wasser eines Ozeans bewegte Objekte aus verschiedenartigen Kunststoffen anhand des Spektrums zu identifizieren und gegebenenfalls die Sortierung vor Ort zu ermöglichen.

Bei Messungen mit dem erfindungsgemäßen Spektrometer aus der Hand durch einen manuellen Scan über das Objekt können Objekte aus der Spurensicherung in der Kriminalistik, aus der Medizin, Hygiene, Archäologie, Botanik, Mineralogie, Landwirtschaft hyperspektral, typischerweise mit deutlich mehr als 10 Spektralkanälen pro Messpunkt und mit Ortsauflösung analysiert werden. Dabei steht in der Regel die spektrale Information im Vordergrund und nicht das perfekte hyperspektrale Bild vom Messobjekt. Dieses hyperspektrale Bild kann trotz einer aufwendigen Bildnachbearbeitung gewisse Rest-Artefakte aufweisen, was in vielen Fällen jedoch als tolerierbar gilt, da bei einer erheblichen Anzahl von Messaufgaben das Hauptinteresse der spektralen Information gilt, die weitestgehend ohne Durchschlupf erfasst werden soll.

Des Weiteren kann die Erfindung zur Wärmebildgebung im Nah- und Fernbereich eingesetzt werden. Es ist aber auch möglich, die Erfindung insbesondere im Terahertz-Bereich für Flughafen-Scanner in Sicherheitsschleusen für Personen und Transportgüter im Durchlichtverfahren mit Spektralauflösung einzusetzen.

### Stand der Technik

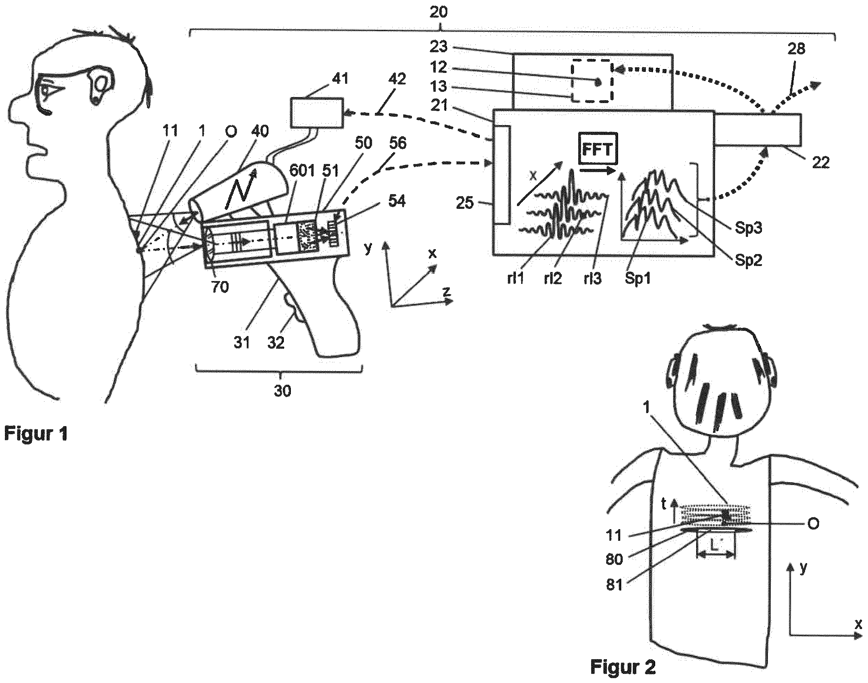

Als Stand der Technik werden hierbei nichtzyklische Zweistrahl-Interferometer-Anordnungen angesehen. Dennoch soll hier soll hier auf das Patent US 4976542 von Smith mit einem zyklischen Interferometer in Form des Sagnac-Interferometers, was eine Common-path-Anordnung darstellt, eingegangen werden. Dort ist in Figur 1 eine Sagnac-Interferometer-Anordnung mit einer Zylinderoptik 38 in Kombination mit einer Fourier-Optik 36 beschrieben. Eine Spaltöffnung 24 wird von der Zylinderoptik 38 in Spaltlängsrichtung scharf auf einem gerasterten Detektor, hier als CCD-Chip abgebildet. Die Zylinderoptik 38 in Kombination mit der Fourier-Optik 36 stellt eine anamorpotische Abbildungsstufe dar. In Spaltquerrichtung entsteht durch die Wirkung der Fourier-Optik 36 in Kombination mit dem Lateral-Shear erzeugenden Sagnac-Interferometer eine Vielzahl von räumlichen Interferogrammen auf dem CCD-Chip. So besteht die Möglichkeit, im Single-Shot-Modus räumliche Interferogramme zu detektieren und zu einem hyperspektralen Teilbild in Streifenform, entsprechend dem Spalt, zu verrechnen. Der Spalt, der hier also als Bildfeldblende oder als Bildfeld-Diskriminator wirkt, ist jedoch vor dem Sagnac-Interferometers angeordnet. Das reduziert den Öffnungswinkel des Strahlenbündels im Sagnac-Interferometer aufgrund der hier vergleichsweise langen optische Wege ganz erheblich.

AKIAKO HIRAI ET AL, "Application of Measurement multiple-image fourier of fast phenomena transform spectral imaging to measurement of fast phenomena", vol. 1, no. 2, doi:10.1007/BF03254863, ISSN 1340-6000, (19940101), pages 205 - 207, OPTICAL REVIEW, SPRINGER VERLAG, TOKYO, JP, URL: http://link.springer.com/article/10.1007/BF03254863/fulltext.html, XP009523497 *[Y] 1-15* offenbart ein neues interferometrisches Verfahren, das gleichzeitig alle optischen Daten erfasst, die zur Rekonstruktion eines Spektralbildes erforderlich sind. Die optischen Daten sind ein zweidimensionales Array zweidimensionaler Interferenzmuster. Die Daten werden von einem optischen System erfasst, das aus einer zweidimensionalen Linsenanordnung und einem Michelson-Interferometer besteht. Die Interferenzmuster sind im Allgemeinen durch die Fehler der Wegunterschiede verzerrt. Diese Fehler können vorher abgeschätzt und bei der Rekonstruktion des Spektralbildes korrigiert werden.

KUDENOV MICHAEL W ET AL, "Compact snapshot real-time imaging spectrometer", ELECTRO-OPTICAL REMOTE SENSING, PHOTONIC TECHNOLOGIES, AND APPLICATIONS V, SPIE, 1000 20TH ST. BELLINGHAM WA 98225-6705 USA, (20111006), vol. 8186, no. 1, doi:10.1117/12.902722, pages 1 - 12, XP060010218 *[Y] 1-15* ^{∗} *page 1 - page* 4 offenbart ein Spektral-Imaging-System, das als Snapshot-Hyperspectral-Imaging-Fourier-Transformation (SHIFT)-Spektrometer bezeichnet wird und in der Lage ist, spektrale Bilddaten einer Szene in einer einzigen Integration einer Kamera zu erfassen, ist ultrakompakt, kostengünstig (handelsüblich), hat keine beweglichen Teile und kann Datenwürfel (x, y, *λ* ) in Echtzeit erzeugen.

Im Patent US 5777736 von Horton ist ein Interferometer vom Mach-Zehnder-Typ beschrieben, welches vergleichsweise lange optische Wege im Interferometer im Vergleich zu einem Michelson-Interferometer aufweist. Als Interferometer vom Mach-Zehnder-Typ ist es grundsätzlich schwerer zu justieren - im Vergleich zu einem Michelsontyp-Interferometer, welches nur einen einzigen Strahlteiler aufweist. Diese Schwierigkeit besteht auch aufgrund der Anordnung von zwei Strahlteilern im Mach-Zehnder-Interferometer. Demzufolge weist ein Interferometer vom Mach-Zehnder-Typ vergleichsweise keine besonders hohe Langzeitstabilität ohne erheblichen technischen Aufwand auf und ist nur bedingt kompakt aufzubauen. In Figur 22 dieses Patents besteht ein starker Astigmatismus, bedingt durch den jeweils einmaligen Durchgang durch eine geneigte Strahlteilerplatte, der im interferometrischen Strahlengang nicht kompensiert wird. Das kann Nichtlinearitäten im räumlichen Interferogramm mit sich bringen, welche die Spektrenberechnung erheblich erschweren, bzw. zu sehr unerwünschten Artefakten im berechneten Spektrum führen können. Es entsteht das Vollbild des Messobjekts, der Szene oder der Lichtquelle auf dem Detektor, überlagert von einem einzigen räumlichen Interferogramm, da als Relay-Optik ein Sammelobjektiv, dort als "exit lens" bezeichnet, angeordnet ist. So ist nur eine serielle Interferogramm-Gewinnung mittels einer Relativbewegung zwischen Interferometer und Messobjekt und kein Single-Shot-Betrieb möglich, da eine gleichmäßige Bewegung der Szene bei der Interferogramm-Gewinnung erforderlich ist und das Interferogramm stets seriell entsteht und erst aus einem Bilderstapel für jeden Messpunkt extrahiert werden muss. Weiterhin sind in der Schrift US 5777736 keine Bildfeld-Diskriminatoren im Interferometer in der Bildposition angeordnet.

Im Patent US3684379A ist von Girard ein kompaktes Michelsontyp-Interferometer mit Keilinterferenzen für den Feldeinsatz beschrieben. Das Bild entsteht auf den beiden Planspiegeln eines Michelson-Interferometers. Es gibt jedoch keinen Bildfeld-Diskriminator im Interferometer. Da es sich um geöffnete Bündel handelt, kann der hier eingeführte Keil unerwünschte Wellenfront-Aberrationen erzeugen, welche ein räumliches Interferogramm erheblich stören können. Dies kann bei der Auswertung durch eine Fourier-Transformation erhebliche Probleme bereiteten und zu nicht akzeptablen Fehlern im Spektrum führen.

Im Patent US 4523846 von Breckingridge ist ebenfalls ein sehr kompaktes Michelsontyp-Interferometer in monolithischer Bauweise mit einer nichtrechtwinkligen Anordnung eines Planspiegels als Interferometer-Endspiegel beschrieben. So entsteht die Interferenz zueinander verkippter Wellenfronten. Auch hier kann der in das Interferometer eingeführte Keil aus refraktivem Material Wellenfront-Aberrationen erzeugen, welche ein räumliches Interferogramm erheblich stören können. Ein Spalt zur Diskriminierung des Bildfeldes, der als effektive Quelle wirkt, befindet sich hierbei am Eingang des Interferometers, also außerhalb desselben. In einer Position des Instruments wird offensichtlich nur das räumliche Interferogramm des gesamten Schlitzes gewonnen, welches in der Pupillenebene detektiert wird. Innerhalb des länglichen Schlitzes gibt es also offenbar keine Ortsauflösung. Die konvexe Fläche mit Spaltblende am Interferometereingang stellt eine Feldlinse dar, steht also am Bildort oder näherungsweise in einer Zwischenbildebene. So kann bei einer Relativbewegung zwischen Interferometer und Messobjekt, hier beispielsweise im Satellitenüberflug einer Landschaft, aus den sequenziell detektierten räumlichen Interferogrammen ein eindimensionales Hyperspektralbild nach dem Pushbroom-Prinzip erstellt werden. Dabei liefert zu einem Zeitpunkt offensichtlich stets nur ein einziger, hier streifenförmiger Bildausschnitt, ein räumliches Interferogramm. Aufgrund der Anordnung des Spaltes am Eingang des Interferometers ist der Öffnungswinkel für das Bündel aufgrund der optischen Weglänge vom Eingang bis zum Ausgang des Interferometers etwas begrenzt. Jedoch kann noch ein halber Öffnungswinkel von gut 10° erreicht werden. Demzufolge kann die Lichtausbeute durch den limitierten Öffnungswinkel jedoch etwas begrenzt sein. Dies kann zu einem nichtoptimalen Signal-Rausch-Verhältnis im gemessenen Interferogramm und somit auch im errechneten Spektrum führen.

Die Lateral-Shear kann in einer optischen Anordnung als Grundlage zur Generierung von Interferenzen zueinander geneigter Wellenfronten genutzt werden. Einen ganz klassischen Ansatz stellt dafür eine Michelson-Interferometer-Anordnung mit zwei Dachkant-Reflektoren dar, um die erforderliche Lateral-Shear zu erzeugen. Dieser Ansatz mit zwei Dachkant-Reflektoren wird in der Regel auch zur Wellenfrontanalyse eingesetzt und ist den Fachleuten gut bekannt, siehe dazu auch Malacara, Optical Shop Testing, John Wiley & Sons, Inc., 1992, S. 140 -141, Figur 4.16 [1] und auch Steel, Interferometry, Cambridge University Press, 1967, S. 83 letzter Absatz bis S. 84 oben [2].

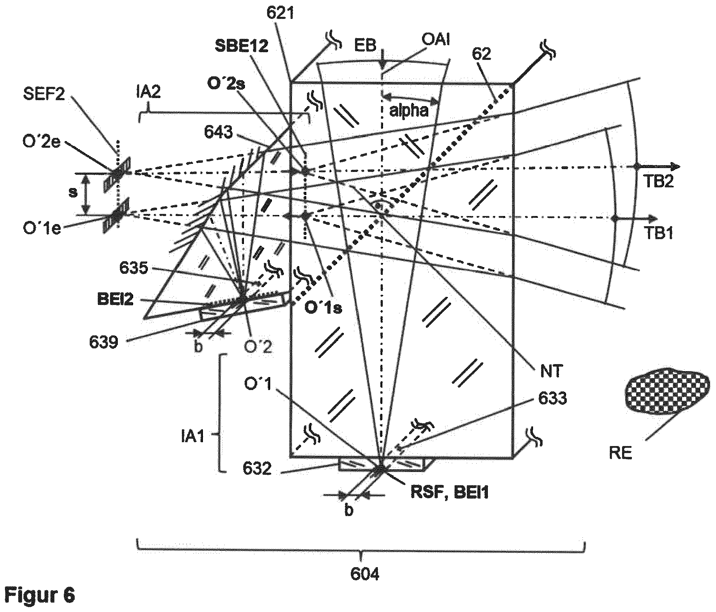

Bekannt ist auch der von Kelsall im Jahr 1959 in Proc. Phys. Society, 73, S. 470, Fig. 1 [3] veröffentlichte Ansatz mit zwei Tripelreflektoren als Endreflektoren eines Michelson-Interferometers. Die laterale Verschiebung eines Tripelreflektors erzeugt ebenfalls eine Lateral-Shear zwischen Objekt- und Referenzwellenfronten am Ausgang eines Michelson-Interferometers. Der Einsatz eines Tripelreflektors im Referenzstrahlengang eines Michelson-Interferometers geht nach bestem Wissen jedoch bereits auf Twyman und Green zurück, s. dazu auch das US-Patent 1 565 533 und dort Figur 6.

In der Schrift US 5131747 und DE 68906154T2 wird von Cerutti-Maori ein hyperspektrales Verfahren mit einem in sich starren Michelson-Interferometer mit einer Doppel-Dachkant-Anordnung beschrieben. Dabei erfährt das Michelson-Interferometer beim Überflug mittels eines Fluggerätes über eine Bodenoberfläche in der Regel eine sehr konstante Bewegung. Dabei entsteht das Bild von der Bodenoberfläche auf dem gerasterten Detektor. Das Interferogramm mit Bildinformation ist durch zueinander verkippte Wellenfronten auf dem gerasterten Detektor gebildet. Durch die konstante Bewegung kann in jedem Pixel des gerasterten Detektors das Signal eines Zweistrahl-Interferogramms im Vorbeiziehen desselben durch eine Synchronisierung der Systemkomponenten zeitseriell aufgezeichnet werden. Mit diesem Verfahren ist jedoch die Single-Shot-Erfassung eines Interferogramms überhaupt nicht möglich. Bei einem unruhig über das Messobjekt bewegtem Interferometer oder bei Objekten mit chaotischer Relativbewegung oder bei turbulenten Szenen wie blubberndes Magma oder ein Feuerwerk kann mit diesem Ansatz kein ungestörtes Interferogramm gewonnen werden. In der Schrift CN 106338342 A von Dou Jianyun und anderen wird ein hyperspektrales Verfahren mit einer Doppel-Dachkant-Anordnung im Michelson-Interferometer für Objekte in Abtastung mit einem rotierenden Scannerspiegel beschrieben. Es entsteht das volle Bild einer statischen Szene auf dem gerasterten flächigen Detektor in der Fourier-Ebene einer Optik. Das Bild ist aufgrund der mittels Interferometer eingeführten Lateral-Shear von einem räumlichen Interferogramm überlagert. Jedoch wird dieses Interferogramm, welches einen Objektpunkt zuzuordnen ist, auch erst zeitseriell im Überflug der Szene gewonnen. Voraussetzung ist also, dass dieser Überflug wie auch die Rotation des Scannerspiegels sehr gleichmäßig sein müssen, da sonst gestörte Interferogramm-Signale entstehen. Somit ist dieses hyperspektrale Verfahren überhaupt nicht für turbulente Szenen, oder gar Objekte mit chaotischer Eigenbewegung oder für Hand-held-Geräte beim Betrieb aus einer vergleichsweise unruhigen oder zitternden Hand geeignet.

Im Fachartikel "Large field-of-view Fourier transform imaging spectrometer using dualchannel stitching" von Chengmiao Liu und anderen in OPTICS EXPRESS, Vol. 24, No. 25 vom 12. Dezember 2016, S. 28473- 28490, http://dx.doi.org/10.1364/OE.24.028473, [4] ist in Figur 1 ein Lateral-Shear-Interferometer mit Dachkantreflektoren dargestellt. Das räumliche Interferogramm entsteht in der Fourier-Ebene eines nachgeordneten Objektivs mit Bildinformation. Dieses System arbeitet für die Gewinnung des Interferogramm-Signals jedoch zeitseriell und ist somit für Single-Shot-Applikationen nicht geeignet.

Mit anderen Worten: Ein spektrales Messverfahren, basierend auf den Schriften US 5131747, CN 106338342 A oder auch [4], liefert zwar stets ein volles Bild des Messobjekts oder der Szene, aber die Gewinnung eines räumlichen Interferogramms erfolgt zeitseriell. Dies setzt also eine nahezu konstante Relativbewegung zwischen Interferometer und Messobjekt voraus, wie es bei einem ruhigen Überflug einer Landschaft durch einen Satelliten oder ein Flugzeug in der Regel gegeben ist. Dieser Ansatz ist somit eher nicht für turbulente Szenen geeignet, da die Interferogramm-Signale durch chaotische Bewegungen in der Szene - wie bei einem Auto-Crashtest - oder bei ungleichmäßigen oder gar chaotischen Bewegungen des Interferometers beim Halten des Instruments mittels einer freien Hand sehr wahrscheinlich gestört und für die übliche Auswertung mittels Fourier-Transformation völlig ungeeignet sind.

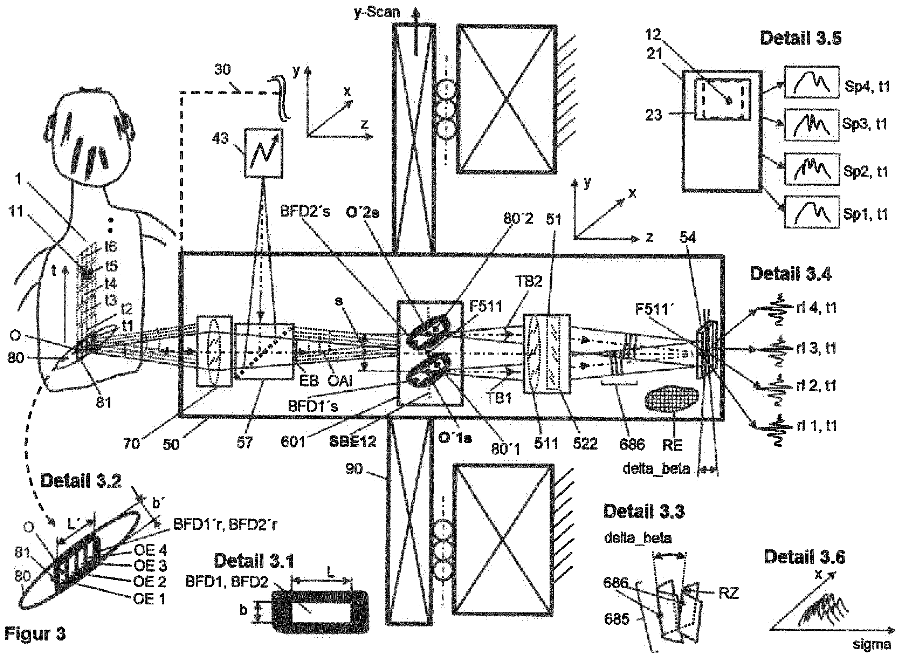

In den Familienpatenten DE 102010006239 B3 sowie EP 2526373B1 und US 8934104 B2 von K. Körner, R. Berger und W. Osten ist jeweils ein Michelsontyp-Interferometer beschrieben, bei dem eine konstante Lateral-Shear durch Verwendung eines speziellen Dreifachspiegels im Interferometer erreicht werden kann, siehe Figur 2. Bei diesem Interferometer stehen die drei Planspiegel des Dreifachspiegels senkrecht zu einer gemeinsamen Referenzebene, zu welcher auch die Strahlteilerfläche senkrecht steht. Der dort beschriebene spezielle Dreifachspiegel erzeugt eine invariante Lateral-Shear. Die Invarianz der Lateral-Shear bei einer Querverschiebung v des speziellen Dreifachspiegels ist in den oben genannten Familienpatenten in Figur 3 dargestellt.

Die in den oben genannten Familienpatenten dargestellte optische Anordnung mit einer speziellen Dreifachreflexion in einem Arm eines Michelsontyp-Interferometers, bei welcher die drei Spiegelflächen jeweils senkrecht zu einer Bezugs- oder Referenzebene stehen, ist für die Erfassung von Abstand, Tiefe, Profil, Form Welligkeit und/oder Rauheit von technischen oder biologischen Objekten ausgelegt. Die Bezugs- oder Referenzebene ist senkrecht zur Strahlteilerfläche des Michelsontyp-Interferometers. Diese dort präsentierte optische Anordnung ist für die Imaging FT-Spektroskopie jedoch völlig ungeeignet. Denn hierbei ist das Messobjekt im Strahlengang des Interferometers angeordnet und ist somit selbst Teil des Michelsontyp-Interferometers. Eine derartige Objektanordnung ist für die Spektroskopie in der Regel schon deshalb ungeeignet, da durch den "interferometric gain" des Interferometers recht stark verfälschte Spektren vom Messobjekt gewonnen werden, die nur bedingt numerisch korrigiert werden können.

Im genannten Stand der Technik ist es in der Regel vergleichsweise einfach, ein verwackeltes Bild aus nicht äquidistanten Bildpunkten zusammenzusetzen, verglichen mit der Gewinnung eines fehlerarmen Spektrums aus einem gestörten Interferogramm, insbesondere für den Fall dass die Art und die Stärke der Störung im Interferometer weitgehend unbekannt sind, was häufig der Fall ist.

Die der Erfindung zugrundeliegende Aufgabe besteht gemäß einem Aspekt darin, einen FT-Spektrometer in einem Single-Shot-Modus zu betreiben. Insbesondere liegt die Aufgabe darin, von einzelnen selektierten Bereichen des Bildes vom zu detektierenden Objekt zeitgleich mindestens zwei räumliche Interferogramme zu erzeugen, bevorzugt jedoch eine größere Anzahl von räumlichen Interferogrammen, welche die Information über das gesuchte Spektrum zumindest teilweise enthalten und so die Erfassung von zwei oder mehreren in der Regel vollständigen räumlichen Interferogrammen in einer einzigen Aufnahme durchzuführen. Gemäß einem Aspekt umfasst ein Fourier-Transformations-Spektrometer, FT-Spektrometer die Merkmale aus Anspruch 1.

Erfindungsgemäß wird eine (2n+1)-Winkelspiegelgruppe insbesondere eine Dreifach-Winkelspiegelgruppe mit n = 1 oder eine andere (2n+1)-Winkelspiegelgruppe mit n = 2, 3, 4 als Endreflektor in einem Michelsontyp-Interferometer zur Erzeugung einer Lateral-Shear s für die Fourier-Spektroskopie verwendet.

In anderen Worten ist ein Fourier-Transformations-Spektrometer für die zumindest teilweise hyperspektrale Bildgebung von einem Messobjekt als Ergebnis einer Berechnung mit einem Rechnersystem zur Errechnung von Spektren mittels Fourier-Transformation ausgelegt, wobei das Fourier-Transformations-Spektrometer ein dem Michelsontyp-Interferometer vorgeordneten Objektiv aufweist, genutzt als Abbildungssystem für das Messobjekt, mit der optischen Achse OAI zur Erzeugung mindestens eines fokussierten Eingangsstrahlenbündels für das Michelsontyp-Interferometer ausgebildet ist und wobei das Michelsontyp-Interferometer ferner aufweist:
Einen Strahlteiler mit ebener Strahlteilerfläche wobei der Strahlteiler sowohl zur Strahlenbündelteilung genutzt wird, wodurch zwei Teilstrahlenbündel gebildet werden, als auch zur zumindest teilweisen Strahlenbündelvereinigung mit einer Lateral-Shear s zwischen den beiden Teilstrahlenbündeln, und wobei am Michelsontyp-Interferometer insbesondere eine Referenzebene bestehen kann, welche durch die Normale der ebenen Strahlteilerfläche und durch die optische Achse des vorgeordneten Objektivs am Eingang des Michelsontyp-Interferometers aufgespannt ist;
bevorzugt mindestens eine Lichtquelle;
einen gerasterten Detektor, wobei mindestens ein räumliches Interferogramm auf dem gerasterten Detektor ausgebildet werden kann,
wobei insbesondere entweder ein zumindest näherungsweise planer Endspiegel oder eine plane Endspiegelfläche im ersten Interferometerarm des Michelsontyp-Interferometers angeordnet sein kann und im zweiten Interferometerarm eine Dreifach-Winkelspiegelgruppe als Endreflektor angeordnet sein kann und die Dreifach-Winkelspiegelgruppe aus einer Anordnung von - in der Summe - drei zumindest näherungsweise planen Spiegeln oder zumindest näherungsweise planen Spiegelflächen in Rachen- oder W-Form und jeweils mit Winkel zueinander bestehen kann, welche im Wesentlichen senkrecht zur Referenzebene ausgerichtet sind, oder
wobei in jedem der beiden Interferometerarme des Michelsontyp-Interferometers jeweils eine Dreifach-Winkelspiegelgruppe als Endreflektor angeordnet sein kann, also
wobei in mindestens einem der beiden Interferometerarme des Michelsontyp-Interferometers als Endreflektor eine (2n+1)-fach-Winkelspiegelgruppe mit n = 2, 3, 4 angeordnet ist, die aus einer Anordnung von - in der Summe (2n+1) planen Spiegeln oder planen Spiegelflächen vorzugsweise in Rachen- oder W-Form oder in einer Mischform und jeweils mit Winkel zueinander besteht, welche im Wesentlichen senkrecht zu einer gemeinsamen Referenzebene ausgerichtet sind, und
wobei in allen Fällen die Gesamtzahl der Spiegel oder Spiegelflächen im Michelsontyp-Interferometer mindestens vier beträgt und - auch bei einer größeren Gesamtzahl als vier - in der Regel geradzahlig ist; und
wobei in allen Fällen an jedem Spiegel oder an jeder Spiegelfläche im Michelsontyp-Interferometer die Strahlen eines Teilstrahlenbündels beim Passieren eines Interferometerarms nur jeweils eine einmalige Reflexion erfahren.

In anderen Worten kann bei einem Single-Shot-Imaging-FT-Spektrometer, insbesondere für das Messen mit chaotischen Relativbewegungen eines Messobjekts und/oder für turbulente Szenen, mit zumindest teilweiser hyperspektraler Bildgebung ein Shear-invariantes Zweistrahl-Interferometer, welches als ein Michelsontyp-Interferometer, ausgebildet ist, mit einem gerasterten Detektor eingesetzt werden. Bei diesem Interferometer werden am Ausgang zwei kohärente Teilstrahlenbündel TB1 und TB2 mit einer Lateral-Shear s gebildet, indem in mindestens einem der beiden Arme des Interferometers als Endreflektor eine in sich bevorzugt starre Winkelspiegelgruppe mit einer ungeradzahligen Anzahl von Planspiegeln, mindestens jedoch bevorzugt drei, zur Erzeugung der Lateral-Shear angeordnet ist. Die Planspiegel sind sowohl mit Winkeln zueinander als auch stets im Wesentlichen senkrecht zu einer gemeinsamen Referenzebene in Rachen- oder W-Form ausgerichtet. Die Gesamtzahl der Spiegel oder Spiegelflächen in den beiden Armen des Michelsontyp-Interferometers beträgt mindestens vier und ist stets geradzahlig. Es wird insbesondere ein Paar kohärenter Bilder oder Teilbilder des Messobjekts oder der Szene im Interferometer gebildet und diese Bilder oder Teilbilder erfahren eine Bildfeld-Diskriminierung bevor mittels anamorphotischer Abbildung des selektierten Bildfeldes räumliche Interferogramme gebildet werden, aus denen Spektren mittels einer schnellen Fourier-Transformation errechnet werden.

Es handelt sich insbesondere um ein FT-Spektrometer, das zusätzlich folgendes aufweist:
- Ein Zweistrahl-Interferometer mit einem ersten und einem zweiten Arm, wobei das Zweistrahl-Interferometer einem Michelsontyp-Interferometer entspricht, umfassend:
   - zumindest eine Strahlteilereinheit mit einer planen Strahlteilerschicht, wobei die Strahlteilereinheit und insbesondere die Strahlteilerschicht ausgelegt ist
      ▪ zum Aufspalten eines einfallenden Lichtbündels eines räumlich ausgedehnten Objekts, in ein erstes Teilstrahlenbündel in den ersten Arm des Michelsontyp-Interferometers mittels Transmission durch die Strahlteilerschicht und ein zweites Teilstrahlenbündel in einen zweiten Arm des Michelsontyp-Interferometers mittels Reflexion an der Strahlteilerfläche; und
      ▪ zum zumindest teilweisen Überlagern des ersten Teilstrahlenbündels und des zweiten Teilstrahlenbündels mit einer Lateral-Shear durch Transmission des zweiten Teilstrahlenbündels durch die Strahlteilerschicht und durch Reflexion des ersten Teilstrahlenbündels an der Strahlenteilerschicht.

Dabei diesem erfindungsgemäßen Michelsontyp-Interferometer die Lateral-Shear invariant ist, ist eine hohe Wellenzahlgenauigkeit, insbesondere nach einer Kalibrierung erreichbar und eine hohe Wellenzahl-Konstanz kann erreicht werden. Dies ist insbesondere für eine hohe spektrale Auflösung sinnvoll. Bei feinen Bildfeld-Diskriminatoren, die fein aufgelöste hyperspektrale Bilder ermöglichen, besteht unter Ausschluss extremer Bedingungen keine Gefahr einer Dejustierung des Interferometers.

Hinsichtlich des der Strahlteilereinheit vorgeordneten Objektivs besteht insbesondere ein Vorteil darin, Licht mit einem vergleichsweise großen Raumwinkel von einem Messort des Messobjekts oder der Szene erfassen zu können und mittels eines Zweistrahl-Interferometers als Interferenzlicht zur Detektion bringen zu können. Dies kann ermöglichen, dass am Interferometerausgang ein nachgeordnetes Objektiv zur Erfassung dieses Interferenzlichts mit einem möglichst großen Aperturwinkel alpha eingesetzt werden kann, um einen möglichst großen Anteil der erfassten Lichtenergie für die Detektion nutzbar zu machen. Dies ermöglicht auch, vergleichsweise kurze Integrationszeiten eines gerasterten Detektors, um Single-Shot-Messungen auch an bewegten Objekten und bei turbulenten Szenen durchführen zu können.

Bei aktiver Beleuchtung mit einer Lichtquelle kann die Energie derselben optimal genutzt werden. Das gesamte Licht derselben kann beispielsweise möglichst und im Wesentlichen vollständig auf ein schmales Objektfeld gelenkt werden. Dann stellt es ggf. unter dem Gesichtspunkt der Energienutzung im Wesentlichen keinen Nachteil dar, wenn nur ein kleines, schmales Feld oder ein Objektausschnitt bei einer Single-Shot-Messung erfasst wird, da ggf. auch nur dieses ausgeleuchtet werden muss.

Insbesondere kann eine hohe Robustheit durch die Reduzierung einer sehr unerwünschten Dejustierung des Interferometers erreicht werden. Dies kann insbesondere dann erfolgen, wenn das Interferometer im Wesentlichen miniaturisiert vorliegt, insbesondere wenn die Strahlteilereinheit und die Endreflektoren des Interferometers miniaturisiert vorliegen, da ggf. z.B. auch Folienstrahlteiler oder Pellikel-Strahlteiler eingesetzt werden sollen. Diese sind bei großer Ausdehnung relativ vibrationsempfindlich. Weiterhin besteht ein Vorteil darin, in den Armen des Interferometers besonders kurze Wege zu verwirklichen zu können.

Darüber hinaus können die beiden Teilstrahlenbündel, welche das Interferometer verlassen, möglichst frei von Astigmatismus erzeugt werden.

Weiterhin ist es ein Vorteil, dass bei Bedarf auch eine Adaptierbarkeit an das Objekt - wenn sich ein Bereich desselben von besonderem Interesse erweist - sowie auch eine Durchmusterung des Messobjekts ermöglicht werden kann. Dabei kann auch eine Flexibilität bei Wahl der lateralen Auflösung im Bild des Messobjekts in weiten Grenzen möglich sein.

Es besteht ein Vorteil insbesondere darin, dass auch bei Vibrationen in der Umgebung der Messanordnung oder bei Turbulenzen in einer zu vermessenden Szene, also z.B. im Feldeinsatz, oder auch bei Hand-held-Instrumenten in der Regel weitgehend unverfälschte Spektren auf der Grundlage der Berechnung mittels einer Fourier-Transformation von möglichst ungestörten räumlichen Interferogrammen erhalten werden können. Dabei werden bei turbulenten Szenen oder bei Messungen aus der Hand ggf. jedoch gewisse Fehler bei der bildlichen Abtastung des Messobjekts oder der Szene, also im lateralen Aufbau des Bildes mit den zugeordneten Spektren, dem Spektralbild, in Kauf genommen. Ein Spektralbild ist auch als hyperspektrales Bild bekannt, welches in der Regel in Form des bekannten Datenkubus (x, y, sigma) mit der Wellenzahl sigma oder (x, y, lambda) mit der Wellenlänge lambda vorliegt.

Ein besonderer Vorteil der Erfindung besteht für die Gewinnung räumlicher Interferogramme im Single-Shot-Modus mittels Zweistrahl-Interferometer mit einer im Vergleich zum Sagnac-Interferometer, auch als zyklisches Interferometer bekannt, geringeren optischen Weglänge im Interferometer. So können Strahlenbündel mit einem größeren Öffnungswinkel als beim Sagnac-Interferometer zum Einsatz kommen. Es ist die Möglichkeit einer erheblichen Miniaturisierung des Interferometers aufgrund der Strahlführung im erfinderischen optischen Setup gegeben. Dazu werden optische Invarianzen genutzt, hierbei eine invariante Lateral-Shear bei einer speziellen Spiegelanordnung. Diese invariante Lateral-Shear ermöglicht das Erreichen einer sehr hohen Wellenzahlgenauigkeit, die somit auch eine hohe spektrale Auflösung sinnvoll macht.

Es kann insbesondere während der Signalaufnahme auch keine im Wesentlichen bewegten und sondern im Wesentlichen (in sich) starre Komponenten im gesamten Spektrometer geben. Dies hat den Vorteil, dass das FT-Spektrometer ggf. eine relativ hohe Robustheit aufweist.

Durch Mitlaufen einer vergleichsweise simplen Monitor-Kamera, bevorzugt angeordnet am Fourier-Transformations-Spektrometer und zur gleichzeitigen Bildaufnahme des Objekts bei der Messung mit dem FT-Spektrometer, ist das Zusammensetzen etwas oder nur mäßig verwackelter Bildserien aus der Spektrometer-Messung mittels Bildnachverarbeitung vergleichsweise einfach - im Vergleich zur Gewinnung eines fehlerarmen Spektrums aus einem gestörten Interferogramm. Diese Monitorkamera kann einerseits bevorzugt für den sichtbaren Spektralbereich ausgelegt sein, andererseits aber auch für den nahinfraroten Spektralbereich ausgelegt sein.

Der Single-Shot-Ansatz ermöglicht ggf. auch den Einsatz von vergleichsweise kostengünstigen und dabei sehr starken Lichtquellen, die hinsichtlich der Ausgangsleistung zeitlich im Wesentlichen nicht sehr stabil sind, also Schwankungen der Ausgangsleistung von durchaus 10% und mehr aufweisen können. Schwankungen der Ausgangsleistung können bei seriellen Verfahren zur Aufnahme von Interferogrammen ggf. ein Problem darstellen, da so eine sehr unerwünschte Modulation im ermittelten Interferogramm entstehen kann.

Bevorzugt ist die Referenzebene durch die Normale der Strahlteilereinheit und durch die optische Achse des vorgeordneten Objektivs aufgespannt.

Bevorzugt entspricht die (2n+1)-Winkelspiegelgruppe einer Rachen- oder einer W-Form.

Bevorzugt ist jede der Spiegelflächen dazu angeordnet, das erste Teilstrahlenbündel oder das zweite Teilstrahlenbündel ein einziges Mal zu reflektieren.

Insbesondere ist mindestens eine aus der ersten Strahlumlenkungseinheit und der zweiten Strahlumlenkungseinheit dazu ausgelegt, das erste Teilstrahlenbündel oder das zweite Teilstrahlenbündel ein einziges Mal mittels einmaliger Reflexion an einer Spiegelfläche bzw. Einzel-Spiegelfläche der entsprechenden Strahlumlenkungseinheit abzulenken.

In einem besonders bevorzugten Fall kann es sich bei senkrechtem Auftritt des Teilstrahlenbündels auf die Spiegelfläche um eine Rückreflexion handeln, also eine Ablenkung, die im Wesentlichen 180° entspricht.

Insbesondere ist mindestens eine aus der ersten Strahlumlenkungseinheit und der zweiten Strahlumlenkungseinheit dazu ausgelegt ist, das erste Teilstrahlenbündel und/oder das zweite Teilstrahlenbündel drei Mal mittels dreimaliger Reflexion an drei Spiegelflächen einer Dreifach-Winkelspiegelgruppe der entsprechenden Strahlumlenkungseinheit abzulenken.

Insbesondere weist die erste Strahlumlenkungseinheit und die zweite Strahlumlenkungseinheit zusammen eine Anzahl von Spiegelflächen, die entweder (2n+1)+1 oder (2n₁+1)+(2n₂+1) entspricht und wobei n eine natürliche Zahl ≥1, n₁ eine natürliche Zahl ≥1 und n₂ eine natürliche Zahl ≥1 ist.

In anderen Worten beträgt die Gesamtzahl der Spiegel oder Spiegelflächen im Michelsontyp-Interferometer in beiden Armen mindestens 4 und zwar entweder (2n+1)+1 oder (2n₁+1)+(2n₂+1), dabei ist die Gesamtzahl eine gerade Zahl.

Beispielsweise kann ein Arm eine (2n+1)-Spiegelgruppe aufweisen, die bei n=1 drei Spiegel umfasst und der andere Arm kann einen Einzelspiegel aufweisen. In dem Fall liegt die Gesamtzahl bei 4 Spiegeln bzw. Spiegelflächen.

Beispielsweise kann ein Arm eine (2n+1)-Spiegelgruppe aufweisen, die bei n=2 fünf Spiegel umfasst und der andere Arm kann einen Einzelspiegel aufweisen. In dem Fall liegt die Gesamtzahl bei 6 Spiegeln bzw. Spiegelflächen.

Beispielsweise kann ein Arm eine (n₁+1)-Spiegelgruppe aufweisen, die bei n₁=1 drei Spiegel umfasst und der andere Arm kann eine weitere (n₂+1)-Spiegelgruppe aufweisen, die bei n₂=1 drei Spiegel umfasst. In dem Fall liegt die Gesamtzahl bei 6 Spiegeln bzw. Spiegelflächen. n₁ bezieht sich dabei auf den ersten Arm und n₂ bezieht sich auf den zweiten Arm. Beispielsweise kann ein Arm eine (ni+1)-Spiegelgruppe aufweisen, die bei n₁=2 fünf Spiegel umfasst und der andere Arm kann eine weitere (n₂+1)-Spiegelgruppe aufweisen, die bei n₂=1 drei Spiegel umfasst. In dem Fall liegt die Gesamtzahl bei 8 Spiegeln bzw. Spiegelflächen.

Erfindungsgemäß umfasst das Michelsontyp-Interferometer ferner zumindest eine der Strahlteilereinheit nachgeordnete Bildfeld-Diskriminatoreinheit, welche derart angeordnet ist, dass das erste Teilstrahlenbündel und/oder das zweite Teilstrahlenbündel räumlich selektiert wird.

Insbesondere ist zwischen zwei der (2n+1) Spiegelflächen der (2n+1)-Winkelspiegelgruppe der zumindest einen Strahlumlenkungseinheit zumindest eine Bildfeld-Diskriminatoreinheit derart angeordnet, dass das erste Teilstrahlenbündel und/oder das zweite Teilstrahlenbündel räumlich selektiert wird.

Insbesondere ist zumindest eine Bildfeld-Diskriminatoreinheit in einer der Spiegelflächen der ersten Strahlumlenkungseinheit und/oder der zweiten Strahlumlenkungseinheit integriert. Insbesondere umfasst das Michelsontyp-Interferometer ferner zumindest eine der Strahlteilereinheit nachgeordnete erste und eine zweite Bildfeld-Diskriminatoreinheit, welche derart angeordnet sind, dass das erste Teilstrahlenbündel und das zweite Teilstrahlenbündel räumlich selektiert werden und dass die erste Bildfeld-Diskriminatoreinheit optisch konjugiert zur zweiten Bildfeld-Diskriminatoreinheit ist.

Insbesondere weist die Bildfeld-Diskriminatoreinheit zumindest eines der folgenden auf: eine spaltförmige Abschattblende,
ein Mikrospalt-Abschattblenden-Array,
eine Pinhole-Abschattblende,
ein eindimensionales oder ein zweidimensionales Pinhole-Abschattblenden-Array in Form einer Blendenscheibe,
ein Mikrospalten-Abschattblenden-Array mit einer Vielzahl von Mikrospalten in lateral versetzter Anordnung,
ein Mikrospalten-Abschattblenden-Array mit mechanisch beweglichen Elementen,
ein reflektiver Bereich spaltförmiger Bereich, der einen Teil der ersten und/oder der zweiten Strahlumlenkungseinheit darstellt.

Erfindungsgemäß handelt es sich um ein Fourier-Transformations-Spektrometer, wobei mindestens ein Bildfeld-Diskriminator dem Strahlteiler des Michelsontyp-Interferometers nachgeordnet ist.

Insbesondere kann es sich um ein Fourier-Transformations-Spektrometer handeln, bei dem ein Bildfeld-Diskriminator in einem Interferometerarm des Michelsontyp-Interferometers in einer reellen Spiegelfläche angeordnet ist.

Insbesondere kann es sich auch um ein Fourier-Transformations-Spektrometer handeln, bei dem im Michelsontyp-Interferometer ein Bildfeld-Diskriminator im ersten Interferometerarm in einer zur scheinbaren Endspiegelfläche des zweiten Interferometerarms optisch konjugierten Fläche angeordnet ist.

Insbesondere kann es sich auch um ein Fourier-Transformations-Spektrometer handeln, bei dem im zweiten Interferometerarm des Michelsontyp-Interferometers ein zweiter Bildfeld-Diskriminator angeordnet ist, welcher zum Bildfeld-Diskriminator im ersten Interferometerarm optisch konjugiert und geometrisch zumindest annäherungsweise gleich dem ersten Bildfeld-Diskriminator ausgebildet ist.

Zusätzlich kann es sich auch um ein Fourier-Transformations-Spektrometer handeln, bei welchem dem Michelsontyp-Interferometer mindestens ein Bildfeld-Diskriminator direkt vorgeordnet ist.

Zusätzlich kann es sich auch um ein Fourier-Transfonnations-Spektrometer handeln, bei dem mindestens ein Bildfeld-Diskriminator dem Bild von einem Messobjekt im Strahlengang im Michelsontyp-Interferometer zugeordnet ist, welches mittels vorgeordnetem Objektiv gebildet ist.

Zusätzlich oder alternativ kann es sich auch um ein Fourier-Transformations-Spektrometer handeln, bei dem im Michelsontyp-Interferometer ein erster Bildfeld-Diskriminator dem planen Spiegel bzw. dem Endspiegel und ein zweiter Bildfeld-Diskriminator der Dreifach-Winkelspiegelgruppe zugeordnet ist.

Zusätzlich oder alternativ kann es sich auch um ein Fourier-Transformations-Spektrometer handeln, bei dem im Michelsontyp-Interferometer der Bildfeld-Diskriminator durch den planen Spiegel bzw. Endspiegel oder durch einen Spiegel der Dreifach-Winkelspiegelgruppe gebildet ist.

Insbesondere kann es sich auch um ein Fourier-Transformations-Spektrometer handeln, bei dem im Michelsontyp-Interferometer mindestens ein Bildfeld-Diskriminator als gerasterter Spiegel oder als eine gerasterte Spiegelfläche ausgebildet ist.

Insbesondere kann es sich auch um ein Fourier-Transformations-Spektrometer handeln, bei dem im Michelsontyp-Interferometer den Elementen des gerasterten Spiegels oder der gerasterten Spiegelfläche rechnersteuerbare Bewegungselemente zugeordnet sind.

Zusätzlich oder alternativ kann es sich auch um ein Fourier-Transformations-Spektrometer handeln, bei dem einer Platzierung von je einem Bildfeld-Diskriminator in jedem Interferometerarm des Michelsontyp-Interferometers diese beiden Bildfeld-Diskriminatoren optisch zueinander konjugiert angeordnet sind.

Insbesondere kann es sich auch um ein Fourier-Transformations-Spektrometer handeln, bei dem mindestens ein Bildfeld-Diskriminator als spaltförmige Abschattblende ausgebildet ist.

Insbesondere kann es sich auch um ein Fourier-Transformations-Spektrometer handeln, bei dem mindestens ein Bildfeld-Diskriminator als Mikrospalt-Abschattblenden-Array ausgebildet ist.

Zusätzlich oder alternativ kann es sich auch um ein Fourier-Transformations-Spektrometer handeln, bei dem mindestens ein Bildfeld-Diskriminator als Pinhole-Abschattblende ausgebildet ist.

Zusätzlich oder alternativ kann es sich auch um ein Fourier-Transformations-Spektrometer handeln, bei dem mindestens ein Bildfeld-Diskriminator als eindimensionales oder zweidimensionales Pinhole-Abschattblenden-Array in Form einer Blendenscheibe ausgebildet ist.

Insbesondere kann es sich auch um ein Fourier-Transformations-Spektrometer handeln, bei dem ein Mikrospalten-Abschattblenden-Array mit Mikrospalten in lateral versetzter Anordnung ausgebildet ist.

Insbesondere kann es sich auch um ein Fourier-Transformations-Spektrometer handeln, bei dem das Mikrospalten-Abschattblenden-Array mit mechanisch beweglichen Elementen ausgebildet ist.

Insbesondere kann es sich auch um ein Fourier-Transformations-Spektrometer handeln, bei dem ein feinstrukturierter Feld-Diskriminator in Spaltenform oder in Punktlinienform dem Messobjekt oder dem Feld einer Lichtquelle zugeordnet ist und die Längsrichtung des Feld-Diskriminators senkrecht zur Referenzebene ausgerichtet ist.

Insbesondere kann es sich auch um ein Fourier-Transformations-Spektrometer handeln, bei dem die Lichtquelle selbst in Spaltenform oder in Form feiner leuchtender Elemente in gerader Linie oder in Zickzack-Linie feinstrukturiert ausgebildet ist und die Längsrichtung derselben senkrecht zur Referenzebene ausgerichtet ist.

Insbesondere kann es sich auch um ein Fourier-Transformations-Spektrometer handeln, bei dem das Feld des Messobjekts und das Feld der Lichtquelle zumindest in einem Teilbereich optisch zueinander konjugiert angeordnet sind.

Insbesondere kann es sich auch um ein Fourier-Transformations-Spektrometer handeln, bei dem das Michelsontyp-Interferometer als Luft-Typ oder als Prismen-Typ oder als hybride Luft-Prismen-Anordnung ausgebildet ist.

Zusätzlich oder alternativ kann es sich auch um ein Fourier-Transformations-Spektrometer handeln, bei dem eine konfokale Anordnung dem Michelsontyp-Interferometer vorgeordnet ist.

Zusätzlich oder alternativ kann es sich auch um ein Fourier-Transformations-Spektrometer handeln, bei dem die konfokale Anordnung mit einer starren Blendenscheibe oder einer rotierenden Blendenscheibe ausgebildet ist.

Insbesondere kann es sich auch um ein Fourier-Transformations-Spektrometer handeln, bei dem die konfokale Anordnung mit einem räumlichen Lichtmodulator in Reflexion oder Transmission ausgebildet ist.

Insbesondere kann es sich auch um ein Fourier-Transformations-Spektrometer handeln, bei dem die konfokalen Diskriminatorelemente einer konfokalen Anordnung mit mindestens einer effektiven Spiegelfläche im Arm eines Michelsontyp-Interferometers zumindest näherungsweise optisch konjugiert sind.

Insbesondere kann es sich auch um ein Fourier-Transformations-Spektrometer handeln, bei dem mindestens ein Bildfeld-Diskriminator im Michelsontyp-Interferometer als steuerbarer räumlicher Lichtmodulator in Reflexion ausgebildet ist.

Insbesondere kann es sich auch um ein Fourier-Transformations-Spektrometer handeln, bei dem mindestens ein Bildfeld-Diskriminator im Fourier-Transformations-Spektrometer-System als steuerbarer räumlicher Lichtmodulator in Transmission ausgebildet ist.

Insbesondere kann es sich auch um ein Fourier-Transformations-Spektrometer handeln, bei dem Bewegungsmittel in der Tiefe dem Messobjekt oder dem mobilen Messkopf oder einer Komponente des mobilen Messkopfs zugeordnet sind.

Insbesondere weist zumindest eine aus der ersten Strahlumlenkungseinheit und der zweiten Strahlumlenkungseinheit ein Prisma mit zumindest einer Reflexionsfläche auf, die dazu ausgelegt ist, das erste Teilstrahlenbündel und/oder das zweite Teilstrahlenbündel einmal zu reflektieren.

Insbesondere umfasst das Fourier-Transformations-Spektrometer ferner eine dem Michelsontyp-Interferometer vorgeordnete konfokale Anordnung.

Insbesondere weist die konfokale Anordnung eine starren Blendenscheibe oder eine rotierenden Blendenscheibe und/oder einen räumlichen Lichtmodulator in Reflexion oder Transmission aufweist.

Insbesondere stellt die Strahlteilereinheit einen Amplituden-Strahlteiler dar und weist eine ebene Strahlteilerschicht, eine Mylarfolie oder ein Gitter auf.

In anderen Worten kann es sich optional um ein Fourier-Transformations-Spektrometer handeln, bei dem der Strahlteiler als ein Amplituden-Strahlteiler, dargestellt durch eine ebene Strahlteilerschicht oder eine Mylarfolie oder ein Gitter, ausgebildet ist.

Gemäß einem weiteren Aspekt umfasst ein Verfahren zur interferometrischen Messung mittels eines Fourier-Transformations-Spektrometers mit Misontyp-Interferometer die Merkmale aus Anspruch 14.

Bevorzugt umfasst das Verfahren die Schritte, die ausgelöst und zumindest teilweise ausgeführt werden mittels zumindest einer Recheneinheit:
- Mehrmaliges gleichzeitiges Erfassen der Vielzahl von räumlichen Interferogrammen zu jeweils unterschiedlichen Zeitpunkten;
- Fourier-Transformierung der zu jeweils unterschiedlichen Zeitpunkten erfassten Vielzahl von räumlichen Interferogrammen zur Erzeugung einer Vielzahl von Spektren; und
- Erzeugung mindestens eines hyperspektralen Bildes des räumlich ausgedehnten Objekts.

Es besteht insbesondere ein Vorteil darin, von einzelnen selektierten Bereichen des Bildes vom zu detektierenden Objekt zeitgleich mindestens zwei räumliche Interferogramme erzeugen zu können und aufzunehmen, in der Regel jedoch eine größere Anzahl von räumlichen Interferogrammen, welche die Information über das gesuchte Spektrum zumindest teilweise enthalten und so die Erfassung von zwei oder mehreren vollständigen räumlichen Interferogrammen in einer einzigen Aufnahme durchgeführt werden kann.

Gemäß eines Beispiels, das nicht unter den Schutzumfang der Erfindung fällt, ist die Verwendung eines Bildfeld-Diskriminators optional. Insbesondere bei der Untersuchung von alleinstehenden Punktlicht-Quellen (Stern ohne Nachbarsteme) kann bei einem

FT-Spektrometer beispielsweise auf einen Bildfeld-Diskriminator verzichtet werden.

Im Folgenden werden weitere Ausführungsformen, Merkmale und Beispiele dargestellt, die einerseits die Erfindung nicht einschränken und andererseits miteinander kombinierbar sind, sofern sie sich nicht ausschließen.

Im Wesentlichen geht es darum, auch bei Vibrationen oder Turbulenzen in einer zu vermessenden Szene, also z.B. im Feldeinsatz, in der Regel weitgehend unverfälschte Spektren zu bekommen, jedoch gewisse Fehler bei der Abtastung des Objektes, also im Aufbau des hyperspektralen Bildes in Form des bekannten Datenkubus (x, y, sigma) mit sigma als Wellenzahl oder (x, y, lambda) mit lambda als Wellenlänge zuzulassen. Die spektralen Informationen sollen im Single-Shot-Modus gewonnenen werden. Dadurch sind diese in der Tendenz weniger fehlerbehaftet als die Ortinformationen vom Objekt, die seriell gewonnen werden.

Bei dieser Erfindung soll sich das Messobjekt in der Regel außerhalb des Interferometers befinden und ist hier in der Regel dem Interferometer vorgeordnet.

Es soll zunächst festgestellt werden, dass hier auch von Single-Shot-Bildgebung geschrieben wird, wenn nur zwei oder wenige räumliche Interferogramme von einem Messobjekt im Single-Shot-Modus gewonnen werden können und zu einem hyperspektralen Teilbild verrechnet werden. In der Regel sollen jedoch typischerweise mindestens einhundert räumliche Interferogramme mit der Erfindung im Single-Shot-Modus gewonnen werden können. Diese können beispielsweise von einer Linie, einem schmalen Bereich oder von einem Raster jeweils auf dem Messobjekt stammen.

Das Gewinnen räumlicher Interferogramme für die Fourier-Spektroskopie mittels Lateral-Shear zwischen Objekt- und Referenzwellenfronten erfolgt am Ausgang eines Michelsontyp-Interferometers unter Nutzung eines dem Interferometer nachgeordneten anamorphotischen Objektivs mit positiver Brechkraft. Ein räumliches Interferogramm kann dabei in der Fourier-Ebene des nachgeordneten Objektivs detektiert werden. Die Fourier-Ebene stellt die nachgeordnete Brennebene des genannten Objektivs dar. Mittels einer numerisch ausgeführten Fourier-Transformation wird das Spektrum aus dem räumlichen Interferogramm errechnet. Bei dieser Erfindung gibt es im Vergleich zu den seriell mit einem räumlichen Interferogramm im Vollbild arbeitenden Verfahren hier ein entweder eindimensional begrenztes Messfeld oder auch flächig in einem eher groben Raster von Messstellen verteilte Spektren. Dieser Nachteil relativiert sich jedoch, wenn bei einer aktiven Beleuchtung des Messobjekts mittels einer künstlichen Lichtquelle die verfügbare Lichtenergie P_total dieser Lichtquelle anstelle auf eine z.B. Kreisfläche A_Kreis auf eine beispielsweise streifenförmige Fläche A_Streifen konzentriert wird, wodurch sich eine wesentlich höhere Beleuchtungsstärke ergibt. Dann ist die Beleuchtungsstärke auf der kleineren streifenförmigen Fläche entsprechend höher und die Integrationszeit eines gerasterten Detektors kann entsprechend verkleinert werden. Also besteht ein Vorteil der erfinderischen Lösung, insbesondere bei der Beleuchtung mit einer an das Messfeld des Messobjekts geometrisch angepassten Lichtquelle. Ein besonderer Vorteil ist gegeben, wenn für ein nichtbiologisches Messobjekt bei der Beleuchtung zumindest kurzzeitig eine sehr hohe Beleuchtungsstärke, beispielsweise in der Form eines schmalen Lichtstreifens auf dem Messobjekt zulässig ist. Die Beleuchtung des Messobjekts kann auch mit einem Bündel-Querschnittswandler durchgeführt werden, der zu einem langgezogenen schmalen Bereich geformt ist.

Die Merkmale der Erfindung werden im Weiteren dargestellt.

Die Erfindung betrifft insbesondere ein Fourier-Transformations-Spektrometer mit zumindest teilweiser hyperspektraler Bildgebung von einem Messobjekt als Ergebnis einer Berechnung mit einem Rechnersystem zur Errechnung von Spektren mittels Fourier-Transformation.

Das Fourier-Transformations-Spektrometer ist insbesondere mit einem dem Michelsontyp-Interferometer vorgeordneten Objektiv mit einer optischen Achse OAI, ausgebildet, welches als fokussierendes Abbildungssystem zur Erzeugung mindestens eines fokussierten Eingangsstrahlenbündels für das Michelsontyp-Interferometer dient. Das vorgeordnete Objektiv kann einstufig, zweistufig oder auch mehrstufig ausgebildet sein. Das Michelsontyp-Interferometer ist dem vorgeordneten Objektiv also nachgeordnet und weist bekannterweise zwei Interferometerarme auf. In der Regel besteht eine Vielzahl von fokussierten Eingangsstrahlenbündeln am Eingang des Michelsontyp-Interferometers, da zu jedem erfassten Objektpunkt ein fokussiertes Eingangsstrahlenbündel gehört. Diese Eingangsstrahlenbündel werden hier also in der Regel als ein Ensemble von Eingangsstrahlenbündeln verstanden, weil ja eine Bildinformation transportiert und zu jedem Bildpunkt ein eigenes Eingangsstrahlenbündel gehört, so dass für viele Bildpunkte auch viele Eingangsstrahlenbündel zugehörig sind. So besteht in der Regel ein Ensemble von Eingangsstrahlenbündeln. In der weiteren Beschreibung in den Figuren geht es bei einem Strahlenbündel letztlich um das Ensemble von Strahlenbündel und repräsentativ ist ein Strahlenbündel genannt oder figürlich dargestellt. Dies gilt im Weiteren auch für Teilstrahlenbündel. Jedes Teilstrahlenbündel ist im Sinne der Erfindung hier für ein Ensemble von Teilstrahlenbündeln repräsentativ.

Das Michelsontyp-Interferometer weist insbesondere weiterhin auf.

Einen Strahlteiler mit ebener Strahlteilerfläche, dargestellt vorzugsweise durch eine ebene Strahlteilerschicht oder vorzugsweise eine Mylarfolie oder vorzugsweise ein Gitter. Der Strahlteiler wird sowohl zur Strahlenbündelteilung genutzt, wodurch zwei Teilstrahlenbündel gebildet werden, als auch zur zumindest teilweisen Strahlenbündelvereinigung mit einer Lateral-Shear s zwischen den beiden Teilstrahlenbündeln TB 1 und TB2.

Am Michelsontyp-Interferometer besteht insbesondere eine Referenzebene RE, welche durch die Normale NT der ebenen Strahlteilerfläche des Michelsontyp-Interferometers und durch die optische Achse OAI des vorgeordneten Objektivs am Eingang des Michelsontyp-Interferometers aufgespannt ist.

Zur Detektion kann dem Michelsontyp-Interferometer ein gerasterter Detektor nachgeordnet sein. Der gerasterte Detektor kann eine UV-Kamera und im sichtbaren Spektralbereich eine CCD- oder eine CMOS-Kamera sein. Im nah-infraroten Spektralbereich kann eine InGaAs-Kamera mit Vorteil eingesetzt werden. Im mittleren Infrarot kommt als gerasterter Detektor ein Focal Plane Array (FPA), auch als IRFPA bekannt, zur Anwendung, das gegebenenfalls auch gekühlt ist. In der hybriden CMOS FPA-Technik wird mit Vorteil ein gerasterter Detektor mit einer Quecksilber-Cadmium Telluride-Verbindung (MCT) eingesetzt.

Im gesamten Infrarotbereich können Bolometer-Matrix-Detektoren, insbesondere Mikrobolometer, eingesetzt werden. Bevorzugt werden für diese Erfindung Matrix-Detektoren eingesetzt.

Es ist aber bevorzugt auch möglich, dem Michelsontyp-Interferometer zwei oder mehrere schnelle Zeilen-Detektoren in einer der vorab bereits genannten Technologien und Spektralbereiche nachzuordnen. Dabei sind die einzelnen Zeilen-Detektoren bei der primären Datenerfassung bevorzugt in einem gemeinsamen Detektorfeld parallel zueinander angeordnet, digital-elektronisch jedoch unabhängig voneinander. Diese werden also getrennt betrieben, um ein Maximum an Auslesegeschwindigkeit erreichen zu können. Die Anzahl der Zeilen-Detektoren entspricht der Anzahl der Messpunkte. Dabei können die einzelnen Pixel einer Zeile auch mit einem besonders hohen Aspektverhältnis ausgebildet sein, um zwecks eines hohen Signal-Rauschverhältnisses einen möglichst großen Betrag an Lichtenergie zu detektieren. Derartige Anwendungen sind insbesondere für Raumfahrt-Applikationen einsetzbar. Dies macht Sinn, wenn in einer Richtung, hier der y-Richtung, eine höhere laterale Auflösung im Bild gewünscht ist, in der x-Richtung aber nicht.

Weiterhin ist dem Michelsontyp-Interferometer zur Beleuchtung des Messobjekts bevorzugt einerseits entweder mindestens eine Lichtquelle beigeordnet, welche bevorzugt auch steuerbar ist und Lichtmuster ausbilden kann. Andererseits kann das Messobjekt auch ein Selbststrahler wie eine heiße Abgaswolke mit Strahlung im infraroten Spektralbereich sein.

Das Fourier-Transformations-Spektrometer ist mit einem dem Michelsontyp-Interferometer vorgeordneten Objektiv, genutzt als Abbildungssystem für das Messobjekt, mit der optischen Achse OAI zur Erzeugung mindestens eines fokussierten Eingangsstrahlenbündels EB für das Michelsontyp-Interferometer ausgebildet.

Vor dem Michelsontyp-Interferometer ist dementsprechend ein vorgeordnetes Objektiv positioniert, welches als Abbildungssystem für das Messobjekt genutzt wird. Es dient insbesondere der Erzeugung mindestens eines fokussierten Eingangsstrahlenbündels EB für ein Michelsontyp-Interferometer. Das vorgeordnete Objektiv ist bevorzugt auf der dem Interferometer zugewandten Seite zumindest näherungsweise telezentrisch ausgebildet. Dieses vorgeordnete Objektiv kann bevorzugt auch beidseitig telezentrisch ausgebildet sein. Eine Telezentrie auf der dem Interferometer zugewandten Seite verringert die Anforderungen an die nachfolgende Optik erheblich, da keine sehr schiefen Strahlen auftreten.

Bevorzugt kann das Michelsontyp-Interferometer mit einem vorgeordneten Objektiv, insbesondere mit einer zum gerasterten Detektor synchronisierten Blitzlichtquelle, auch als mobiler Messkopf ausgebildet sein.

Entweder nur ein räumliches Interferogramm ist auf dem gerasterten Detektor ausgebildet. Dieses wird bevorzugt mittels eines Fourier-Objektivs erzeugt und es kommen zumindest näherungsweise ebene Wellen zur Interferenz. Andererseits ist auch ein Ansatz ohne ein Fourier-Objektiv und ohne weitere optische Komponenten am Interferometerausgang umsetzbar. Dies führt dann zur Interferenz von zwei Kugelwellen auf dem gerasterten Detektor, da keine Strahlenbündelformung erfolgt. Die Interferenz von Kugelwellen, insbesondere mit kleinem Krümmungsradius, stellt jedoch keine technisch sehr zufriedenstellende Lösung für die FT-Spektroskopie dar, da das räumliche Interferogramm im Randbereich Nichtlinearitäten aufweisen kann, welche die Errechnung von Spektren erheblich erschweren können.

Gemäß der Erfindung wird anstelle nur eines räumlichen Interferogramms gleichzeitig eine Vielzahl von räumlichen Interferogrammen auf dem gerasterten Detektor mittels einer anamorphotischen Abbildungsstufe gebildet, welche dem Michelsontyp-Interferometer nachgeordnet ist.

Das Michelsontyp-Interferometer kann bevorzugt mit einem Winkel zwischen den beiden Interferometerarmen von 90 Altgrad aufgebaut sein. Weiterhin kann das Michelsontyp-Interferometer bevorzugt aber auch mit einem Winkel zwischen den beiden Interferometerarmen weit verschieden von 90 Altgrad (1 Altgrad =1/360 des Vollkreises) konstruiert sein. Die beiden Interferometerarme können bevorzugt durchaus auch einen Winkel von bis zu 135 Altgrad oder auch vorzugsweise bis zu 150 Altgrad oder bevorzugt von 45 Altgrad auch vorzugsweise bis zu 30 Altgrad zueinander aufweisen. Eine Abkehr von der Rechtwinklichkeit der Interferometerarme kann unter speziellen Randbedingungen - wie ein konstruktiv vorgegebener langgestreckter Bauraum - erhebliche Vorteile bieten.

Das Messobjekt kann beleuchtet sein und reflektiertes, streuendes oder auch fluoreszierendes Licht kann spektral ausgewertet werden. Aber auch die Auswertung transmittierten Lichts, auch als fluoreszierendes Licht, ist möglich. Das Messobjekt kann jedoch auch ein selbstleuchtendes Objekt sein, beispielsweise in Form heißer Verbrennungsgase des Triebswerks eines Luftfahrzeugs mit einem dominanten Spektrum auch im mittleren Infrarot-Spektralbereich.

Die Erfindung ist nun zum einen auch dadurch gekennzeichnet, dass vorzugsweise entweder ein zumindest näherungsweise planer Endspiegel oder eine plane Endspiegelfläche im ersten Interferometerarm des Michelsontyp-Interferometers angeordnet ist. Dieser plane Endspiegel oder plane Endspiegelfläche ist bevorzugt schmal ausgebildet. Im zweiten Interferometerarm ist eine Dreifach-Winkelspiegelgruppe als Endreflektor angeordnet. Die Dreifach-Winkelspiegelgruppe besteht definitionsgemäß aus einer Anordnung von - in der Summe - drei zumindest näherungsweise planen Spiegeln oder zumindest näherungsweise planen Spiegelflächen in Rachen- oder W-Form und jeweils mit Winkel zueinander. Diese zumindest näherungsweise planen Spiegeln oder zumindest näherungsweise planen Spiegelflächen sind dabei in der Regel senkrecht zur Referenzebene RE ausgerichtet, wobei die Referenzebene RE im Michelsontyp-Interferometer in der Regel senkrecht zur Strahlteilerfläche ist.

In der Patentschrift DE 10 2010 06 239B3, Figur 3 ist der Fall einer invarianten Lateral-Shear bei einer Dreifach-Winkelspiegelgruppe nach oben genannter Definition dargestellt. Beim Auftreten einer Lateralverschiebung v einer Dreifach-Winkelspiegelgruppe bleibt die Lateral-Shear zwischen Eingangs- und Ausgangsstrahlen unverändert.

Die Erfindung ist zum anderen auch dadurch gekennzeichnet, dass vorzugsweise in jedem der beiden Interferometerarme des Michelsontyp-Interferometers jeweils eine Dreifach-Winkelspiegelgruppe nach oben genannter Definition als Endreflektor angeordnet ist.

Weiterhin ist die Erfindung nun zum anderen auch dadurch gekennzeichnet, dass vorzugsweise in mindestens einem der beiden Interferometerarme des Michelsontyp-Interferometers als Endreflektor vorzugsweise eine (2n+1)-fach-Winkelspiegelgruppe mit n = 2, 3, 4... angeordnet ist, die aus einer Anordnung von - in der Summe (2n+1) planen Spiegeln oder planen Spiegelflächen in Rachen- oder W-Form oder in einer Mischform und jeweils mit Winkel zueinander besteht. Diese zumindest näherungsweise planen Spiegel oder zumindest näherungsweise planen Spiegelflächen sind in der Regel senkrecht zu einer gemeinsamen Referenzebene RE ausgerichtet sind, wobei auch hier die Referenzebene RE im Michelsontyp-Interferometer in der Regel senkrecht zur Strahlteilerfläche ist.

In allen Fällen gilt, dass die Gesamtzahl der Spiegel oder Spiegelflächen im Michelsontyp-Interferometer mindestens vier beträgt und bei einer größeren Gesamtzahl als vier in der Regel geradzahlig ist.

Der Einsatz einer Dreifach-Winkelspiegelgruppe nach oben genannter Definition wird im Vergleich zu Winkelspiegelgruppen mit einer größeren Spiegelanzahl als drei als technisch besonders vorteilhaft angesehen, da hier die im Vergleich optischen Wege am kürzesten sind und deren konstruktiver Aufbau vergleichsweise einfach ist.

Dabei ist eine Dreifach-Winkelspiegelgruppe oder eine (2n+1)-fach-Winkelspiegelgruppe mit n=2, 3, ... so ausgebildet und im Interferometer so angeordnet, dass die Spiegel und Spiegelflächen derselben in der Regel senkrecht zu einer Referenzebene RE angeordnet sind. Der Einfallswinkel des Hauptstrahls eines Teilstrahlenbündels im Interferometer auf einen der planen Spiegel oder eine der planen Spiegelflächen ist dabei in der Regel größer als zwei Altgrad. Der Aperturwinkel des Teilstrahlenbündels ist in der Regel kleiner als der Einfallswinkel des Hauptstrahls und somit besteht in keinem Fall ein senkrechter Einfall von Strahlen - auch nicht von Randstrahlen - auf einen der planen Spiegel oder eine der planen Spiegelflächen.

An jedem Spiegel oder an jeder Spiegelfläche im Michelsontyp-Interferometer erfahren die Strahlen eines Teilstrahlenbündels beim Passieren eines Interferometerarms in allen Fällen nur jeweils eine einmalige Reflexion.

Durch eine Dreifach-Winkelspiegelgruppe oder eine (2n+1)-fach-Winkelspiegelgruppe mit n=2, 3, ... besteht am Ausgang des Interferometers in der Regel eine invariante Lateral-Shear. Auch bei einem lateralen Bewegen dieser Winkelspiegelgruppe ist die sich aus der Geometrie dieser Winkelspiegelgruppe ergebende Lateral-Shear in der Regel völlig unveränderlich.

Mittels dieser konstanten Lateral-Shear entstehen bevorzugt zum einen entweder in der Fourier-Ebene eines Fourier-Objektivs zueinander gekippte Planwellenfronten oder bevorzugt zueinander gekippte Zylinderwellenfronten. Diese zueinander gekippten Zylinderwellenfronten entstehen am Ausgang einer dem Interferometer nachgeordneten anamorphotischen Abbildungsstufe. Diese durch Strahlteilung erzeugte Zylinderwellen bilden in der Regel räumliche Interferenzen und werden der Detektion mittels eines gerasterten Empfängers zugeführt.

Bei einer Vielzahl von räumlichen Interferogramm wird aus jedem räumlichen Interferogramm das Spektrum mittels Fourier-Transformation errechnet und einem Bildpunkt zugeordnet.

Zum anderen können aber auch Kugelwellen mit Lateral-Shear am Ausgang des Interferometers zur Interferenz kommen.

Vorzugsweise überdecken die einzelnen Spiegel oder Spiegelflächen den Querschnitt eines Teilstrahlenbündels in der Regel zumindest näherungsweise voll, Somit passiert bevorzugt kein Strahlenbündel eine Dachkante oder wird durch diese geteilt.

Vorzugsweise ist das Michelsontyp-Interferometer hinsichtlich der optischen Weglänge für die Hauptstrahlen in den beiden Interferometerarmen abgeglichen, so dass für diese Differenz der optischen Weglängen (optische Weglängendifferenz, optical path difference = OPD) der Wert gleich null besteht, und symmetrische Interferogramme gebildet sind. Jedoch kann durch das Einsetzen refraktiver Materialien, wie einer vergleichsweise dünnen, transparenten und achssenkrecht angeordneten Planparallelplatte in einem Arm des Michelsontyp-Interferometers - oder je einer Planparallelplatte in jedem Interferometerarm mit etwas ungleicher Dicke - die optische Weglängendifferenz OPD für die Hauptstrahlen bevorzugt ungleich null gemacht werden. So kann bei gegebenem Detektor durch eine asymmetrische Position des räumlichen Interferogramms auf dem gerasterten Detektor die spektrale Auflösung in bekannter Weise bis fast zum Faktor zwei gesteigert werden.

Dabei ist bei dem Fourier-Transfonnations-Spektrometer mit Michelsontyp-Interferometer der Strahlteiler dieses Interferometers vorzugsweise als ein Amplituden-Strahlteiler ausgebildet. Das ist vorzugsweise eine ebene Strahlteilerschicht oder eine Mylarfolie oder ein Gitter.

Dieser Strahlteiler kann mittels Teilerschichten für das UV-Spektralgebiet, für den sichtbaren Bereich, für den nah-infraroten Bereich, für den mittleren Infrarotbereich (MIR) oder mittels einer Mylarfolie auch für den fernen Infrarotbereich (FIR) ausgebildet sein. Für den Terahertz-Bereich oder den fern-infraroten Spektralbereich kann auch ein Strahlteiler mittels einer feinen Drahtgitter-Struktur, auch als Grid-Struktur bekannt, hergestellt sein.

Erfindungsgemäß ist dem Fourier-Transformations-Spektrometer mindestens ein Bildfeld-Diskriminator dem Strahlteiler des Michelsontyp-Interferometers in Lichtrichtung nachgeordnet. Der Bildfeld-Diskriminator kann im einfachsten Fall als ein Pinhole, also als eine sehr feine Blendenöffnung, ausgebildet sein.

Bevorzugt ist der Bildfeld-Diskriminator als eine Spaltblende oder als ein Mikrospiegel oder als ein schmaler, länglicher Spiegel ausgebildet.

Vorzugsweise ist bei dem Fourier-Transformations-Spektrometer ein Bildfeld-Diskriminator in einem Interferometerarm in einer reellen Spiegelfläche angeordnet. Die reelle Spiegelfläche ist - wie bereits beschrieben - durch die Fläche eines einzelnen Planspiegels im Arm des Michelsontyp-Interferometers gebildet. Dort entsteht vorzugsweise auch das scharfe Bild des Messobjekts. Der Planspiegel kann dabei durch seine spezielle Form selbst die Bildfeld-Diskriminierung bewirken, indem dieser vorzugsweise selbst sehr schmal oder sogar als Mikrospiegel ausgebildet ist.

Ein Bildfeld-Diskriminator kann dabei vorzugsweise auch als eine auf dem Planspiegel aufgebrachte Abschattblende ausgebildet sein. Diese Abschattblende kann vorzugsweise aber auch durch einen Aufdruck auf dem Spiegel gebildet sein. Vorzugsweise kann der Spiegel auch als ein linienhaftes oder schmales flächenhaftes Array von Mikrospiegeln ausgebildet sein.

Andererseits ist bei dem Fourier-Transformations-Spektrometer ein Bildfeld-Diskriminator im ersten Interferometerarm in einer zur scheinbaren Endspiegelfläche des zweiten Interferometerarms optisch konjugierten Fläche angeordnet. In einem Interferometerarm mit drei Spiegeln oder drei Spiegelflächen in einer Winkelanordnung liegt die daraus resultierende scheinbare Endspiegelfläche außerhalb der Endspiegel-Anordnung.

Bei einer Endspiegel-Anordnung mit einer ungeradzahligen Anzahl von Spiegeln oder Spiegelflächen in einer Winkelanordnung größer als drei sind die optischen Wege in der Regel gegenüber einer Anordnung mit drei Spiegeln oder Spiegelflächen jedoch deutlich vergrößert, wodurch sich der Öffnungswinkel reduziert, was als eher technisch nicht vorteilhaft angesehen wird.

Durch eine in der Summe im Michelsontyp-Interferometer in der Regel geradzahlige Anzahl von Spiegeln oder Spiegelflächen, die jeweils nur eine einzige Reflexion im Strahldurchgang aufweisen, und dabei einer in der Regel ungeradzahligen Anzahl Spiegeln oder Spiegelflächen mindestens in einem Arm des Interferometers, also 3, 5, ... 2n+1, n ganzzahlig, sind die am Ausgang des Michelsontyp-Interferometers zur Interferenz kommenden Wellenfronten gleichsinnig orientiert, also nicht invertiert. Dies stellt eine ganz wesentliche Voraussetzung für das Entstehen räumlicher Interferogramme mit hohem Kontrast von einer lateral ausgedehnten Lichtverteilung am Interferometereingang dar.

Vorzugsweise ist bei dem Fourier-Transformations-Spektrometer der Bildfeld-Diskriminator optisch der scheinbaren Endspiegelfläche der Dreifach-Winkelspiegelanordnung zugeordnet. Dazu befindet sich der Bildfeld-Diskriminator vorzugsweise zwischen dem ersten und dem dritten Spiegel dieser ersten Dreifach-Winkelspiegelanordnung. Vorzugsweise kann der Bildfeld-Diskriminator bei einer symmetrischen Anordnung auch der zweiten Spiegelfläche durch eine auf der Spiegelfläche aufgebrachte Abschattblende ausgebildet sein. Vorzugsweise kann auch die zweite Spiegelfläche durch einen schmalen Spiegel ausgebildet sein, so dass bei einem scharfen Bild auf der Spiegelfläche eine Bildfeld-Diskriminierung gegeben ist.

Im zweiten Interferometerarm besteht vorzugsweise eine Winkelspiegelanordnung als Endspiegelanordnung mit einer ungeradzahligen Anzahl von Spiegeln oder Spiegelflächen gleich drei oder größer als drei in Rachen oder in W-Form. Diese Spiegel oder Spiegelflächen stehen in der Regel senkrecht zur Referenzebene. In dieser zweiten Dreifach-Winkelspiegelanordnung ist vorzugsweise ein zweiter Bildfeld-Diskriminator angeordnet, welcher zum Bildfeld-Diskriminator im ersten Interferometerarm optisch konjugiert ist und vorzugsweise geometrisch zumindest näherungsweise gleich dem ersten Bildfeld-Diskriminator ausgebildet ist. Es ist jedoch nicht zwingend, dass die Geometrie der beiden Winkelspiegelanordnungen gleich ist.

Vorzugsweise ist bei dem Fourier-Transformations-Spektrometer dem Michelsontyp-Interferometer in jedem Arm des Interferometers eine Dreifach-Winkelspiegelanordnung angeordnet und der Bildfeld-Diskriminator ist zumindest näherungsweise dem zweiten Spiegel oder der zweiten Spiegelfläche der Dreifach-Winkelspiegelanordnung zugeordnet.

Vorzugsweise ist bei dem Fourier-Transformations-Spektrometer dieser zweite Spiegel oder diese zweite Spiegelfläche in einer Richtung parallel zur Referenzebene schmal ausgebildet und diskriminiert so das Bildfeld. Auf dem zweiten Spiegel oder der zweiten Spiegelfläche ist jeweils auch ein zumindest näherungsweise scharfes Bild vom Objekt mittels fokussierendem Abbildungssystem am Ort mindestens einer Dreifach-Winkelspiegelanordnung zumindest näherungsweise gebildet.

Vorzugsweise ist bei dem Fourier-Transformations-Spektrometer dem Michelsontyp-Interferometer in jedem Arm des Interferometers eine Dreifach-Winkelspiegelanordnung angeordnet, bei welcher sich die Lateral-Shear addiert, also im Betrag derselben eine Addition erfolgt. So kann eine besonders große Lateral-Shear bei moderater Baugröße jeder einzelnen Dreifach-Winkelspiegelanordnung erreicht werden.

Vorzugsweise ist bei dem Fourier-Transformations-Spektrometer dem Michelsontyp-Interferometer in jedem Arm des Interferometers eine Dreifach-Winkelspiegelanordnung angeordnet, bei welcher sich die Lateral-Shear weitgehend kompensiert, also im Betrag derselben eine Subtraktion erfolgt. So kann eine besonders kleine Lateral-Shear weitgehend unabhängig von der Bildfeldbreite erreicht werden.

Eine Vielzahl von räumlichen Interferogramm kann durch einen in Richtung senkrecht zur Referenzebene RE länglich ausgebildeten Bildfeld-Diskriminator erzeugt werden. Diese Richtung wird hier als Höhenrichtung definiert, welche hier die x-Richtung ist. Für jeden x-Wert gibt es nur genau ein räumliches Interferogramm. Bei einer Vielzahl von räumlichen Interferogramm wird aus jedem räumlichen Interferogramm - unter Kenntnis des x-Wertes desselben - das Spektrum mittels Fourier-Transformation errechnet und einem Bildpunkt in x-Richtung zugeordnet. Der y-Wert im Bild ergibt sich aus der aktuellen Relation zwischen Objekt und Bildfeld-Diskriminator sowie der geometrischen Ausbildung des Bildfeld-Diskriminators. Bei einer spaltförmigen Ausbildung des Bildfeld-Diskriminators mit senkrechter Lage des Spalts zur Referenzebene wird den räumlichen Interferogrammen bei einer Aufnahme mittels gerastertem Detektor jeweils der gleiche y-Wert zugewiesen. Bei Nutzung eines Bildfeld-Diskriminators mit vorzugsweiser Anordnung von Pinholes in einer Zick-Zack-Linie gibt es eine Reduzierung von optischem Übersprechen. Dabei gibt es für jeden x-Wert nur genau ein einziges Pinhole und damit nur genau ein einziges räumliches Interferogramm und die Spektren werden den entsprechenden Bildern der Pinholes in x- und in y-Richtung zugeordnet.

Vorzugsweise ist bei dem Fourier-Transformations-Spektrometer im zweiten Interferometerarm ein zweiter Bildfeld-Diskriminator angeordnet, welcher zum Bildfeld-Diskriminator im ersten Interferometerarm optisch konjugiert und geometrisch zumindest annäherungsweise gleich dem ersten Bildfeld-Diskriminator ausgebildet ist.

Vorzugsweise ist bei dem Fourier-Transformations-Spektrometer dem Michelsontyp-Interferometer mindestens ein Bildfeld-Diskriminator direkt vorgeordnet.

Vorzugsweise kann der Bildfeld-Diskriminator als Spaltblende oder in Reflexion als schmaler Spiegel- oder Spiegelflächenbereich ausgebildet sein.

Vorzugsweise kann der Bildfeld-Diskriminator als starre Anordnung ausgebildet sein.

Vorzugsweise kann der Bildfeld-Diskriminator als bewegte Anordnung in Rotation oder Translation ausgebildet sein.

Vorzugsweise kann der Bildfeld-Diskriminator auch als starres oder als bewegtes Pinhole-Array ausgebildet sein.

Vorzugsweise kann der Bildfeld-Diskriminator auch als räumlicher Lichtmodulator ausgebildet sein.

Vorzugsweise ist bei dem Fourier-Transformations-Spektrometer mindestens ein Bildfeld-Diskriminator dem Bild von einem Messobjekt im Strahlengang im Michelsontyp-Interferometer zugeordnet. Dieses Bild ist mittels vorgeordnetem Objektiv als Abbildungssystem vor dem Michelsontyp-Interferometer gebildet. Vorzugsweise kann der Bildfeld-Diskriminator als eine Spaltblende oder in Reflexion als ein schmaler Spiegel- oder Spiegelflächenbereich ausgebildet sein.

Vorzugsweise ist bei dem Fourier-Transformations-Spektrometer ein erster Bildfeld-Diskriminator dem Endspiegel und ein zweiter Bildfeld-Diskriminator der Dreifach-Winkelspiegelgruppe zugeordnet.

Vorzugsweise ist bei dem Fourier-Transformations-Spektrometer der Bildfeld-Diskriminator durch den Endspiegel oder durch einen Spiegel der Dreifach-Winkelspiegelgruppe gebildet. Vorzugsweise ist dabei der Endspiegel oder ein Spiegel der Dreifach-Winkelspiegelgruppe vergleichsweise schmal ausgebildet.

Vorzugsweise ist bei dem Fourier-Transformations-Spektrometer mindestens ein Bildfeld-Diskriminator als gerasterter Spiegel oder als eine gerasterte Spiegelfläche ausgebildet.

Vorzugsweise sind bei dem Fourier-Transformations-Spektrometer den Elementen des gerasterten Spiegels oder der gerasterten Spiegelfläche rechnersteuerbare Bewegungselemente zugeordnet.

Vorzugsweise sind bei dem Fourier-Transformations-Spektrometer bei einer Platzierung von je einem Bildfeld-Diskriminator in jedem Interferometerarm des Michelsontyp-Interferometers diese beiden Bildfeld-Diskriminatoren optisch zueinander konjugiert angeordnet.

Vorzugsweise ist bei dem Fourier-Transformations-Spektrometer mindestens ein Bildfeld-Diskriminator als spaltförmige Abschattblende ausgebildet.

Vorzugsweise ist bei dem Fourier-Transformations-Spektrometer mindestens ein Bildfeld-Diskriminator als Mikrospalt-Abschattblenden-Array ausgebildet.

Vorzugsweise kann der Bildfeld-Diskriminator als Mikrospalt-Abschattblenden-Array als eine bewegte Anordnung in Rotation oder Translation ausgebildet sein.

Vorzugsweise ist bei dem Fourier-Transformations-Spektrometer mindestens ein Bildfeld-Diskriminator als Pinhole-Abschattblende ausgebildet.

Vorzugsweise kann die Pinhole-Abschattblende als bewegte Anordnung in Rotation oder Translation ausgebildet sein. Dabei kann die Pinhole-Abschattblende bevorzugt als rotierende Kreisscheibe mit bevorzugt nur einer Spur von Pinholes ausgebildet sein. Vorzugsweise kann die Pinhole-Abschattblende beleuchtet sein, um mittels einer zugeordneten Abbildungsoptik das Objekt mit bevorzugt feinen Lichtflecken - mit Lichtrichtung weg vom Interferometer - zu beleuchten. Dies stellt eine Auflichtanordnung dar. Vorzugsweise ist diese zugeordnete Abbildungsoptik als eine mikroskopische Abbildungsstufe ausgebildet. Vorzugsweise werden die Bilder der feinen Lichtflecke vom Objekt im Rücklauf des Lichts, also mit Lichtrichtung hin zum Interferometer, an den Pinholes der Pinhole-Abschattblende, die bevorzugt als Blendenscheibe ausgebildet ist, konfokal diskriminiert, um in bekannter Art und Weise das Streulicht vom Messobjekt zu minimieren und Licht außerhalb des Schärfentiefebereiches der Lichtflecken weitgehend auszublenden. Vorzugsweise kann eine Relativbewegung zwischen Messobjekt und dem Fourier-Transformations-Spektrometer in der Tiefe durchgeführt werden, um tiefenaufgelöste Spektren, also Spektren, denen auch eine Tiefenposition im Messobjekt zugeordnet ist, zu gewinnen. So ist es möglich, entweder durch die Kombination von lateraler Bewegung der Pinhole-Abschattblende oder einer lateralen Relativbewegung zwischen Messobjekt und dem Fourier-Transformations-Spektrometer als auch einer Relativbewegung in der Tiefe zwischen Messobjekt und dem Fourier-Transformations-Spektrometer räumlich verteilten Punkten im Messobjekt spektrale Informationen zuzuordnen, so dass ein vierdimensionaler Datensatz mit drei räumlichen Koordinaten besteht. Dieser Ansatz stellt die Kombination eines konfokalen Mikroskops mit dem erfindungsgemäßen Fourier-Transformations-Spektrometer dar. Für den Terahertzbereich oder das ferne Infrarot kann diese beschriebene Kombination mit konfokaler Diskriminierung auch ins Grobe skaliert werden, so dass die Lichtflecken im Objektbereich dann bevorzugt in der Größenordnung von einem Millimeter oder beziehungsweise auch bevorzugt im Zehntelmillimeter-Bereich liegen können. Insbesondere im Terahertz-Bereich oder im fernen Infrarot-Bereich kann für Durchlichtanordnungen bevorzugt eine strukturierte Beleuchtung des Messobjekts mit feinen Lichtflecken erfolgen und mittels Bildfeld-Diskriminator eine konfokale Diskriminierung durchgeführt werden. Dabei kann die konfokale Diskriminierung vor dem Interferometer, aber auch im Interferometer erfolgen. Bevorzugt ist dann im letztgenannten Fall im Interferometer in jedem Arm des Interferometers ein Bildfeld-Diskriminator angeordnet, wobei die beiden Bildfeld-Diskriminatoren zueinander optisch konjugiert sind.

Vorzugsweise kann der Bildfeld-Diskriminator auch als räumlicher Lichtmodulator ausgebildet sein.

Zu bemerken ist hier, dass der Begriff Licht hier als Synonym für elektromagnetische Strahlung auch im infraroten, fern-infraroten und im Terahertzbereich verwendet wird, also keine Begrenzung auf den sichtbaren Spektralbereich darstellt.

Vorzugsweise ist bei dem Fourier-Transformations-Spektrometer mindestens ein Bildfeld-Diskriminator als eindimensionales oder zweidimensionales Pinhole-Abschattblenden-Array in Form einer Blendenscheibe ausgebildet. Bevorzugt kann diese Blendenscheibe zur konfokalen Diskriminierung eingesetzt werden. Bevorzugt kann diese Blendenscheibe starr angeordnet sein oder mit Bewegungsmitteln rotierend ausgebildet sein.

Vorzugsweise ist bei dem Fourier-Transformations-Spektrometer ein Mikrospalten-Abschattblenden-Array mit Mikrospalten in lateral versetzter Anordnung ausgebildet.

Vorzugsweise ist bei dem Fourier-Transformations-Spektrometer das Mikrospalten-Abschattblenden-Array mit mechanisch beweglichen Elementen ausgebildet.

Vorzugsweise ist bei dem Fourier-Transformations-Spektrometer ein feinstrukturierter Feld-Diskriminator in Spaltenform oder in Punktlinienform dem Messobjekt oder dem Feld der Lichtquelle zugeordnet. Damit können einzelne, feine Objektbereiche von besonderem Interesse erfasst werden, um vom Licht, welches nur von diesen Objektbereichen ausgeht, jeweils ein räumliches Interferogramm zu bilden. Die Längsrichtung des Feld-Diskriminators ist dabei senkrecht zur Referenzebene RE ausgerichtet.

Vorzugsweise ist bei dem Fourier-Transformations-Spektrometer die Lichtquelle selbst in Spaltenform oder in Form bevorzugt feiner leuchtender Elemente in gerader Linie oder in Zickzack-Linie feinstrukturiert ausgebildet ist und die Längsrichtung derselben, welche die x-Richtung darstellt, ist senkrecht zur Referenzebene RE ausgerichtet. Eine Anordnung leuchtender Elemente in Zickzack-Linie reduziert in der Regel das optische Übersprechen aufgrund des größeren Abstandes der leuchtenden Elemente im Vergleich zu einer Anordnung der Elemente in gerader Linie.

Vorzugsweise sind bei dem Fourier-Transformations-Spektrometer das Feld des Messobjekts und das Feld der Lichtquelle zumindest in einem Teilbereich optisch zueinander konjugiert angeordnet.

Vorzugsweise ist bei dem Fourier-Transformations-Spektrometer das Michelsontyp-Interferometer als Luft-Typ oder als Prismen-Typ oder als hybride Luft-Prismen-Anordnung ausgebildet.

Vorzugsweise ist bei dem Fourier-Transformations-Spektrometer eine konfokale Anordnung dem Michelsontyp-Interferometer vorgeordnet. Somit kann Streulicht aus einer lichtstreuenden Probe ferngehalten und auch eine Tiefendiskriminierung im Messobjekt erreicht werden.

Vorzugsweise ist bei dem Fourier-Transformations-Spektrometer die konfokale Anordnung bevorzugt mit einer starren Blendenscheibe oder einer rotierenden Blendenscheibe vor dem Michelsontyp-Interferometer ausgebildet. Hierbei wird im Michelsontyp-Interferometer selbst auf jegliche Bildfeld-Diskriminierung verzichtet, da diese bereits durch die konfokale Anordnung effektiv gegeben ist.

Vorzugsweise ist bei dem Fourier-Transformations-Spektrometer die konfokale Anordnung mit einem räumlichen Lichtmodulator in Reflexion oder Transmission ausgebildet. Dieser räumliche Lichtmodulator ist bevorzugt als ein digitales Mikrospiegel-Array oder als ein Flüssigkristall-Display ausgebildet.

Vorzugsweise sind bei dem Fourier-Transformations-Spektrometer die konfokalen Diskriminatorelemente, welche der vorgeordneten konfokalen Anordnung mit mindestens einer effektiven Spiegelfläche im Arm eines Michelsontyp-Interferometers zumindest näherungsweise optisch konjugiert. Die konfokalen Diskriminatorelemente sind bevorzugt als Pinholes oder als Mikrospiegel, bevorzugt auch als steuerbare Mikrospiegel eines digitalen Mikrospiegel-Arrays, ausgebildet.

Vorzugsweise ist bei dem Fourier-Transformations-Spektrometer mindestens ein Bildfeld-Diskriminator (BFD) im Michelsontyp-Interferometer als steuerbarer räumlicher Lichtmodulator in Reflexion ausgebildet.

Vorzugsweise ist bei dem Fourier-Transformations-Spektrometer mindestens ein Bildfeld-Diskriminator (BFD) im Fourier-Transformations-Spektrometer-System als steuerbarer räumlicher Lichtmodulator in Transmission ausgebildet.

Bevorzugt gibt es eine Relativbewegung zwischen Messobjekt und mobilem Messkopf auch in der Tiefe. Dazu sind Bewegungsmittel in der Tiefe dem Messobjekt oder dem mobilen Messkopf zugeordnet. Vorzugsweise sind Bewegungsmittel in der Tiefe auch einer Komponente des mobilen Messkopfs zugeordnet.

Es kann aber auch vorzugsweise eine abbildende Komponente des mobilen Messkopfs als brechkraftvariabel ausgebildet sein, beispielsweise als eine rechnersteuerbare Flüssiglinse.

So können Spektren mit zugeordneter Tiefeninformation vom vergleichsweise kleinen Messobjekt oder kleinen Bereichen desselben im Nahbereich gewonnen werden. Dazu erfolgt vorzugsweise einerseits eine feine strukturierte Beleuchtung des Messobjekt in Auflicht oder in Transmission mittels eines - bevorzugt länglichen - Lichtfleck-Musters. Andererseits können bevorzugt auch Bildpunkte von Pinholes einer konfokalen Anordnung, welche als eine Komponente des mobilen Messkopfs ausgebildet ist, dabei das Messobjekt beleuchten.

### Beschreibung der Figuren

Im Folgenden werden einige Ausführungsbeispiele näher beschrieben, wobei die Erfindung nicht auf die beschriebenen Ausführungsbeispiele beschränkt sein soll. Einzelne Merkmale, die in einer bestimmten Ausführungsform beschrieben werden, können beliebig kombiniert werden, vorausgesetzt, sie schließen einander nicht aus. Darüber hinaus sind verschiedene Merkmale, die in den beispielhaften Ausführungsformen zusammen bereitgestellt werden, nicht als die Erfindung einschränkend anzusehen.

Es folgt eine Beschreibung der Zeichnungen, die beispielhafte Ausfiihrungsformen zeigen:
**Fig. 1** ist eine schematische seitliche Darstellung einer beispielhaften Anwendung eines beispielhaften FT-Spektrometers an einem Patienten;
**Fig. 2** ist eine schematische Rückdarstellung einer beispielhaften Anwendung eines beispielhaften FT-Spektrometers an einem Patienten;
**Fig. 3** ist eine schematische Darstellung eines beispielhaften FT-Spektrometers, insbesondere eines Single-Shot-Linien-Spektrometers;
**Fig. 4** ist eine schematische Darstellung eines beispielhaften Michelsontyp-Interferometers;
**Fig. 5** ist eine schematische Darstellung eines beispielhaften Michelsontyp-Interferometers;
**Fig. 6** ist eine schematische Darstellung eines beispielhaften Michelsontyp-Interferometers;
**Fig. 7** ist eine schematische Darstellung einer beispielhaften Dreifach-Winkelspiegelgruppe;
**Fig. 8** ist eine schematische Darstellung eines beispielhaften Michelsontyp-Interferometers;
**Fig. 9** ist eine schematische Darstellung eines beispielhaften Michelsontyp-Interferometers;
**Fig. 10** ist eine schematische Darstellung einer beispielhaften Dreifach-Winkelspiegelgruppe;
**Fig. 11** ist eine schematische Darstellung einer beispielhaften asymmetrischen Dreifach-Winkelspiegelgruppe;
**Fig. 12** ist eine schematische Darstellung einer beispielhaften symmetrischen Dreifach-Winkelspiegelgruppe;
**Fig. 13** ist eine schematische Darstellung beispielhafter scheinbarer Bildpunkte mit nachgeordnetem, anamorphotischen Objektiv;
**Fig. 14** ist eine schematische Darstellung beispielhafter scheinbarer Bildpunkte mit nachgeordnetem, anamorphotischen Objektiv;
**Fig. 15** ist eine schematische Darstellung einer beispielhaften Anwendung eines beispielhaften FT-Spektrometers;
**Fig. 16** ist eine schematische Darstellung eines beispielhaften FT-Spektrometers;
**Fig. 17** ist eine schematische Darstellung eines beispielhaften FT-Spektrometers;
**Fig. 18** ist eine schematische Darstellung eines beispielhaften FT-Spektrometers;
**Fig. 19** ist eine schematische Darstellung eines beispielhaften FT-Spektrometers;
**Fig. 20** ist eine schematische Darstellung eines beispielhaften FT-Spektrometers.

Es erfolgt eine Beschreibung mittels 20 Figuren und von drei Ausführungsbeispielen ohne Figur (nicht schematisch anhand einer Zeichnung dargestellt). Der Begriff Licht wird in der folgenden Beschreibung in der Regel als Synonym für elektromagnetische Strahlung vom UV- bis zum Terahertz-Bereich, einschließlich von Wärmestrahlung, verwendet.

Der Begriff Spektrometersystem steht hier insbesondere für ein kompaktes Single-Shot-Fourier-Transformations-Spektrometer mit einem Michelsontyp-Interferometer, welches eine Lateral-Shear s erzeugt. Dabei erfolgt insbesondere entweder eine aktive Beleuchtung des Messobjekts, welche vorzugsweise auch eine strukturierte Beleuchtung sein kann, oder das Messobjekt ist selbstleuchtend. Der Begriff "Single-Shot" bezieht sich hier auf die Gewinnung von räumlichen Interferogrammen mittels der Zweistrahl-Interferometrie.

Bei einem Single-Shot-Verfahren führt der Detektor - hier in der Regel ein gerasterter Matrix-Detektor im FT-Spektrometer - nach einem externen oder internen digitalen Startbefehl für den Matrix-Detektor jeweils eine einzelne Bildaufnahme durch. Mittels Michelsontyp-Interferometer besteht auf dem Matrix-Detektor eine Anzahl nebeneinander angeordneter räumlicher Interferogramme. Die Anzahl derselben liegt beispielsweise bei üblichen Matrix-Detektoren in der Größenordnung von Einhundert bis Eintausend. Die räumlichen Interferogramme auf einem Kamerabild gehören in der Regel jeweils zu einem linienhaften Teilbereich des Messobjekt. Zumindest handelt es sich um einen selektierten Teilbereich des Messobjekts mittels räumlicher Diskriminierung. Die räumlichen Interferogramme werden mittels schneller Fourier-Transformation (FFT) numerisch zu Spektren verrechnet, gegebenenfalls auch erst nach einer Zwischenspeicherung. Die schnelle Fourier-Transformation (englisch fast Fourier transform, daher meist FFT abgekürzt) ist ein Algorithmus zur effizienten Berechnung der diskreten Fourier-Transformation (DFT).

Die Aufnahmezeit, also das Zeitfenster für den Single-Shot, ergibt sich dabei in der Regel aus der Integrationszeit (allgemeiner: Bildaufnahmezeit) des gerasterten Matrix-Detektors, die sich je nach Detektortyp und Lichtverhältnissen vom einstelligen Mikrosekundenbereich bis in den dreistelligen Millisekundenbereich erstrecken kann. Im Extremfall kann sich bei extrem geringer Lichtenergie und vergleichsweise sehr geringer Dynamik im Messobjekt die Integrationszeit auch bis in den einstelligen Sekundenbereich erstrecken. Bei Blitzbeleuchtung oder einer gepulsten Beleuchtung des Messobjekts, Synchronisation vorausgesetzt, definiert jedoch die Blitz- oder Pulsdauer das Zeitfenster für den Single-Shot wie auch analog die Offenzeit einer gesteuerten Blende, vorausgesetzt, dass diese jeweils kürzer als die o. g. Integrationszeit des Matrix-Detektors sind.

Mehrere Aufnahmen des gerasterten Matrix-Detektors in möglichst schneller Folge, beispielhaft mit einer Aufnahme-Frequenz von 60Hz, führen dann zu vielen benachbarten, in der Regel linienhaften Teilbereichen eines Messobjekts, so dass mittels Relativbewegung nach und nach zu jedem auflösbaren Flächeninkrement eines flächenhaften Messobjekts ein Spektrum vorliegen kann und so ein hyperspektrales Bild gegeben ist. Die erreichbare laterale Auflösung im hyperspektralen Bild ergibt sich dabei bekannterweise aus den Parametern der optischen Komponenten im abbildenden System und ggf. auch der Rasterkonstante des (gerasterten) Matrix-Detektors des FT-Spektrometers. Moderne Hochgeschwindigkeits-Matrix-Detektoren ermöglichen auch Integrationszeiten (Bildaufnahmezeit) im Bereich von 10 Mikrosekunden, ggf. auch noch darunter.

Die Fig. 1 stellt eine Anwendung eines beispielhaften FT-Spektrometers dar. Fig. 1 stellt schematisch insbesondere eine Anordnung eines Patienten mit Schutzbrille dar, wobei ein aus medizinischer Sicht auffälligen Hautmerkmal 2 auf dem Rücken 1 des Patienten untersucht werden soll. Insbesondere soll mittels Diagnostik die Frage geklärt werden, ob es sich bei dem Hautmerkmal 2 um ein Muttermal oder ein Melanom handelt. Die Diagnose soll insbesondere auch mittels einer spektralen Messung mittels eines Spektrometersystems 20 möglichst objektiv abgesichert werden. Dazu dient für den Dermatologen zum Haut-Screening ein im Wesentlichen mobiler Messkopf 30 im Single-Shot-Betrieb, welcher als Hand-held-Gerät für einen Scan über ein Messobjekt, hier der Bereich eines Rückens 1, ausgeführt ist. Diesem Gerät ist insbesondere eine gepulste NIR-Lichtquelle 40 zur im Wesentlichen aktiven Beleuchtung in Streifenform und/oder zur feinen strukturierten Beleuchtung des Rückens 1 und eine Optik-Einheit 50 mit einem integrierten Michelsontyp-Interferometer 601 beigeordnet.

Dieser im Wesentlichen mobile Messkopf 30 wird insbesondere relativ langsam, z.B. mit einer Geschwindigkeit etwa v=1cm/s von Hand über den Rücken 1 des Patienten, hier beispielsweise von unten nach oben also in +y-Richtung, geführt.

Die Integration einer VIS-Monitorkamera in den mobilen Messkopf 30 für die Hautoberfläche unter Diagnose auf dem Rücken 1 ist hier gegeben, jedoch nicht dargestellt. Die mittels dieser Monitorkamera aufgenommenen Bilddaten dienen insbesondere zusätzlich der Unterstützung beim Aufbau eines hyperspektralen Bildes, insbesondere dann, wenn die Scan-Bewegung des mobilen Messkopfes 30 aus der Hand nicht mit einer im Wesentlichen konstanten Geschwindigkeit erfolgt. Dazu sind auf dem Rücken 1 hier zwei nicht dargestellte Positionsmarken aufgebracht. Die im Scan bei einer moderaten Bewegung des mobilen Messkopfes 30 nach und nach anfallenden räumlichen Interferogramme rI, die in der Regel im Single-Shot-Modus mittels einer InGaAs-Kamera 54 für den nahinfraroten Spektralbereich hier entlang einer Linie in waagerechter Richtung gewonnen werden, werden insbesondere in Spektren SP umgerechnet und zu einem hyperspektralen Bilddatensatz zusammengesetzt. Die InGaAs-Kamera 54 ist bevorzugt wie auch die gepulste NIR-Lichtquelle 40 durch den Rechner 21 steuerbar ausgebildet. Es erfolgt eine Synchronisation dieser beiden Komponenten (40 und 54) durch den Rechner 21.

Die mittels Interferometer 601 in der Regel "in einem Schuss" aufgenommenen räumlichen Interferogramme rI können also bevorzugt mittels eines Programmes 101 zur Ausführung der schnellen Fourier-Transformation (FFT) zu Spektren entlang dieser Linie verrechnet werden. Dies erfolgt insbesondere linienweise, also zeilenweise, für den auf dem Rücken 1 ausgewählten Hautbereich, welcher in der Breite von der Optik-Einheit 50 des mobilen Messkopfes 30 erfasst werden kann. Nach Beendigung der Aufnahmen von räumlichen Interferogrammen rI in einer Aufwärtsbewegung, die das Hautgebiet von diagnostischem Interesse abdeckt, können die errechneten Spektren punktweise einer Bildkarte des untersuchten Hautgebiets zugeordnet werden, so dass nach und nach ein hyperspektrales Bild aufgebaut wird. Dabei wird es hier als zulässig angesehen, wenn das Bildpunktraster aufgrund der nicht perfekt bzw. nicht optimal gleichmäßigen und nicht perfekt lateral geführten Handbewegung des Dermatologen bei Benutzung des mobilen Messkopfes 30 gewisse Dehnungen und Stauchungen aufweist, jedoch ohne Lücken ist. Mittels eines Programmes 22 zur Bewertung der Spektren SP, ausgeführt auf einem leistungsfähigen Rechner 21 und/oder Rechnersystem, können aufgrund der spektralen Merkmale derselben insbesondere Risiko- und Hochrisikobereiche für eine Hautkrebserkrankung ermittelt werden.

Dabei können Algorithmen zur Spektrenauswertung, beispielsweise nach dem Principal Component Analysis-Ansatz, zur Anwendung oder auch Ansätze mit künstlicher Intelligenz in Betracht kommen. Die Algorithmik zur Spektrenauswertung hinsichtlich der Abschätzung eines Tumor-Risikos steht im Rahmen dieser Erfindung nicht im Fokus, da es hier insbesondere um die schnelle Bereitstellung von optischen Primärdaten, also räumlichen Interferogrammen rI, geht. Das Ergebnis der Spektrenauswertung kann auf einem Monitor 23 dargestellt werden.

Anders als in **Fig. 1** dargestellt, erfolgt in einem weiteren Ausführungsbeispiel 1 ohne Figur (also nicht schematisch anhand einer Zeichnung dargestellt) die Beleuchtung im Wesentlichen koaxial durch die Einkopplung des Lichts mittels eines Strahlteilers. Nur dann ist eine im Wesentlichen stete Überdeckung eines projizierten Lichtstreifens und des Messfeldes in beliebiger Positionierung desselben gegeben. Dann kann auch im Wesentlichen problemlos eine Zoom-Funktion in die Optik-Einheit 50 des mobilen Messkopfes 30 integriert werden. Der Ansatz mit koaxialer Einkopplung des Lichts zur Beleuchtung des Rückens 1 kommt beispielsweise in der **Fig. 3** zum Einsatz.

Die **Fig. 2** stellt schematisch die Rückenansicht des Patienten dar. Der projizierte beispielhafte Lichtstreifen 80, welcher mittels NIR-Streifenlichtquelle auf dem Rücken 1 des Patienten erzeugt wird, ist deutlich länger als das Messfeld 81 der Optik-Einheit 50 des mobilen Messkopfes 30 ausgebildet. Die Länge des Messfeldes 81 ist L'. Somit ist auch bei moderaten Abstandsänderungen des mobilen Messkopfes 30 eine im Wesentlichen vollständige Ausleuchtung des Messfeldes gegeben. Beim Messen kann die Bewegung des mobilen Messkopfes 30 insbesondere aus der Hand und lateral über einen Bereich des Rückens erfolgen. Dabei können mittels des Michelsontyp-Interferometers 601 in der Optik-Einheit 50 ständig räumliche Interferogramme rI im Single-Shot-Modus aufgenommen werden, wobei von der Lichtquelle 40 synchronisiert geblitztes Licht zwecks Beleuchtung des Messfeldes 81 in Form eines Streifens 80 erzeugt werden kann. Da in der **Fig. 1** eine Geradeaus-Anordnung, also eine im Wesentlichen entfaltete Anordnung, dargestellt ist, entsprechen die Koordinatenrichtungen y und x auf dem Rücken 1 eines Menschen denen des Michelsontyp-Interferometers 601. Ein derartiger mobilen Messkopf 30 kann bei einer chirurgischen Operation unter Narkose auch zur Gewebedifferenzierung eingesetzt werden.

Die **Fig. 3** stellt das Prinzip des Ansatzes für ein Single-Shot-Linien-Spektrometer mit einer Beleuchtung des Rückens 1 eines Menschen mit einem Streifen 80 dar. Mittels eines Transportschlittens 90 mit einem hier nicht dargestellten Schrittmotorantrieb kann hier ein y-Scan erfolgen. Dieser y-Scan mobilen Messkopfes 30 kann insbesondere quer zum Streifen 80 über den Rücken 1 eines ruhig sitzenden Menschen erfolgen. Der Rücken 1 kann dabei mittels einer gepulsten Streifenlichtquelle 43 in Form eines Streifen 80 im Wesentlichen intensiv beleuchtet werden. Zur koaxialen Einkopplung des Lichts in die Optik-Einheit 50 des mobilen Messkopfes 30 dient in diesem Beispiel ein Einkoppelstrahlteilerwürfel 57. So kann das Messfeld 81 mittels Streifen 80 ausgeleuchtet werden. Der so beleuchtete Bereich auf dem Rücken 1 kann mittels eines vorgeordneten Objektivs 70 im Wesentlichen scharf in das Michelsontyp-Interferometer 601 durch viele einzelne Strahlenbündel abgebildet werden. Insbesondere kann zu jedem Objektpunkt ein Strahlenbündel gehören. Repräsentativ ist ein Strahlenbündel mit durchgezogenen Linien dargestellt, welches zu einem Objektpunkt O auf der optischen Achse gehören kann. In gepunkteten Linien sind in Fig. 3, repräsentativ für mehrere Strahlenbündel, drei weitere Strahlenbündel eingezeichnet.

Hierbei besteht für die Objektabbildung zumindest näherungsweise Telezentrie auf der dem Michelsontyp-Interferometer 601 zugewandten Seite des vorgeordneten Objektivs 70 mit der optischen Achse OAI, jedoch hier ohne Darstellung einer Telezentrieblende. Weiterhin kann das vorgeordnete Objektiv 70 eine Autofokus-Funktion aufweisen. Die Autofokus-Funktion kann mittels dieser ermittelten Objektentfernung bei der Aufnahme ständig dem Auswerteprogramm als Information übergeben bzw. übermittelt werden, da eine Abstandsänderung in der Regel den Abbildungsmaßstab bei der Abbildung verändert, was bei der Auswertung und Darstellung des hyperspektralen Bildes bevorzugt berücksichtigt wird. Das Michelsontyp-Interferometer 601 erzeugt eine Lateral-Shear s und weist in jedem der beiden Interferometerarme einen jeweils baugleichen Bildfeld-Diskriminator BFD1 und BFD2 auf, die in der Detailabbildung 3.1 als Bildfeld-Diskriminatoren BFD1 und BFD2 jeweils mit der Breite b und der Länge L dargestellt sind. In **Fig. 3** sind in symbolischer Darstellung - und perspektivisch zur Sichtbarmachung herausgedreht - die scheinbaren Bilder BFD1's und BFD2's der Bildfeld-Diskriminatoren BFD1 und BFD2 im ebenfalls symbolisch dargestellten Michelsontyp-Interferometer 601 in der scheinbaren Bildebene SBE12 eingezeichnet.

Mindestens ein Punkt jeweils eines dieser scheinbaren Bilder BFD1 's und BFD2's der Bildfeld-Diskriminatoren BFD1 und BFD2 ist mit jeweils einem scheinbaren Bildpunkt O'1s und O'2s des Objektpunktes O optisch konjugiert, wobei die scheinbaren Bildpunkte O'1s und O'2s durch Strahlteilung im Michelsontyp-Interferometer 601 optisch kohärent, also interferenzfähig, sind und im Fall der präzisen Justierung desselben gemeinsam in der scheinbaren Bildebene SBE12 - jedoch separiert mit einer Lateral-Shear s - liegen. Die scheinbare Bildebene SBE12 stellt hier für das nachgeordnete anamorphotische Objektiv 51 wiederum die Objektebene dar. Am Ausgang des Zweistrahl-Interferometers 601 entstehen paarweise kohärente Teilstrahlenbündel, die beispielhaft hier in den Figuren durch zwei Teilstrahlenbündel TB1 und TB2 dargestellt sind, welche jedoch in der Regel repräsentativ für viele Teilstrahlenbündel sind.

Die beiden Bildfeld-Diskriminatoren BFD1 und BFD2 sind im Zweistrahl-Interferometer 601 so angeordnet, dass eine optische Konjugation für diese besteht, d. h. , dass diese vom Eingang des Interferometers aus in das Interferometer hinein gesehen optisch nicht mehr unterscheidbar sind. Die Bildfeld-Diskriminatoren BFD1 und BFD2 sind dabei lateral so ausgedehnt, dass trotz Diskriminierung immer noch mehrere Strahlenbündel von mehreren Objektpunkten diese passieren können. Im Verständnis gehört immer ein Punkt zu einem Strahlenbündel. Auf dem Bildfeld-Diskriminator BFD1 entsteht durch Abbildung ein Bild 80'1 und auf dem Bildfeld-Diskriminator BFD2 entsteht durch Abbildung ein Bild 80'2 vom Streifen 80. Von jedem dieser beiden Bilder 80'1 und 80'2 kann in jedem Interferometerarm im Wesentlichen nur ein Teil des Lichts ein Feld der Breite b und der Länge L die Diskriminatoren BFD1 und BFD2 passieren. Dieser Teil des Lichts ist der selektierte Lichtanteil. Licht außerhalb dieses Feldes wird von der weiteren Abbildung ausgeschlossen, geht also erwünscht verloren. Durch die Rückabbildung der beiden Bildfeld-Diskriminatoren BFD1 und BFD2 sind auf dem Rücken 1 über den Abbildungsmaßstab des vorgeordneten Objektivs 70 die Breite des Messfeldes b' sowie deren Länge L' festgelegt.

In der Detailabbildung 3.2 ist das Messfeld 81 mit der Breite b' und der Länge L' dargestellt. Hier in der Detailabbildung 3.2 sind, beispielhaft und stellvertretend für viele Objektelemente, jedoch nur vier angemessene kleinflächige Objektelemente, nämlich OE1 bis OE4, mit zur besseren Sichtbarmachung einer vergrößerten Höhe dargestellt. Die reale Anzahl der Objektelemente liegt dagegen typischerweise in der Größenordnung von etwa 500 im MIR-Spektralbereich, entsprechend des Einsatzes einer MIR-Kamera mit näherungsweise etwa 500 Pixeln in x-Richtung. Für jedes Objektelement OE wird im Messverfahren genau ein Spektrum Sp ermittelt, wenn dieses Objektelement OE für eine Messung hinreichend kooperativ ist. Die Breite des Messfeldes b' beträgt auf dem Rücken 1 hier etwa 0,2mm. Die Breite des Messfeldes b' kann insbesondere zwischen etwa 0,05mm und 20mm liegen. Außerdem sind in der Detailabbildung 3.2 die beiden zusammenfallenden reellen Bilder BFD1'r und BFD2'r der beiden Bildfeld-Diskriminatoren BFD1 und BFD2 andeutungsweise dargestellt, die sich durch eine gedachte bzw. fiktive Rückabbildung mittels des vorgeordneten Objektivs 70 auf den Rücken 1 ergeben.

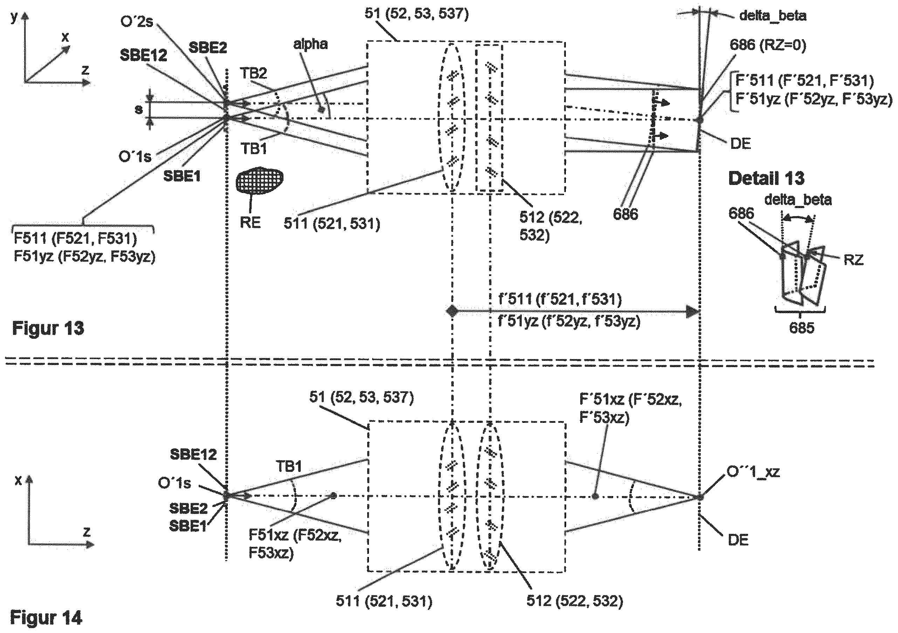

Die beleuchteten Bildfeld-Diskriminatoren BFD1 und BFD2, welche im Wesentlichen zwei kohärente Lichtquellen darstellen, werden von einem dem Michelsontyp-Interferometer 601 nachgeordneten anamorphotischen Objektiv 51, welches auch bevorzugt chromatisch korrigiert ist, am Ausgang desselben abgebildet. Durch die Ausbildung des Objektivs 51, welches neben einer rotatorischen Komponente 511 auch mit einer Zylinderkomponente 512 ausgebildet ist, entstehen jeweils im Wesentlichen zwei zueinander geneigte Zylinderwellen, die hier als gerasterten Detektor auf einen Detektor, insbesondere auf eine InGaAs-Kamera 54 treffen, wobei jedes Paar von Zylinderwellen jeweils ein räumliches Interferogramm rI bildet, welches einem Objektelement OE zugeordnet ist. Die Zylinderwellenfronten 385 sind in der Detailabbildung 3.3 mit den zueinander um delta_beta geneigten Scheitellinien 386 dargestellt. Die spezielle optische Funktion des anamorphotischen Objektivs 51 ist in **Fig. 13** und **14** anschaulich sichtbar gemacht. Die präzise Justierung des Interferometers einschließlich des Abgleichs der optischen Weglängen der Arme des Michelsontyp-Interferometers 601 ist bekannt. Dabei ist zu bemerken, dass aufgrund des vergleichsweise geringen Wertes der Breite b von etwa 0,2mm die Anforderungen an die Justierung eines Endspiegels oder einer Dreifach-Winkelspiegelgruppe im Michelsontyp-Interferometer 601 hier nicht sehr hoch sind, was einen besonderen Vorteil hinsichtlich Robustheit darstellt, beispielsweise bei rauen Umgebungsbedingungen und mit vergleichsweise schnellen Temperaturwechseln. Je geringer der Wert b gewählt ist, desto unkritischer ist die Justierung des Interferometers 601. Auch eine Kippung eines Interferometerendspiegels um eine Kippachse im Michelsontyp-Interferometer 601, bei welcher zur Referenzebene RE Parallelität besteht, ist vergleichsweise unkritisch. Diese Unempfindlichkeit ergibt sich aus der Ortsauflösung der räumlichen Interferogramme rI mittels gerastertem Empfänger. In der Detailabbildung 3.4 sind einige räumliche Interferogramme rI dargestellt.

Die bei einer dennoch nicht ganz perfekten Justierung des Interferometers 601 etwas im Feld variierenden optischen Gangunterschiede in den räumlichen Interferogrammen rI stellen für die numerische Auswertung derselben beim Stand der Technik kein Problem dar.

Nur wenn der Kontrast der räumlichen Interferogramme rI hinreichend gut ist, kann ein hohes Signal/Rauschverhältnis im Spektrum erreicht werden. Es ist deshalb sehr wichtig, durch die Interferometer-Hardware einen möglichst hohen Kontrast der räumlichen Interferogramme rI zu sichern, da die gesuchten spektralen Signaturen, insbesondere in biologischen Messobjekten, ohnehin oft nicht sehr ausgeprägt sind.

In diesem Ausführungsbeispiel nach **Fig. 3** können die Bildfeld-Diskriminatoren BFD1 und BFD2 auch schmale Spiegel der Breite b und mit großer Länge L darstellen. Licht außerhalb des schmalen Spiegels gerät in Lichtfallen oder auf eine mattschwarze Maskierung des schmalen Spiegels, welche die Breite b aufweist.

Die Detailabbildung 3.5 stellt errechnete Spektren Sp1 bis Sp4 aus einer beispielhaften Aufnahme dar, die gemäß Detailabbildung 3.5 hier der x-Koordinate gemäß Detailabbildung 6 zugeordnet werden. Mittels y-Scan des kann der bekannte Datenwürfel in der Form (x, y, Wellenzahl) oder (x, y, Wellenlänge) für einen Bereich des Rückens 1 bestimmt werden.

In einem weiteren Ausführungsbeispiel 2 ohne Figur wird ein Mehrachsen-Roboterarm zur Bewegung des mobilen Messkopfes 30 eingesetzt. Dieser schafft noch wesentlich mehr Flexibilität als ein linear arbeitender Transportschlitten 90 und ist bei einer chirurgischen Operation aufgrund seiner Flexibilität der Positionierung vor Ort oft von größtem Vorteil.

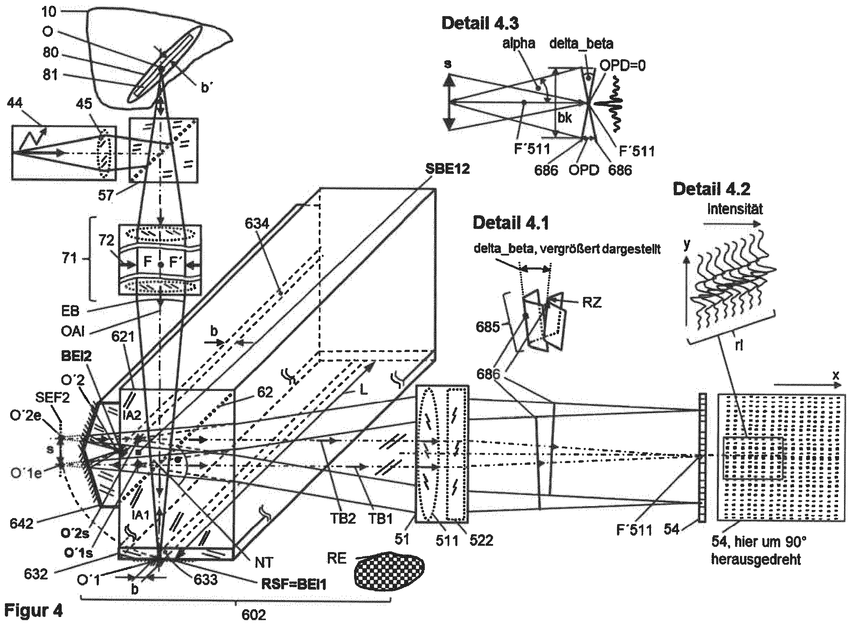

Die Fig. 4 zeigt ein besonders kompaktes beispielhaftes Michelsontyp-Interferometer 602, das insbesondere für den NIR-Bereich ausgelegt ist. Die Interferometeranordnung ist hier recht langgestreckt ausgebildet, um bei den entsprechenden vor- und nachgeordneten Objektiven 71 und 51, die aus Gründen der Darstellbarkeit hier verkleinert gezeichnet sind, das hier vergleichsweise lange Messfeld der Länge L, insbesondere langgestreckt ausgebildet in x-Richtung, in einem Schuss erfassen zu können. Die gepulste Lichtquelle 44 dient insbesondere zur Projektion eines angepasst langen Streifens 80 auf das Messobjekt 10, beispielhaft mit einer Länge von 20 mm, und besitzt dazu eine integrierte Abbildungsoptik 45. Das Licht wird mittels Einkoppelstrahlteiler 57 im Wesentlichen koaxial in den Beleuchtungsstrahlengang eingekoppelt und dient der Beleuchtung des Messobjekts 10 in Form eines Lichtstreifens 80. Das von einem Lichtpunkt O, stellvertretend für die leuchtenden Objektpunkte, vom beleuchteten Messobjekt 10 auf der Achse ausgehende Licht gelangt nach nochmaligem Passieren des Einkoppelstrahlteilers 57 in Transmission über die telezentrische Abbildungsstufe 71 als im Wesentlichen fokussiertes Einfallsbündel EB in das Michelsontyp-Interferometer. Die hier nur symbolisch und recht klein dargestellte Abbildungsstufe 71 weist in den zusammenfallenden Fokusebenen eine telezentrische Blende 72 auf. Das Michelsontyp-Interferometer 602 ist hier mittels gekittetem Glasblock 621, der hier jedoch stark vergrößert dargestellt ist, mit einer Strahlteilerschicht 62 ausgebildet. An derselben erfolgt die Bündelaufspaltung in die beiden Arme IA1 und IA2 des Michelsontyp-Interferometers 602. Das hindurchgehende Teilstrahlenbündel trifft im Arm IA1 auf den schmalen Planpiegel 633 mit äußerer Schutzschicht, der auf einer Spiegelplatte aufgebracht ist, deren Dicke angepasst ist. Der Planpiegel 633 definiert im Wesentlichen die reelle Spiegelfläche RSF, welche weitestgehend deckungsgleich mit dem Planpiegel 633 ist.

Dieser Planpiegel 633 steht in der Bildebene im Michelsontyp-Interferometer 602 bei der Abbildung eines Teils des beleuchteten Messobjekts 10 und stellt den ersten Bildfeld-Diskriminator BFD1 dar, dessen Rückabbildung das Messfeld 81 auf dem Messobjekt 10 bestimmt.

Das an der Strahlteilerschicht 62 reflektierte Teilstrahlenbündel gelangt in die Dreifach-Winkelspiegelgruppe 642, beispielhaft ausgebildet als Prismenanordnung in W-Form, in welcher sich der schmale Spiegel 634 befindet. Dieser schmale Spiegel 634, beispielhaft mit einer Breite von 1 mm, steht in der Bildebene im Michelsontyp-Interferometer 602 bei der Abbildung des beleuchteten Messfeldes mittels Streifen 80. Dieser Spiegel stellt 634 den zweiten Bildfeld-Diskriminator dar, dessen Rückabbildung die Messfeldgröße auf dem Messobjekt 10 bestimmt, da die beiden Spiegel sich in Positionen befinden, die optisch zueinander konjugiert sind. Darüber hinaus sind diese Spiegel 633 und 634 viel breiter als das Airy-Scheibchen, beispielhaft mit einer Breite von bis zu 1 mm, ausgelegt, so dass vergleichsweise viel Licht zur Detektion kommen kann und somit ein schnelles Messen, beispielhaft mit einer Bildaufnahmerate von 100 Hz, möglich ist. Es erfolgt also eine Bildfeld-Diskriminierung oder -Begrenzung im Michelsontyp-Interferometer 602 selbst. Bei Bedarf nach einer sehr hohen lateralen Auflösung, beispielhaft von 0,1 mm auf dem Objekt, können auch zwei sehr schmale Spiegel 633 und 634, beispielhaft mit einer Breite von deutlich weniger als 0,1 mm, als Bildfeld-Diskriminatoren eingesetzt werden.

Das vom schmalen Spiegel 633 reflektierte Licht als Teilstrahlenbündel TB1 und das von der Dreifach-Winkelspiegelgruppe 642 zurückkommende bzw. zurückreflektierte Teilstrahlenbündel TB2, nun mit einer Lateral-Shear s, gelangen über eine anamorphotische Abbildungsstufe 51, auch mit Zylinderkomponente 522, zur Interferenz von Zylinderwellen 685. Diese Zylinderwellen 685 sind in der Detailabbildung 4.1 dargestellt und dienen der Erzeugung räumlicher Interferogramme rI. Die Detektion der räumlichen Interferogramme rI erfolgt mittels eines Detektors, bevorzugt mittels einer InGaAs-Kamera 54 für den NIR-Bereich. Der Winkel delta_beta zwischen den interferierenden zylindrischen Wellenfronten 685, also der Winkel delta_beta zwischen den Scheitellinien 686, ist hier stark vergrößert dargestellt. Das Licht von den kohärenten und scheinbaren Lichtquellenpunkten O'1s und O'2s in der scheinbaren Bildebene SBE12 führt jeweils zur Generierung eines räumlichen Interferogramms rI. Dieses räumliche Interferogramm rI ist hier im Wesentlichen mittig auf dem Chip der InGaAs-Kamera 54, da hier in der Mitte des Chips bei entsprechender Justierung des Interferometers der Ort mit dem optischen Gangunterschieds etwa null ist. Hier ist das räumliche Interferometer rI hinsichtlich seiner optischen Weglängen in den beiden Interferometerarmen IA1 und IA2 präzise abgeglichen. Ein Messobjekt-Scan-Mechanismus für die y-Richtung ist vorhanden, aber hier nicht dargestellt.

Zylinderwellen stellen - im Gegensatz zu Kugelwellen - in der Form Ausschnitte von Zylinderflächen mit jeweils einer Scheitellinie dar.

Der maximal erreichbare optische Gangunterschied (OPD) im Michelsontyp-Interferometer kann mittels der Darstellung in der Detailabbildung 4.3 angenähert errechnet werden. Wird hier von einer numerischen Apertur A=sin(alpha) von etwa 0,1 ausgegangen, was einem Aperturwinkel alpha in Luft von etwa etwa 5,7 Altgrad entspricht und einen vergleichsweise geringen Wert darstellt, ergibt sich bei einer Lateral-Shear etwa s= 1,04mm ein daraus folgender maximaler Gangunterschied OPD auf dem Chip der InGaAS-Kamera 54 in der Detektionsebene DE des Objektivs 51 mit OPD=A* s zu näherungsweise zu 0,104mm. Dabei liegt das räumliche Interferogramm im Wesentlichen zentriert auf dem Chip. Mit einer angenommenen Dreieck-Apodisation der Intensitätswerte des räumlichen Interferogramms rI bei Berechnung des Spektrums mittels einer schnellen Fourier-Transformation kann mit dem reziproken Wert des optischen Gangunterschieds OPD, hier etwa 1/0,102mm, eine spektrale Auflösung von näherungsweise 96cm⁻¹ erreicht werden. Das entspricht bei einer Wellenlänge von etwa 1000nm einer spektralen Auflösung im Wellenlängenbereich von etwa 9,6nm. Für eine Brennweite f'511 von etwa 60mm des Objektivs 511 ergibt sich mit etwa a=2A* f 511 die benötigte minimale Kantenlänge der InGaAs-Kamera 54 zu etwa 15,0mm. Die InGaAs-Kamera Goldeye P-032 SWIR von Allied Vision besitzt eine Breite bk von etwa 15,9mm und einen Pixel-Pitch von etwa 25µm und etwa 636 x 508 Pixel. So kann ein symmetrisches, räumliches Interferogramm rI mit dem optischen Gangunterschied von etwa 0,104mm im Spektralbereich von etwa 900nm bis 1700nm unter Einhaltung des Abtast-Theorems vollständig aufgenommen werden, da die Interferenzstreifen der kürzesten Wellenlänge von etwa 900nm auf dem Kamera-Chip hier mit näherungsweise etwa 52µm Breite breiter als der doppelte Pixel-Pitch dieses InGaAs-Kamera-Chips sind. Die Interferenzstreifenbreite ergibt sich näherungsweise aus dem Quotienten f'511 dividiert durch die Lateral-Shear s und multipliziert mit der jeweiligen Wellenlänge.

Mit dieser Anordnung kann unter sorgfältiger Beachtung der physiologischen Bedingungen auch eine Messung am menschlichen Auge durchgeführt werden.

Mit einer derartigen Anordnung mit entsprechenden Modifikationen ist darüber hinaus auch das Spektrum von Fluoreszenzlicht messbar, insbesondere wenn eine Anregungslichtquelle im ultravioletten Spektralbereich genutzt wird.

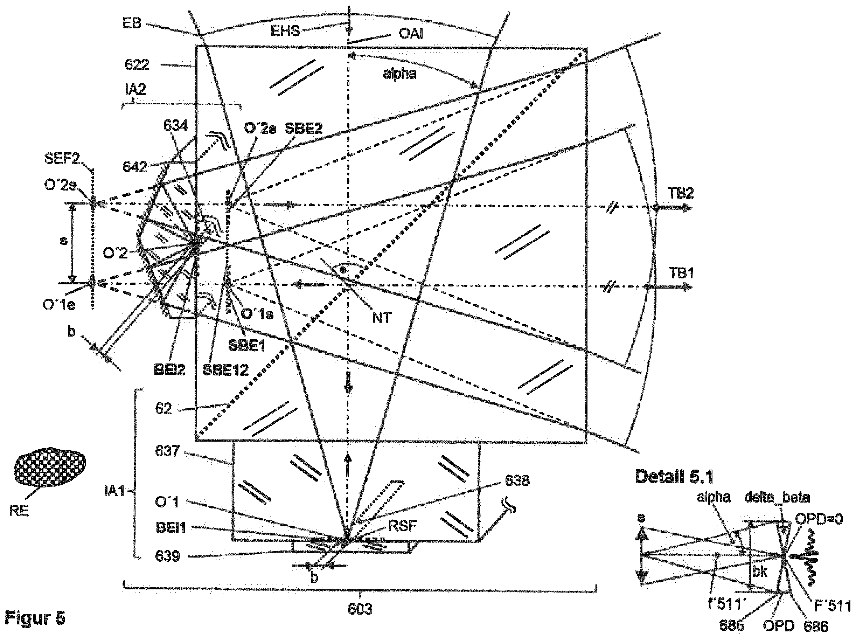

Die **Fig. 5** soll das Potenzial für einen vergleichsweise großen Aperturwinkel alpha eines einfallenden Strahlenbündels EB der Anordnung mit Strahlteilerwürfel 622 und Ausgleichsplatte 637 mit schmaler Spiegelfläche 638 aufzeigen. Die schmale Spiegelfläche 638, beispielhaft hier mit einer Breite b von 0,2 mm, stellt eine reelle Spiegelfläche RSF in der Bildebene BEI1 dar und ist auch der Bildfeld-Diskriminator BFD1 in der Bildebene BEI1. Im Interferometerarm IA2 ist beispielhaft eine Dreifach-Winkelspiegelgruppe 642 angeordnet, welche als Prismenanordnung in W-Form ausgebildet ist. Es wird hier im Michelsontyp-Interferometer 603 ein maximaler Aperturwinkel von bis zu etwa 15 Altgrad, also im refraktiven Material erreicht. Dies stellt einen vergleichsweise großen Aperturwinkel dar. Dies ist jedoch mit einem nur schmalen Messfeld der Breite b von 0,2 mm zu erreichen. Zu beachten ist, dass der Streifenspiegel 634 im Wesentlichen nicht achssenkrecht im Strahlengang angeordnet ist und es so, insbesondere bei großen Streifenbreiten b, beispielhaft oberhalb von 0,2 mm, eine gewisse unscharfe Begrenzung des Messfeldes im Interferometerarm IA2 geben kann.

Der maximal erreichbare optische Gangunterschied (OPD) im Michelsontyp-Interferometer 603 kann mittels der Darstellung in der Detailabbildung 5.1 angenähert errechnet werden, wobei hier von einer Anwendung im VIS-Bereich ausgegangen wird. Das hier nicht dargestellte anamorphotische Objektiv 51, siehe dazu **Fig. 13** und **14****,** welches das Objektiv 511 enthält, ist ebenfalls für den sichtbaren Spektralbereich ausgelegt. Es wird hier eine CMOS-Kamera 55 mit näherungsweise 20 Millionen Pixeln zur Detektion eingesetzt. Wird hier für das Rechenbeispiel von einer numerischen Apertur A=sin(alpha) in Luft des anamorphotischen Objektivs 51 von etwa A=0,2 ausgegangen, was einem Aperturwinkel alpha in Luft von etwa 11,5 Altgrad entspricht, ergibt sich bei einer Lateral-Shear etwa s=1,6mm ein daraus folgender maximaler Gangunterschied OPD auf dem Chip der hier nicht dargestellten CMOS-Kamera 55 in der Detektionsebene DE des Objektivs 51 mit OPD=A^{∗} s zu näherungsweise 0,32mm. Dabei liegt das räumliche Interferogramm zentriert auf dem Chip der CMOS-Kamera 55. Mit einer angenommenen Dreieck-Apodisation der Intensitätswerte des räumlichen Interferogramms rI bei Berechnung des Spektrums mittels einer schnellen Fourier-Transformation kann mit dem reziproken Wert des optischen Gangunterschieds OPD, hier etwa 1/0,32mm, eine spektrale Auflösung von näherungsweise 31,3cm⁻¹ erreicht werden. Das entspricht bei einer Wellenlänge von etwa 400nm einer spektralen Auflösung im Wellenlängenbereich von etwa 0,5nm. Für eine Brennweite f'511 von 20mm des Objektivs 511 ergibt sich mit a=2A* f'511 die benötigte minimale Kantenlänge der CMOS-Kamera 55 zu etwa 8,0mm. Eine beispielhafte CMOS-Kamera mit etwa 20 Millionen Pixel besitzt eine Breite bk von etwa 8mm und einen Pixel-Pitch von etwa 2µm und etwa 4000 Pixel hier in der Breitenrichtung (y). So kann ein symmetrisches, räumliches Interferogramm rI mit dem optischen Gangunterschied von etwa 0,32mm im Spektralbereich von etwa 400nm bis 900nm unter Einhaltung des Abtast-Theorems vollständig aufgenommen werden, da die Interferenzstreifen der kürzesten Wellenlänge von etwa 400nm auf dem Kamera-Chip hier mit einer Interferenzstreifenbreite von näherungsweise 5µm breiter als der doppelte Pixel-Pitch von etwa 2µm dieses CMOS-Kamera-Chips 55 sind. Die Interferenzstreifenbreite ergibt sich näherungsweise aus dem Quotienten f' 511 dividiert durch s und multipliziert mit der Wellenlänge.

Die **Fig. 6** stellt eine weitere Anordnung eines Michelsontyp-Interferometers 604 mit einer Dreifach-Winkelspiegelgruppe 643, ausgebildet als Prismenanordnung in Rachen-Form, also einen Dreifach-Planspiegel-Prismenreflektor dar. Die linienhaften Spiegel 633 und 635 wirken hier als Bildfeld-Diskriminatoren. Auch hier ist der Aperturwinkel alpha im refraktiven Material vergleichsweise groß. Der Dreifach-Planspiegel-Prismenreflektor 643 kann insbesondere auch in einem Michelsontyp-Interferometer nach **Fig. 4** oder **Fig. 5** im sichtbaren und/oder im nah-infraroten Spektralbereich unter Nutzung geeigneter refraktiver Materialien eingesetzt werden.

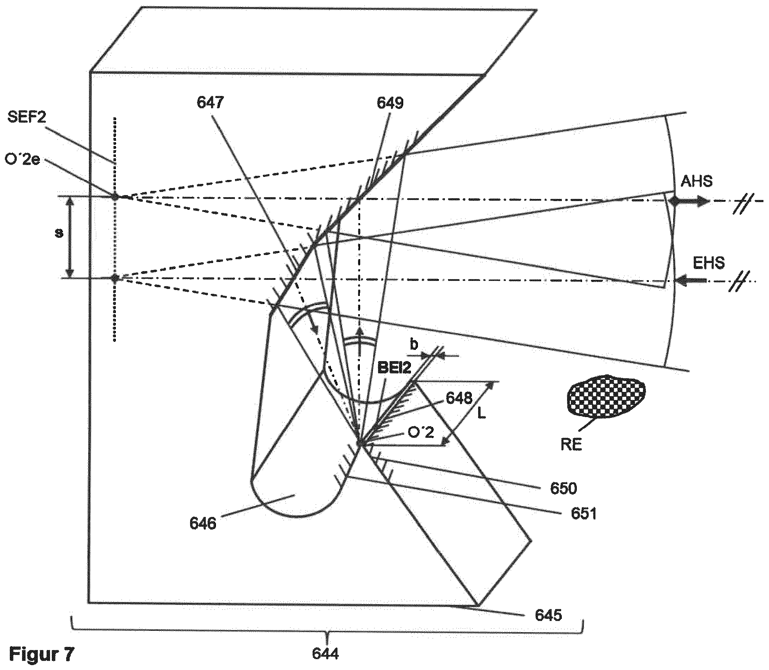

In **Fig. 7** ist eine Dreifach-Winkelspiegelgruppe 644 in einem Metallblock 645 dargestellt, welche einen linienhaften Spiegel 648 mit der Breite b aufweist, der hier sehr schmal ausgebildet ist, beispielhaft mit einer Breite von 0,1 mm und einer Länge von 10 mm. So kann eine effektive eindimensionale Bildfeld-Diskriminierung erreicht werden.

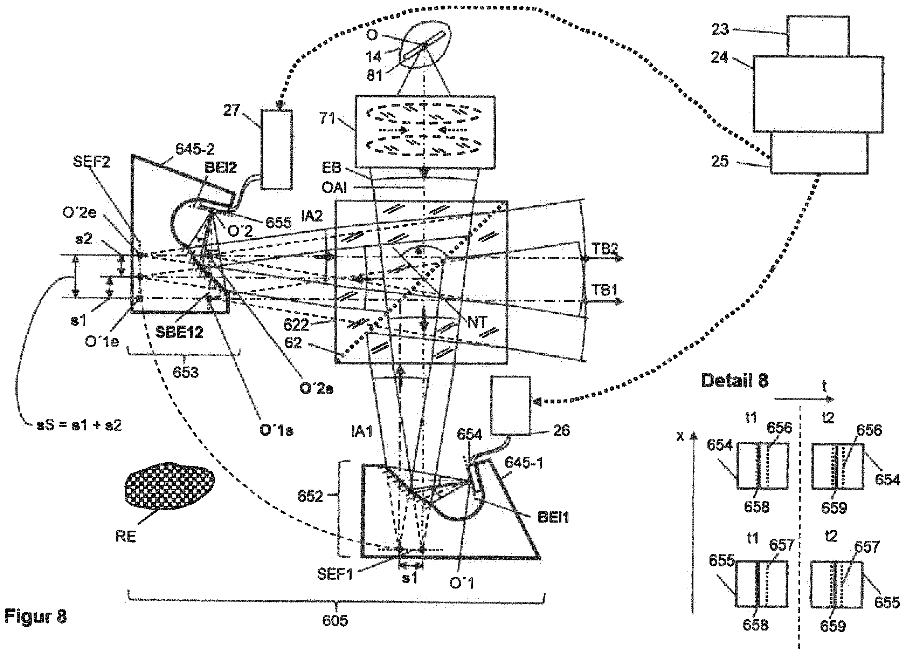

In der **Fig. 8** sind die beiden Dreifach-Winkelspiegelgruppen 652 und 653 in je einem Metallblock 645-1 und 645-2 so angeordnet, dass die resultierende Lateral-Shear sS sich durch die Addition der Beträge der Lateral-Shear s1 und der Lateral-Shear s2 ergibt. Hier in **Fig. 8** ist die scheinbare Bildebene SBE12 mit den scheinbaren Bildpunkten O'1s und O'2s zur verdeutlichenden Darstellung eingezeichnet. Mittels Detail 8 soll verdeutlicht werden, dass die beiden digitalen Mikrospiegel-Arrays 654 und 655 synchronisiert betrieben werden. Diese stellen die Bildfelddiskriminatoren BFD1 und BFD2 dar. So werden zu einem Zeitpunkt t1, t2, [...], tn jeweils dieselben streifenförmigen Bildausschnitte mittels der spiegelnden, einprogrammierten Bereiche 658 und 659 durch Synchronisation der beiden digitalen Mikrospiegel-Arrays 654 und 655 diskriminiert, also erfasst, wodurch - bei sukzessiver und synchronisierter Verschiebung der streifenförmigen Bildausschnitte - zeitseriell ein Messobjekt nach und nach vollständig abgetastet wird. Dies stellt einen internen Scan der beiden optisch konjugierten Bilder vom Messobjekt in den optisch konjugierten Bildebenen BEI1 und BEI2 dar. So kann mittels einer Anordnung nach Figur 8 auf eine Relativbewegung zwischen einem Messobjekt und dem FT-Spektrometer ganz verzichtet werden und dennoch ein hyperspektrales Vollbild durch den internen Scan gewonnen werden. Dies setzt jedoch eine vergleichsweise geringe Dynamik im Messobjekt in Bezug auf die Bildrate eines hier nicht dargestellten gerasterten Detektors voraus, die im Kilohertz-Bereich liegen kann. So ist es möglich, von Vorgängen, die "im Schneckentempo" auflaufen - wie beispielhaft langsam fließendes, glühendes Magma als Messobjekt - eine hyperspektrale Video-Sequenz von diesem glühenden Magma erzeugen. Bei geeigneten Lichtverhältnissen kann hier auch eine Hochgeschwindigkeitskamera - beispielsweise mit einer Bildwiederholungsrate von 10 kHz - als gerasterter Detektor eingesetzt werden.

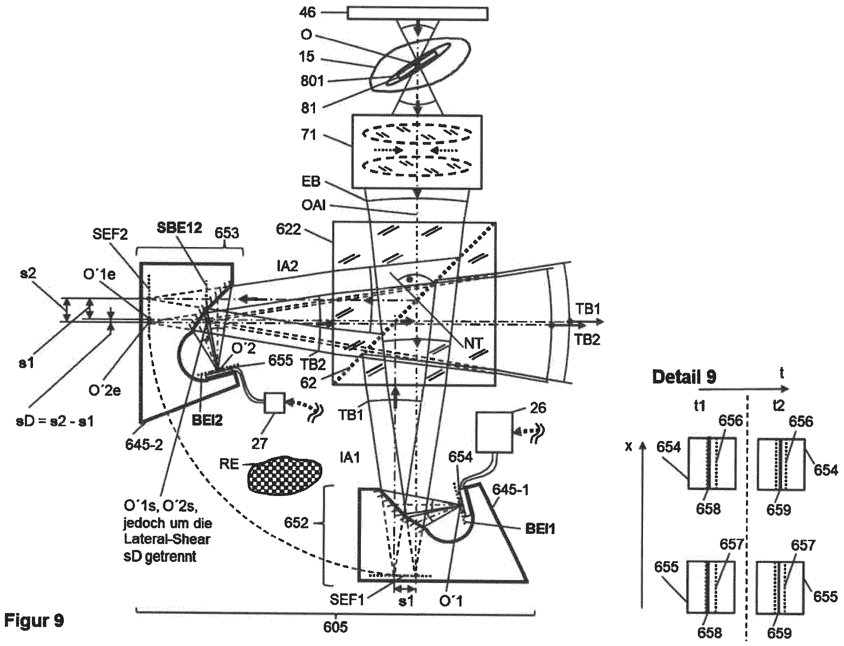

In der **Fig. 9** sind die beiden Dreifach-Winkelspiegelgruppen 652 und 653 in je einem Metallblock 645-1 und 645-2 in einem Michelsontyp-Interferometer 605 so angeordnet, dass die resultierende Lateral-Shear sD sich teilweise kompensiert und sich durch die Differenz der Beträge der Lateral-Shear s1 und der Lateral-Shear s2 ergibt. Bei der Dreifach-Winkelspiegelgruppe 652 ist deshalb die Rachengeometrie etwas kleiner ausgebildet. Damit der optische Gangunterschied dennoch kompensiert bleibt, muss die Dreifach-Winkelspiegelgruppe 652 im Interferometerarm IA1 dafür einen etwas größeren Abstand vom Strahlteilerwürfel 622 mit der Strahlteilerschicht 62 aufweisen und zwar in der Größenordnung von einigen Zehntelmillimetern. Die **Fig. 9** stellt nur aus Gründen der Darstellung die Wellenfronten am Ausgang des Michelsontyp-Interferometers 605 in der Tiefe etwas versetzt dar. In **Fig. 9** wurde auf die Darstellung von Datenleitungen völlig verzichtet, da diese denen in der **Fig. 8** entsprechen. Hier in **Fig. 9** sind die scheinbaren und dabei um die Lateral-Shear sD nur wenig getrennten Bildpunkte O'1s und O'2s und auch die entfaltete Bildebene SBE12 eingezeichnet. Das Detail 9 entspricht dem Detail 8 in Figur 8 und ist dort beschrieben.

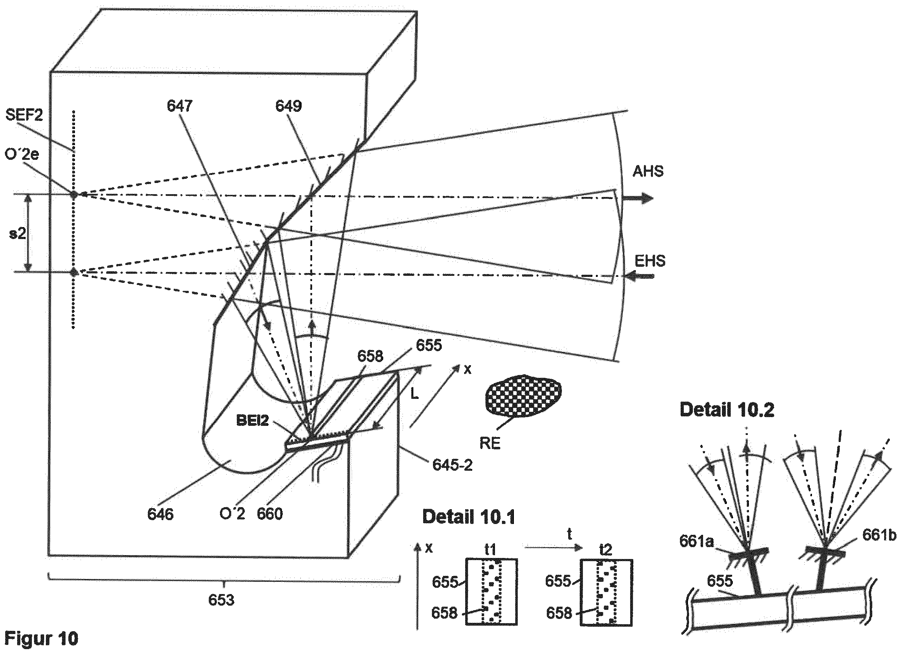

Die **Fig. 10** stellt noch einmal die zweite Dreifach-Winkelspiegelgruppe 653 in einem modifizierten Metallblock 645-2 dar. Eine Spiegelfläche ist mittels digitalem Mikrospiegel-Array 655 gebildet. So sind verschiedene Muster für die Bild-Diskriminierung in ein digitales Mikrospiegel-Array 655 einprogrammierbar, was in der Detailabbildung 10.1 symbolisch dargestellt ist. Die in der Detailabbildung 10.2 links dargestellte Stellung eines Mikrospiegels 661a des digitalen Mikrospiegel-Arrays 655 wird zur Reflexion des weiterhin genutzten und somit zur Detektion gebrachten, also selektierten Lichts benutzt. Die rechts dargestellte Stellung eines Mikrospiegels 661b des digitalen Mikrospiegel-Arrays 655 führt zum Wegreflektieren des Lichts. In der **Fig. 10** wurde auf die Darstellung von Datenleitungen völlig verzichtet, da diese denen in der Figuren 8 entspricht.

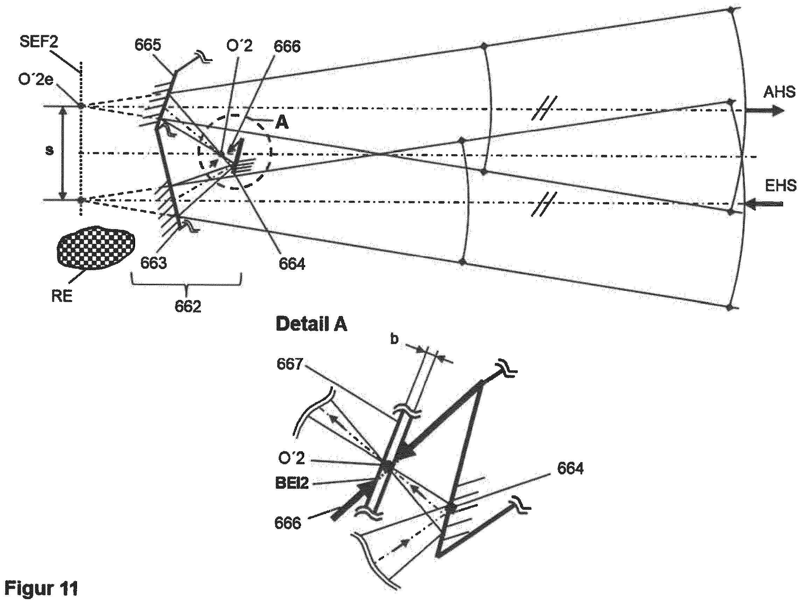

**Fig. 11** zeigt eine im Wesentlichen asymmetrische Dreifach-Winkelspiegelgruppe 662 mit einer Spaltblende 666 mit einem Spalt 667. Der Bildpunkt O'2 liegt hierbei im Spalt 667 in Luft und somit an einer für die Bildfeld-Diskriminierung geeigneten Stelle. Eine Spaltblende 666 ist bei der Bildfeld-Diskriminierung sehr effektiv, da diese außerhalb des freien Spaltes das Licht im Wesentlichen total blockiert.

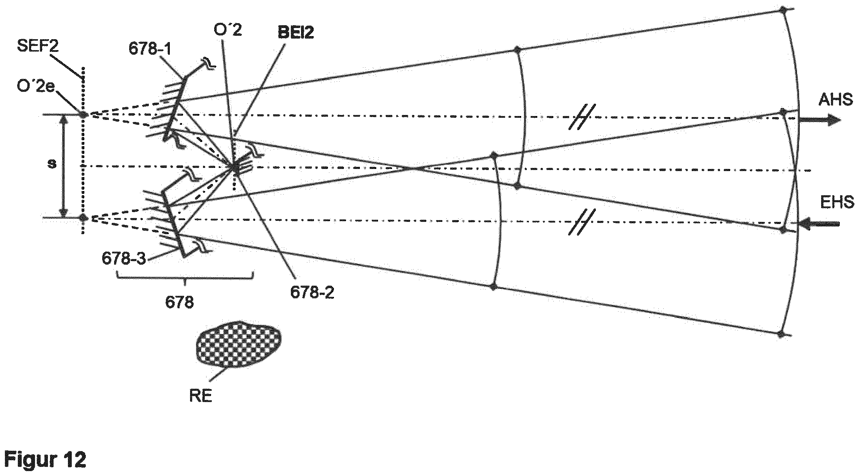

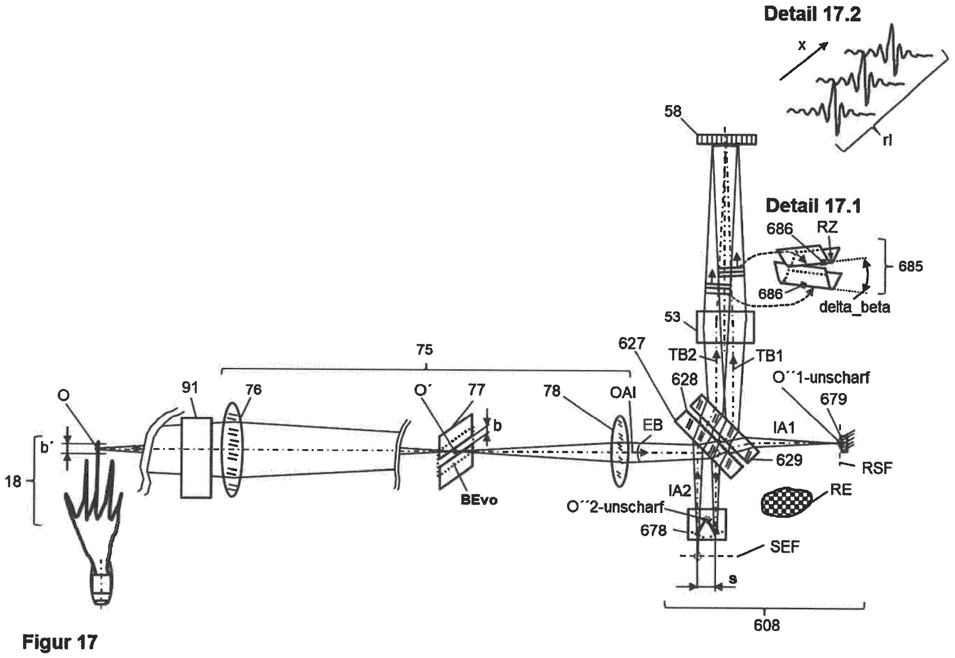

**Fig. 12** stellt eine im Wesentlichen symmetrische Dreifach-Winkelspiegelgruppe 678 ohne Spaltblende dar. Diese zusammengesetzte symmetrische Dreifach-Winkelspiegelgruppe ist als Anordnung in W-Form in Luft und für den MIR-Bereich ausgebildet. Die Bildfeld-Diskriminierung erfolgt hier mittels des zweiten Planspiegels der Dreifach-Winkelspiegelgruppe 678-2, der hier schmal ausgebildet ist und hier beispielhaft eine Breite von 0,2 mm aufweist. Diese Dreifach-Winkelspiegelgruppe 678 ist in einem Michelsontyp-Interferometer 608 in **Fig. 17** eingesetzt.

Die Figuren 13 und 14 stellen die Abbildungseigenschaften eines dem Michelsontyp-Interferometer nachgeordneten, anamorphotischen und weitgehend achromatischen Objektivs 51 zur Detektion räumlicher Interferogramme rI dar. Dabei wird die Funktion dieses Objektivs 51 in der Messanordnung aus der **Fig. 4** erläutert.

Die Zeichenebene in **Fig. 13** ist die yz-Ebene und enthält die scheinbaren Bildpunkt O'1s und O'2s, die sich in der scheinbaren Bildebene SBE12 befinden, in welcher im Wesentlichen auch die beiden scheinbaren Bildebene SBE1 und SBE12 liegen. Die scheinbare Bildebene SBE12 enthält im Wesentlichen auch den Brennpunkt F511 des anamorphotischen Objektivs 51. Die scheinbaren Bildpunkte O'1s und O'2s stellen durch Amplitudenteilung im Interferometer im Wesentlichen optisch kohärente Bildpunkte dar, welche um die Lateral-Shear s voeneinander getrennt sind. In der yz-Ebene, muss für den gesamten genutzten Spektralbereich des Fourier-Spektrometers eine besonders gute Korrektur der Aberrationen erfolgen und zwar, ausgehend von Objekt bis zum gerasterten Detektor, damit die räumlichen Interferogramme weitgehend ungestört sind. Diese hier beschriebenen Abbildungseigenschaften weisen prinzipiell auch die anamorphotischen Objektive 52 und 53 auf, die deshalb in Klammern angegeben sind, sowie auch das nachgeordnete Spiegelobjektiv 537, welches aus den Freiformflächen 675, 676 und 677 gebildet ist.

Die Zeichenebene in **Fig. 14** ist die xz-Ebene und enthält den Bildpunkt O"1_xz, der in die Detektorebene DE mittels des Objektivs 51 abgebildet wird. In der xz-Ebene ist die Brechkraft zumindest näherungsweise doppelt so groß wie die in der yz-Ebene. Der gerasterte Detektor, hier die InGaAs-Kamera 54, steht in der Detektionsebene DE. Diese hier dargestellten Abbildungseigenschaften gelten auch für die weiteren nachgeordneten, anamorphotischen und weitgehend achromatischen Objektive 52 und 53, die deshalb in Klammern angegeben sind, sowie auch für das nachgeordnete Spiegelobjektiv 537.

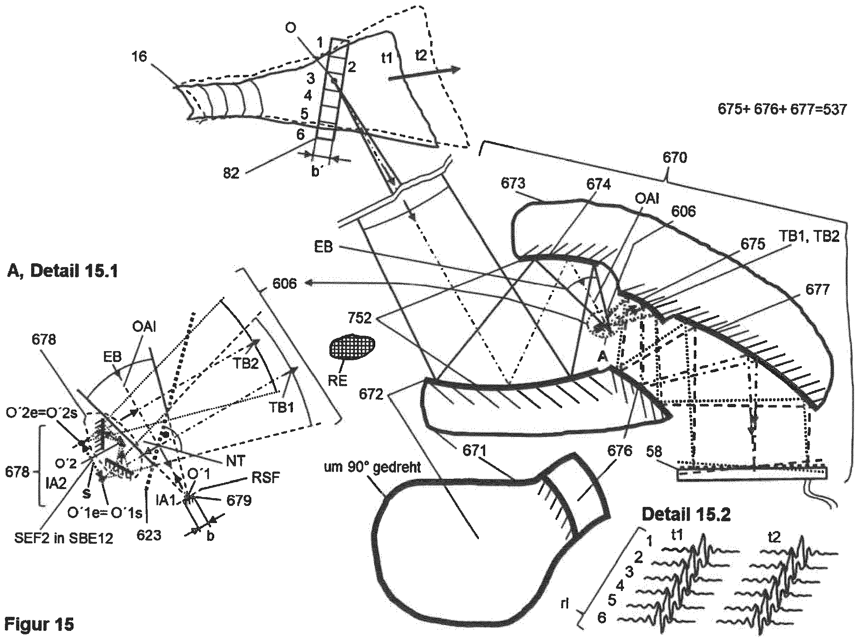

Die **Fig. 15** zeigt eine strömende heiße Abgaswolke 16 oberhalb einer Fabrikanlage zum Zeitpunkt t1, welche sich hier von links nach rechts bewegt und dabei sowohl spektral im fern-infraroten Spektralbereich als auch ortsaufgelöst untersucht werden soll. Dazu wird im Spektrometersystem am Boden eine Anordnung 670 für die Analyse der heißen Abgaswolke 16 in einer größeren Entfernung eingesetzt. Diese Anordnung 670 ist mit einem Michelsontyp-Interferometer 606 mit den Spiegelblöcken 671 und 673 ausgebildet.

Hierbei spielt bei der Messung und bei der Auswertung die Bildinformation über die Abgaswolke 16 selbst eine eher untergeordnete Rolle, da die Objektform derselben bei der Analyse von Luftschadstoffen von geringerem Interesse ist. Vielmehr soll hier nur näherungsweise ortsaufgelöst die spektrale Zusammensetzung ermittelt werden, die insbesondere auf Schadstoffe schließen lässt. Dabei soll jedoch in keinem Fall ein Nichterfassen, Übersehen oder Durchschlüpfen von wesentlicher spektraler Information vorkommen, die beispielsweise giftige Komponenten kenntlich macht. Wo örtlich ganz genau diese giftige Komponente sich in der Abgaswolke befindet, ist zunächst nicht von allererstem Interesse. Es genügt in vielen Fällen sicher schon, wenn man die spektrale Information, welche auf Schadstoffe hinweist, wenigstens, beispielsweise unter Kenntnis der aktuellen Windrichtung, einem einzelnen Schornstein einer Fabrikanlage, welche die Abgaswolke erzeugt, zuordnen kann.

Die von der Abgaswolke 16, insbesondere im fern-infraroten Spektralbereich emittierte Strahlung erreicht den Spiegelblock 671 mit der spiegelnden Freiformfläche 672 zur Fokussierung der ankommenden Strahlung. Der Spiegelblock 671 ist verkleinert dargestellt. Nach Reflexion und weiterer Fokussierung mittels einer zweiten spiegelnden Freiformfläche 674 auf dem Spiegelblock 673 tritt die Strahlung in das Michelsontyp-Interferometer 606 ein. Die scheinbare Endspiegelfläche SEF2 liegt hierbei in sowohl der scheinbaren Bildebene SBE2 als auch in der scheinbaren Bildebene SBE12, welcher auch die scheinbare Bildebene SBE1 zugeordnet ist.

Die räumlichen Interferogramme entstehen nach Strahlformung mittels der zweiten spiegelnden Freiformfläche 675 sowie mittels der spiegelnden Freiformfläche 676 und mittels der dritten spiegelnden Freiformfläche 677 auf dem Bolometer-Array 58. Die drei spiegelnden Freiformflächen 675, 676 und 677 bilden das anamorphotisches Spiegelobjektiv 537.

In einem Ausführungsbeispiel 3 ohne Figur auf der Basis von **Fig. 15** sind anstelle der beiden starren Spiegel in den Armen des Michelsontyp-Interferometers zwei digitale Mikrospiegel-Arrays eingesetzt, die bei der Bewegung der Abgaswolke 16 diese durch Rechnersteuerung synchronisiert nachverfolgen und dabei jeweils zumindest näherungsweise denselben Objektausschnitt zur Detektion kommen lassen. So kann das Messergebnis unter Inkaufnahme gewisser Unschärfen in der Ortsauflösung der Abgaswolke 16 durch Akkumulation von Interferogrammen, welche das Signal-Rauschverhältnis erhöht, signifikant verbessert werden. Die Information zur Nachverfolgung und Rechnersteuerung der digitalen Mikrospiegel-Arrays in Echtzeit werden mittels Kamera-Monitoring aus der erfassten Bewegung der Abgaswolke 16 gewonnen.

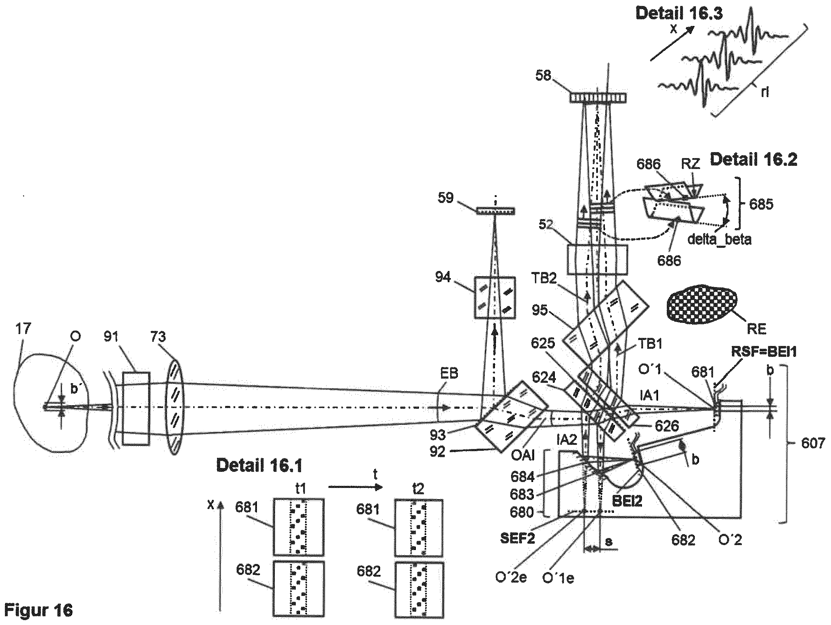

Die **Fig. 16** zeigt ein unzugängliches, organisches Messobjekt 17 in mittlerer Entfernung, welches Wärmestrahlung aussendet. Dieses wird mittels eines 2D-Spiegel-Scanners 91 zeitseriell in schmalen Feldern abgetastet. Das vom Messobjekt 17 in Form von Wärmestrahlung erfasste Licht gelangt durch ein vorgeordnetes, das Messobjekt 17 teilweise abbildendes Objektiv 73, das für den MIR-Spektralbereich ausgelegt ist, über eine geneigte CaF2-Platte 92 zur Kompensation von Astigmatismus in das Michelsontyp-Interferometer 607 für den MIR-Spektralbereich. In diesem Michelsontyp-Interferometer 607 sind eine Strahlteilerplatte 624 mit der Strahlteilerschicht 625 und eine Kompensationsplatte 626 angeordnet. Beide Platten können jeweils CaF2 aufweisen und insbesondere aus CaF2 bestehen. Die aktiven Endreflektoren für den MIR-Bereich sind durch ein erstes digitales Mikrospiegel-Array 681 insbesondere mit Goldbeschichtung und ein zweites digitales Mikrospiegel-Array 682 insbesondere auch mit Goldbeschichtung gebildet. Die Platten 92 und 95, welche ebenfalls bevorzugt aus CaF2 gefertigt sein können, werden im Wesentlichen nur zur Kompensation von Astigmatismus benötigt, der durch die stark geneigten Strahlteilerplatten entsteht. Der verbleibende Öffnungsfehler ist in das Objektiv 73 einkorrigiert. Es erfolgt eine Diskriminierung des Bildfeldes am ersten digitalen Mikrospiegel-Array 681 im Michelsontyp-Interferometer 607. Da bei der Dreifachspiegel-Anordnung kein im Wesentlichen senkrechter Strahleinfall auf das Mikrospiegel-Array 682 besteht, ist nur ein vergleichsweise schmales Feld in lateraler Richtung möglich und die Diskriminierung des Bildfeldes ist auf dem digitalen Mikrospiegel-Array 682 zumindest in den Randbereichen ggf. nicht ganz perfekt. In der Detailabbildung 16.1 werden die - in die digitalen Mikrospiegel-Arrays 681 und 682 einprogrammierten - aktiven Bereiche dargestellt. Für jeden x-Bereich gibt es nur einen aktiven Bereich, hier schwarz gekennzeichnet, von welchem Licht mittels Bolometer-Matrix-Detektor 58 anschließend zur Detektion kommt.

Am Ausgang des Michelsontyp-Interferometers 607 entstehen mittels eines anamorphotischen Objektivs 52, welches auch mit Zylinderkomponente für den MIR-Spektralbereich mit CaF2 ausgebildet ist, von jedem aktiven Bereich ein Paar kohärenter Zylinderwellen. Diese Zylinderwellen gehen von zueinander kohärenten Bildpunkten O"1 und O"2 aus, die um die Lateral-Shear s voneinander im Wesentlichen separiert sind. Zwei ausgewählte Bildpunkte O"1 und O"2 sind hier im entfalteten Zustand der optischen Anordnung in Bezug auf das anamorphotische Objektiv 52 dargestellt. Diese zueinander geneigten Zylinderwellen 685 sind in der Detailabbildung 16.2 dargestellt und kommen auf einem Bolometer-Matrix-Detektor 58 zur Interferenz, wobei hier der Krümmungsradius RZ der Zylinderwellen 685 sehr klein ist, beispielhaft in der Größenordnung von weniger als 20 Mikrometer. Zu verschiedenen Zeitpunkten t1, t2, ... werden verschiedene aktive Bereiche in die beiden digitalen Mikrospiegel-Arrays 681 und 682 eingeschrieben, so dass sich über der Zeit eine vollständige Erfassung des schmalen Feldes ergibt. Nach vollständiger Erfassung desselben wird mittels 2D-Spiegel-Scanner 91 ein weiteres schmales Feld erfasst, beispielsweise ein benachbartes oder eines von besonderem Interesse. Die Detailabbildung 16.3 zeigt räumliche Interferogramme rI, die zu einzelnen aktiven Bereichen eines schmalen Feldes gehören. Es soll noch einmal festgestellt werden, dass die Diskriminierung des Bildfeldes hier im Michelsontyp-Interferometer erfolgt. Diese Vorgehensweise gestattet zeitseriell die Gewinnung eines hyperspektralen Bildes vom Messobjekt 17.

In **Fig. 16** wurde ebenfalls auf die Darstellung von Datenleitungen völlig verzichtet. Diese Datenleitungen entsprechen im Wesentlichen denen aus der **Fig. 8****.**

Während in den vorangegangenen Figuren die Bildfeld-Diskriminierung in der Regel innerhalb des Michelsontyp-Interferometers erfolgte, wird einschließlich ab **Fig. 17** der Ansatz mit einer Bildfeld-Diskriminierung vor dem Interferometer dargestellt. Demzufolge gibt es keine Bildfeld-Diskriminierung im Michelsontyp-Interferometer 608, jedoch die Entstehung von zwei Bildern, welche aufgrund von Aberrationen auch recht unscharf sein können. Dazu wird eine zweistufige Voroptik 75 insbesondere für den mittel-infraroten Spektralbereich (MIR) mit einem ersten Objektiv 76, einem Spaltblenden-Diskriminator 77 sowie einem zweiten Objektiv 78 eingesetzt. Der Spaltblenden-Diskriminator 77 steht in der Bildebene BEvo vor dem Michelsontyp-Interferometer 608. Das Messobjekt in **Fig. 17** ist hier beispielhaft eine Triebwerksflamme 18 in der Entfernung von einigen Metern vom Messsystem, welche sich in einem stationären Betrieb befindet. Die heißen Abgase derselben strahlen auch im MIR-Spektralbereich ab und sollen im MIR-Spektralbereich zum einen spektral als auch mit deren Entstehungsorten erfasst werden. Somit können die ermittelten Spektren im Bild den jeweiligen räumlichen Bereichen zugeordnet und ein hyperspektrales Bild zumindest teilweise erstellt werden.

Ein besonderer Vorteil dieser Anordnung nach **Fig. 17** besteht darin, dass die ungeradzahlige Anzahl von Reflexionen in den jeweils beiden Armen des Michelsontyp-Interferometers 608, nämlich eine Reflexion im ersten Interferometerarm IA1 und drei Reflexionen im zweiten Interferometerarm IA2, nach Verlassen des Michelsontyp-Interferometers 608 den Astigmatismus auf einen Öffnungsfehler hin reduziert. Dazu ist das Michelsontyp-Interferometers 608 für das MIR mit einem Strahlteiler bevorzugt aus einer KBr-Strahlteilerplatte 627 und einer KBR-Kompensationsplatte 629 und einer Strahlteilerschicht 628 aufgebaut. Weiterhin sind passive Endreflektoren für den MIR-Bereich im Michelsontyp-Interferometer 608 angeordnet. Diese Endreflektoren sind bevorzugt in Form eines schmalen vergoldeten Planspiegel 679 im ersten Arm und einer zusammengesetzten Dreifach-Winkelspiegelgruppe 678 bevorzugt mit vergoldeten Planspiegelflächen im zweiten Arm des Michelsontyp-Interferometers 608 dargestellt. Letztere ist als symmetrische Anordnung in W-Form und in Luft ausgebildet.

Diese Reduktion der Aberration erfolgt aber erst am Ausgang des Michelsontyp-Interferometers 608 nach zweimaliger Passage der Strahlteilerbaugruppe mit den KBr-Substraten 627 und 629. Deshalb gibt es keine scharfe Bildentstehung im Michelsontyp-Interferometer 608 und somit ist eine Bildfeld-Diskriminierung dort nicht gut möglich. Deshalb wird in diesem Fall eine Bildfeld-Diskriminierung nicht im Michelsontyp-Interferometer 608, sondern bereits vor dem Strahleintritt in das Interferometer durchgeführt, was demzufolge jedoch eine zusätzliche Abbildungsstufe 75 mit einem Bildfeld-Diskriminator erforderlich macht. Dieser Bildfeld-Diskriminator ist hier ein dem Michelsontyp-Interferometer 608 vorgeordneter Spaltblenden-Diskriminator 77, welcher in der vorgeordneten Bildebene BEvo positioniert ist. Die mittels eines anamorphotischen Spiegelobjektivs 53, insbesondere optimiert für den MIR-Spektralbereich und auch mit einem hier nicht dargestellten Zylinderspiegel, erzeugten Zylinderwellen 685, dargestellt in der Detailabbildung 17.1, kommen auf einem Bolometer-Matrix-Detektor 58 zur Zweistrahl-Interferenz. Für jede x-Position gibt es ein räumliches Interferogramm rI, symbolisch dargestellt in der Detailabbildung 17.2. Die im Michelsontyp-Interferometer 608 bestehenden Öffnungsfehler sind im anamorphotischen Spiegelobjektiv 53 einkorrigiert, so dass weitgehend ungestörte Zylinderwellen zur Interferenz kommen.

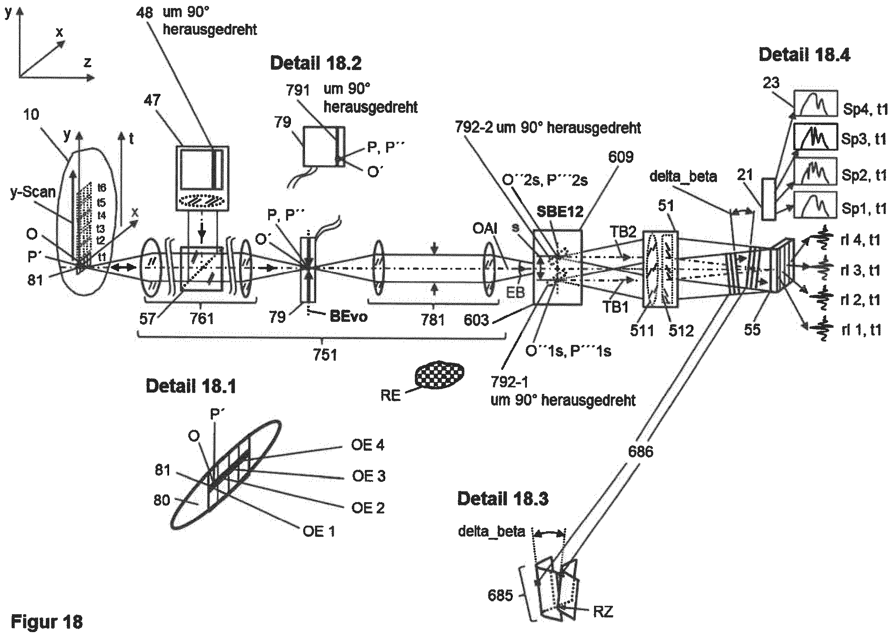

In der **Fig. 18** erfolgt die Bildfeld-Diskriminierung im sichtbaren Spektralbereich bevorzugt mittels eines Flüssigkristall-Displays 79 (LCD) vor dem Michelsontyp-Interferometer 609 in einer konfokalen Anordnung 751 mit der dem Michelsontyp-Interferometer 609 vorgeordneten Bildebene BEvo. Das Michelsontyp-Interferometer 609 entspricht dem bereits in der **Fig. 5** dargestellten Michelsontyp-Interferometer 603, ist hier jedoch ohne Bildfeld-Diskriminatoren im Interferometer ausgebildet. In dem hier beschriebenen, nicht unter den Schutzumfang der Erfindung fallenden Beispiel zu **Fig. 18** gibt es also keine Bildfeld-Diskriminierung im Interferometer.

Hier ist das Prinzip des Ansatzes für ein Single-Shot-Linien-Spektrometer mit starker streifenförmiger Beleuchtung und Ortsauflösung im Streifen, also dem Pushbroom-Ansatz, mit konfokaler Diskriminierung des Objektlichts aufgezeigt. Es gibt einen eindimensionalen y-Scan relativ über das biologische Messobjekt 10 und quer zum Streifen. Der Scan erfolgt mittels der Bewegung des Messobjekts 10 in y-Richtung, welches fest auf einem hier nicht dargestellten y-Schlitten angeordnet ist.

Ein Teil des Messobjekts 10 wird mittels einer steuerbaren Streifenlichtquelle 47 mit einem OLED-Array 48 und einer Strahlformungsoptik in Form eines Streifens 80 beleuchtet, nachdem das Licht - hier im sichtbaren Spektralbereich - einen Einkoppelstrahlteilerwürfel 57 und einen Teil des erstes, zusammengesetzten Objektivs 761 passiert hat. Das zurückgestreute Licht passiert das erste, zusammengesetzte Objektivs 761 mit dem Einkoppelstrahlteilerwürfel 57 und gelangt somit abgebildet - nach Passieren eines hier nicht dargestellten Polarisators, auf ein Flüssigkristall-Display 79 (LCD) als steuerbarer Spaltblenden-Diskriminator in der Bildebene des Objektivs 761, welche in Bezug auf das Michelsontyp-Interferometer 603 eine vorgeordnete Bildebene BEvo ist. Dort gibt es einen in das Flüssigkristall-Display 79 eingeschriebenen - im Sinne von einprogrammierten - schmalen Durchlassbereich 791, siehe Detailabbildung 18.2, der vom Bild des Streifens 80 voll überdeckt wird, so dass dieser Durchlassbereich 791 hier den Bildfeld-Diskriminator darstellt. Die rechnergesteuerte Lichtquelle 47 und das Flüssigkristall-Display 79 sind mittels Rechnersystem 21 zueinander synchronisiert. Die hierzu notwendigen Daten- und Steuerleitungen sind hier nicht dargestellt. Somit können mittels Rechnersteuerung auf dem Messobjekt 10 synchronisiert verschiedene Bereiche beleuchtet werden, zu denen es dann einen angepassten Durchlassbereich auf dem Flüssigkristall-Display 79 gibt. Das Flüssigkristall-Display 79 mit dem jeweils beleuchteten Durchlassbereich 791 wird von einem zweiten, zusammengesetzten Objektiv 781 erfasst und in das Michelsontyp-Interferometer 609 abgebildet, welches vergleichbar dem Michelsontyp-Interferometer 603, dargestellt in **Fig. 5** ist, hier jedoch ohne Bildfeld-Diskriminatoren genutzt wird. Dieses System besitzt als gerasterten Detektor eine CMOS-Kamera 55 für den sichtbaren Spektralbereich. Von jedem erfassten Objektelement OE, dargestellt in der Detailabbildung 18.1, wird hier ein Paar zueinander geneigter Zylinderwellen erzeugt, deren Scheitellinien 686 der Zylinderwellenfronten 685 zueinander geneigt sind, siehe Detailabbildung18.3. Diese Zylinderwellen generieren auf einer CMOS-Kamera 55 räumliche Interferogramme, hier beispielhaft als rI1 bis rI4 anstelle einer Vielzahl von räumlichen Interferogrammen in der Detailabbildung 18.4 dargestellt, aus welchen Spektren Sp errechnet werden, die hier beispielhaft als Sp1 bis Sp4 dargestellt sind.

Ein Flüssigkristall-Display 79 als steuerbarer Spaltblenden-Diskriminator in der vorgeordneten Bildebene BEvo lässt im Wesentlichen nur einen auf dem Messobjekt 10 hell beleuchteten Streifen 80 hindurch, der von der gesteuerten Lichtquelle 47 erzeugt wird. Dies stellt eine konfokale Diskriminierung dar.

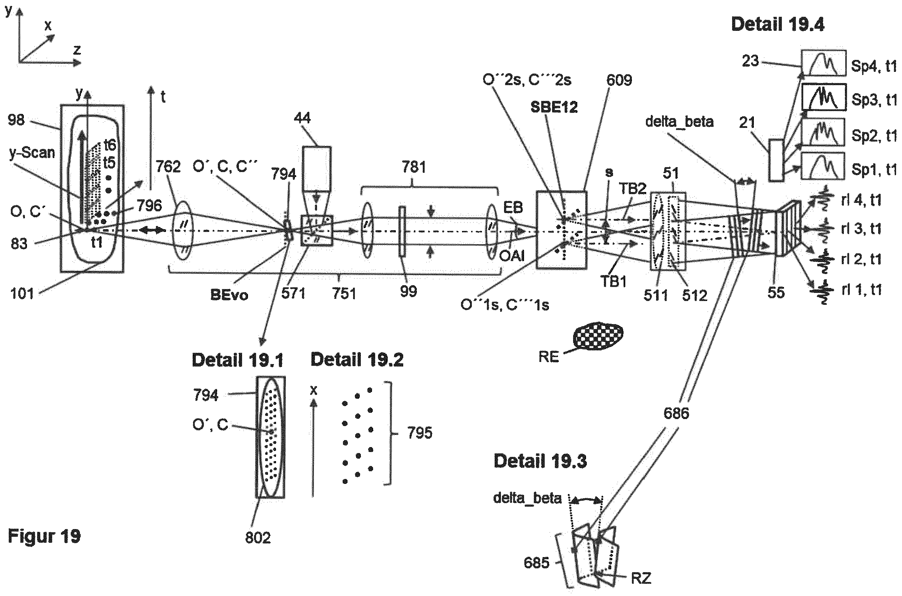

In der **Fig. 19** erfolgt eine konfokale Bildfeld-Diskriminierung mittels einer konfokalen Anordnung 751 vor dem Michelsontyp-Interferometer 609. Von einer gepulsten UV-Lichtquelle 44 mit integrierter Strahlformungsoptik wird ein Streifen 802 ultravioletten Lichts auf starre Blendenscheibe 794 mit Pinholes 802 projiziert. Die starre Blendenscheibe 794 ist in der Detailabbildung 19.1 dargestellt. Diese Blendenscheibe 794 weist ein Pinhole-Muster 795 in einer Zick-Zack-Anordnung, siehe Detailabbildung 19.2, der Pinholes auf, so dass sich in jeder x-Position nur genau ein Pinhole befindet.

Mittels des ersten Objektivs 762 der konfokalen Anordnung 751, welches als Mikroskopobjektiv ausgebildet ist, entsteht auf dem mit Fluoreszenzmarkern markierten biologischen Messobjekt 101 ein Bild 796 des beleuchteten Pinhole-Muster 795. Es kommt an den Lichtpunkten zur Fluoreszenz. Fluoreszenz-Licht, welches von einem scharf abgebildeten Bild eines Pinholes stammt, kann gemäß dem konfokalen Prinzip im Rücklauf das zugehörige Pinhole der Blendenscheibe 794 passieren und es entsteht konfokal selektiertes Licht. Nach Transmission des Lichts am Einkoppelstrahlteilerwürfel 571, gelangt das konfokal selektierte Fluoreszenz-Licht mittels des zweiten, zusammengesetzten Objektivs 781 der konfokalen Anordnung 751 unter Passieren des UV-Sperrfilters 99 in das Michelsontyp-Interferometer 603, welches in **Fig. 5** dargestellt und in der Beschreibung zu **Fig. 5** in seiner Funktionsweise erklärt ist. Hierbei besitzen jedoch die schmalen Spiegel 634 und 638 im Michelsontyp-Interferometer 603, dargestellt in **Fig. 5****,** keine das Bildfeld begrenzende Funktion. Es gibt im nicht unter den Schutzumfang der Erfindung fallenden Beispiel zu **Fig. 19** also keine Bildfeld-Diskriminierung im Michelsontyp-Interferometer 609. Von jedem durch Strahlteilung entstandenen Bildpunkt O" 1 und O"2 eines Objektpunkts O vom Messobjekt 101 entsteht mittels anamorphotischem Objektiv 51 jeweils eine Zylinderwelle auf einer CMOS-Kamera 55 für den sichtbaren Spektralbereich. Dargestellt sind hier die scheinbaren Bildpunkte O"1s und O"2s in der scheinbaren Bildebene SBE12. Je ein Paar von kohärenten Zylinderwellen, deren Scheitellinien 686 der Zylinderwellenfronten 685 zueinander geneigt sind, siehe Detailabbildung 19.3, bildet durch Zweistrahl-Interferenz ein räumliches Interferogramm rI. In der Detailabbildung 19.4 sind hier beispielhaft für eine Vielzahl von Interferogrammen nur vier räumliche Interferogramme rI1 bis rI4 dargestellt, die zu vier verschiedenen Pinholes gehören. Aus diesen Interferogrammen werden mittels schneller Fourier-Transformation (FFT) mittels hier nicht dargestelltem Rechnersystem 21 die zugehörigen Spektren berechnet, hier beispielhaft für eine Vielzahl von Spektren als Spektren Sp1 bis Sp4 dargestellt, siehe Detailabbildung 19.4. Aufgrund des Single-Shot-Ansatzes bei der Detektion räumlicher Interferogramme rI sind im Messaufbau bei angepassten Integrationszeiten der CMOS-Kamera 55 Restschwingungen in der Regel gut zu tolerieren. Durch einen feinen Y-Scan mittels eines Transportschlittens 98 kann aus einem, jeweils "in einem Schuss", also innerhalb der Pulszeit der gepulsten UV-Lichtquelle 44, erstellten Streifen 802 mit den zugeordneten Spektren Sp nach und nach ein Bildausschnitt mit hyperspektralen Informationen vom biologischen Messobjekt 101 erstellt werden. Dabei sind die gepulste UV-Lichtquelle 44 und die CMOS-Kamera 55 synchronisiert.

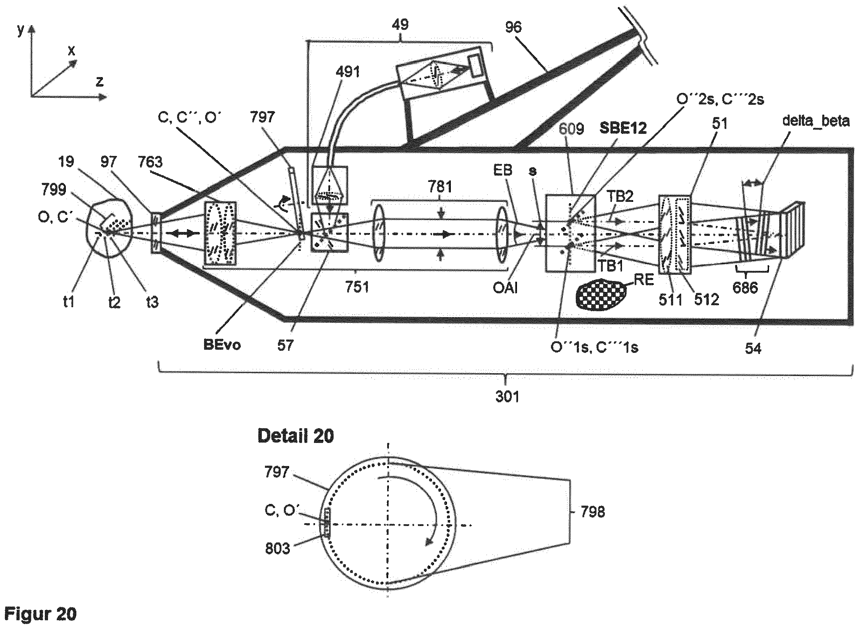

In der **Fig. 20** ist ein miniaturisierter mobiler Messkopf 301 mit einer konfokalen Anordnung 751 an einem Roboterarm 96 als hyperspektrales Diagnose-Instrument dargestellt. Dieses Diagnose-Instrument kommt in einem Operationssaal zum Einsatz. Der Roboterarm 96 befindet sich an einem hier nicht dargestellten 5-Achsen-Roboter, welcher neben der Translation in x- und y-Richtung auch eine vergleichsweise feine rechnergesteuerte Tiefenbewegung in der z-Richtung gestattet. Die Lichtzufuhr in den mobilen Messkopf 301 erfolgt mittels einer externen, fasergekoppelten, nah-infraroten Kaltlichtquelle 49 mit einer Strahlformungsoptik 491. Das Licht gelangt von derselben nach Passieren eines Einkoppelstrahlteilerwürfels 57 auf eine rotierende Blendenscheibe 797 mit Pinholes 798, die in einem schmalen Kreisring angeordnet sind. Die Detailabbildung 20 stellt diese Blendenscheibe 797 dar. Auf einige der Pinholes 798, die sich in einem geschlossenen Kreisring auf der Blendenscheibe 797 befinden, wird mittels der Strahlformungsoptik 491 über den Einkoppelstrahlteilerwürfel 57 ein Lichtstreifen 803 projiziert. Die beleuchteten Pinholes 798 dienen mittels der ersten, zusammengesetzten, mikroskopischen Objektivstufe 763 der konfokalen Anordnung 751 zur Beleuchtung des Gewebes 19. Dort entstehen die Bilder 799 der leuchtenden Pinholes 798. Das Gewebe 19 ist Teil eines chirurgischen Operationsgebietes am offenen Körper eines Menschen unter den Bedingungen einer chirurgischen Operation. Gemäß dem konfokalen Prinzip kann im Wesentlichen nur vom Operationsgebiet 19 gestreutes Licht die Pinholes 798 der Blendenscheibe im Rücklauf wieder passieren, welches dort im oder am Gewebe einen scharfen Fokuspunkt gebildet hat. Die konfokale Bildfeld-Diskriminierung erfolgt hier also mittels der Pinholes 798 der rotierenden Blendenscheibe 797. Das konfokal selektierte Licht gelangt über die zweite Abbildungsstufe 781 der konfokalen Anordnung 751 in das Michelsontyp-Interferometer 609, Hier in **Fig. 20** besitzen die schmalen Spiegel 634 und 638 des Michelsontyp-Interferometers 603, dargestellt in **Fig. 5****,** jedoch keine, das Bildfeld begrenzende Funktion, da diese Spiegel in 609 breiter als das auf diesen sich ergebende Bild ausgebildet sind. Es gibt im nicht unter den Schutzumfang der Erfindung fallenden Beispiel zu **Fig. 20** also keine Bildfeld-Diskriminierung im Michelsontyp-Interferometers 609. Von jedem Bildpunkt eines Messobjekts, wobei hier nur die scheinbaren Bildpunkte O"1s und O"2s symbolisch dargestellt sind, entsteht mittels anamorphotischem Objektiv 51 jeweils eine Zylinderwelle auf einem Detektor, insbesondere auf einer InGaAs-Kamera 54 für den nah-infraroten Spektralbereich. Je ein Paar von kohärenten Zylinderwellen bildet durch Zweistrahl-Interferenz ein räumliches Interferogramm. Aufgrund des Single-Shot-Ansatzes bei der Interferogramm-Detektion sind Restvibrationen des 5-Achsen-Roboters bei angepassten Integrationszeiten der InGaAs-Kamera 54 in der Regel zu tolerieren. Durch feine Lateralbewegungen kann aus einem, jeweils in "in einem Schuss", also innerhalb einer Integrationszeit der InGaAs-Kamera 54, erfassten Bildstreifen 803 nach und nach ein Bildausschnitt mit hyperspektralen Informationen vom Gewebe 19 erstellt und der medizinischen Bewertung unterzogen werden. Zu verschiedenen Zeitpunkten t1, t2, t3, ... können mittels Robotik auch verschiedene Tiefenpositionen angefahren werden, um das Gewebe 19 konfokal diskriminiert auch in der Tiefe zu untersuchen, um Spektren in verschiedenen Tiefen zu gewinnen. In der **Fig. 20** wurde auf die Darstellung der notwendigen Treiber-, Steuer- und Datenleitungen sowie des Rechnersystems mit seinen Komponenten aus Gründen der besseren Darstellbarkeit vollständig verzichtet.

Im Folgenden werden verwendete Begriffe näher erläutert, beschrieben und/oder definiert.

Der Begriff Licht wird in dieser Schrift als Synonym für elektromagnetische Strahlung, und zwar insbesondere vom UV- bis zum Terahertz-Bereich verwendet.

Es geht hierbei um Fourier-Transformations-Spektrometer mit einer eher niedrigen bis zu einer moderaten spektralen Auflösung, bevorzugt im Bereich von delta_sigma gleich 4cm⁻¹ bis zu 1000cm⁻¹.

Der Begriff Lateral-Shear leitet sich aus dem Phänomen der Interferenz zweier reflektierter Lichtstrahlenbündel mit lateralem Versatz, also einem Querversatz ab. Dabei werden in konventionellen Anordnungen typischerweise ein zu testendes Lichtstrahlenbündel an einer äußeren Fläche und ein Lichtstrahlenbündel an einer inneren Fläche einer sogenannten Scherplatte (shear plate) derart reflektiert, dass sie räumlich (und zeitlich) zueinander verschoben reflektiert werden. Insbesondere kann eine Lateral-Shear üblicherweise mit einem Scher-Interferometer erzeugt werden, wobei das Scher-Interferometer ein optisch vergleichsweise einfaches Mittel in Form einer Platte darstellt, um eine Wellenfront-Analyse durchzuführen. Damit kann die Kollimation von Lichtstrahlenbündeln getestet werden, insbesondere von Laserquellen, deren Kohärenzlänge in der Regel deutlich größer ist als die optische Dicke der Platte. Das Scher-Interferometer, ausgebildet als Platte, umfasst üblicherweise ein im Wesentlichen hochwertiges optisches Glas wie beispielsweise N-BK7 oder auch Quarzglas mit besonders ebenen und glatten optischen Oberflächen, die normalerweise in einem sehr kleinen Winkel zueinander stehen, also im Wesentlichen nicht parallel zueinander angeordnet sind und damit einen sehr schwach keilförmigen Charakter aufweisen. Dabei fällt im Test ein gut kollimiertes Strahlenbündel, beispielsweise in einem Winkel von etwa 45° auf das Scher-Interferometer in Form der Platte ein und wird zweimal reflektiert. Die beiden reflektierten Lichtstrahlenbündel sind aufgrund des schwach keilförmigen Charakters nach Passieren der Platte zueinander leicht verkippt und zeigen bei perfekter Kollimation des Eingangsstrahlenbündels (ebene Wellenfront) Interferenzstreifen nach der Scherplatte, welche üblicherweise bei perfekter Kollimation parallel zur Richtung der Lateral-Shear orientiert sind, jedoch bei nichtperfekter Kollimation zu dieser Richtung der Lateral-Shear verdreht sind. Diese durch die Scherplatte erzeugte Trennung bzw. laterale Verschiebung der Strahlenbündel wird als Scherung bzw. Shear, insbesondere als Lateral-Shear bezeichnet. Die Scherung bzw. Lateral-Shear kann auch durch Gitter erzeugt werden oder, wie im vorliegenden Fall, durch eine geeignete Spiegelgruppe, insbesondere durch einen erfindungsgemäßen Dreispiegel-Winkelreflektor. Die Lateral-Shear ist in den jeweiligen Zeichnungen mit dem Bezugszeichen "s" angedeutet.

Der Begriff "Zweistrahl-Interferometer" umfasst insbesondere ein Michelsontyp-Interferometer.

Mit dem Akronym FIR ist insbesondere der ferninfrarote Spektralbereich gemeint, wobei diese insbesondere in etwa zwischen 50 µm und 1000 µm liegt.

Bei einem Michelsontyp-Interferometer ist es insbesondere so, dass das hinlaufende und das rücklaufende Bündel in jedem Interferometerarm zumindest näherungsweise parallel zueinander verläuft und die Strahlteilung und Strahlvereinigung im Wesentlichen an derselben Strahlteilerfläche erfolgen. Es wird vorliegend hauptsächlich der Begriff "Michelsontyp-Interferometer" anstelle von "Michelson-Interferometer" verwendet, da insbesondere hierin Anordnungen beschrieben werden, in denen mehr als ein Planspiegel in mindestens einem der Interferometerarme IA1 und IA2 vorliegen und somit kein reines "Michelson-Interferometer" im engeren Sinne zu verstehen ist.

Das Akronym MIR bezieht sich insbesondere auf den mitteleren Infrarotbereich, der insbesondere zwischen etwa 3 µm und 50 µm liegt.

Ein Bildfeld-Diskriminator ist insbesondere eine Öffnung in einer Blende, wobei die Öffnung bevorzugt eine Spaltblende 645, ein Pinhole, ein Pinhole-Array, welches eine Mehrzahl von Pinholes aufweist, und/oder ein Durchlassbereich 656, 657 eines Flüssigkristall-Displays 655 sein.

Ein nichtdiskriminierender Bereich eines Bildfeld-Diskriminators umfasst beispielsweise den Durchlassbereich bei einem spaltförmigen Bildfeld-Diskriminators, also im einfachsten Fall die Spaltöffnung und/oder den reflektierenden Bereich beispielsweise bei einem sehr schmalen Planspiegel als Bildfeld-Diskriminator. Der nichtdiskriminierende Bereich kann auch durch einen räumlichen Lichtmodulator in Transmission (Flüssigkristall-Display) und/oder in Reflexion (Digitales Mikrospiegel-Array) dargestellt sein. Der nichtdiskriminierende Bereich kann in sich auch noch eine feine Strukturierung aufweisen.

Ein Bildfeld-Diskriminator kann insbesondere auch einen schmalen reflektierenden Bereich auf einem Planspiegel und/oder auf einem Mikrospiegel-Array und/oder auch einen schmalen Planspiegel aufweisen.

Die genannten Formen eines Bildfeld-Diskriminators sind insbesondere dazu ausgelegt, zumindest einen Teil eines Lichtbündels auf einem wohldefinierten Strahlengang in den weiteren Abschnitt des Strahlengangs passieren und zur Detektion kommen zu lassen, ganz besonders bevorzugt in Form eines schmalen Streifens, der bevorzugt ein Zehntel bis zu einem Tausendstel der Ausdehnung des Bildes entsprechen kann, beispielsweise ein Zehntel bis zu einem Tausendstel der Höhe des Bildes vom Messobjekt im Zweistrahl-Interferometer. Die faktische "Eindimensionalität" des schmalen Streifens - mit Bildelementen bevorzugt nur in einer einzigen Reihe - ermöglicht es letztlich, aus jedem nach der Selektion noch verbliebenen Bildelement jeweils genau ein räumliches Interferogramm zu erzeugen. In anderen Worten werden insbesondere solche Anteile eines Lichtbündels mittels des Bildfeld-Diskriminators ausgeblendet bzw. blockiert, die nicht dem vorbestimmten Strahlengang folgen sollen. Beispielsweise kann auch Streulicht mittels des Bildfeld-Diskriminators ausgeblendet bzw. blockiert werden. Das bedeutet, dass auch Streulicht am Passieren des Bildfeld-Diskriminators in den weiteren Abschnitt des Strahlengangs gehindert wird. Mit dem Begriff "räumliches Selektieren" ist die Auswahl bzw. das Passieren des Lichtes gemeint, das durch den Bildfeld-Diskriminator, beispielsweise den Spalt treten bzw. fallen kann und letztlich zur Detektion kommt. Es wird in anderen Worten nicht nur das Bildfeld selektiert, sondern auch das hier unerwünschte Streulicht minimiert.

Ein Prisma, alternativ auch Spiegelprisma genannt, weist insbesondere ein refraktives Material, wie z.B. CaF₂, Si, BK7, Quartz und/oder andere gängige optische Materialien auf. Das Prisma kann zumindest teilweise beschichtet, entspiegelt oder auch vollständig unbeschichtet sein. Insbesondere weist das Prisma zumindest eine für den Ein-und/oder Austritt eines Lichtbündels geeignete Ein- und/oder Austrittsfläche auf. Insbesondere weist das Prisma auch zumindest eine Reflexionsfläche bzw. eine Spiegelfläche auf, die unter geeigneten Bedingungen dazu ausgelegt ist, zumindest einen Teil des in das Prisma eingetretenen Lichtbündels zu reflektieren bzw. zu spiegeln. Eine im Wesentlichen vollständige Reflexion kann insbesondere auch unter Winkeln der Totalreflexion erfolgen. Insbesondere kann das Prisma auch eine zweite Reflexionsfläche bzw. Spiegelfläche, die sich dazu eignet, zumindest einen Teil des in das Prisma eingetretenen Lichtbündels ein zweites Mal zu reflektieren bzw. zu spiegeln, aufweisen.

Das Prisma kann eine Spiegelschicht auf zumindest einem Abschnitt einer Reflexionsfläche aufweisen. Beispielsweise kann die Oberfläche einer Reflexionsfläche zumindest teilweise mit Gold und/oder Silber und/oder Aluminium beschichtet sein.

Im Allgemeinen entsprechen sich die Begriffe Reflexionsfläche und Spiegelfläche. Ein Spiegeln bzw. eine Reflexion kann an einer zumindest teilweisen verspiegelten Fläche erfolgen oder insbesondere auch unter bestimmten Winkeln an einem Übergang zwischen Medien unterschiedlicher Brechungsindizes, beispielsweise beim Auftreffen eines Lichtbündels, das ein optisch dichtes Medium durchläuft auf eine Grenzfläche zu einem optisch dünnen Medium. Unter besonderen Winkeln kann eine Totalreflexion erfolgen, bei der im Wesentlichen der gesamte Anteil des Lichtbündels vollständig reflektiert wird.

Ein Spiegelprisma, bei dem zwei Reflexionsflächen genutzt werden sollen, kann insbesondere ein Prisma sein, bei dem die beiden Reflexionsflächen einen rechten Winkel einschließen und die Ein- und Austrittsfläche des Prismas diesem Winkel gegenüber liegt.

Ein Strahlteiler einer Strahlteilereinheit entspricht insbesondere einem Strahlteilerwürfel oder zwei einzelnen Planparallelplatten, die bevorzugt für den infraroten Spektralbereich jeweils aus Quarzglas, kristallinem Quarz, Kalziumflorid (CaF₂), Zinkselenid (ZnS) oder Kaliumbromid (KBr) ausgebildet sind, und eine Strahlteilerschicht aufweisen oder einen Polarisationsstrahlteilerwürfel oder einen Plattenstrahlteiler mit zwei Platten und einer Strahlteilerschicht. Ein Strahlteiler ist insbesondere dazu ausgelegt, zumindest einen Anteil des einfallenden Lichtbündels an einer Auftrittsoberfläche zu transmittieren, um das erste Teilstrahlenbündel zu erzeugen und zumindest einen weiteren Anteil des einfallenden Lichtbündels zu reflektieren, um das zweite Teilstrahlenbündel zu erzeugen. Ein Strahlteiler ist ferner insbesondere dazu ausgelegt, zumindest einen Anteil des ersten Teilstrahlenbündel an einer Strahlteilerfläche zu reflektieren und zumindest einen Anteil des zweiten Teilstrahlenbündels zu transmittieren.

Eine Strahlteilereinheit weist insbesondere in einem Strahlteilerwürfel oder in einem System aus Planparallelplatten zumindest eine plane Strahlteilerschicht auf. Die Strahlteilerschicht hat eine erste dem einfallenden Lichtbündel zugewandten Seite und eine dem einfallenden Lichtbündel abgewandte Seite. Die Strahlteilerfläche ist dazu ausgelegt, einfallendes Licht in Teilen zu transmittieren, um einen ersten Teilstrahlbündel zu erzeugen und in Teilen zu reflektieren, um ein zweites Teilstrahlbündel zu erzeugen. Insbesondere ergeben sich somit in einem üblichen Michelsontyp-Interferometer zwei im Wesentlichen zueinander senkrecht verlaufende Teilstrahlbündel.

Eine Referenzebene, die z.B. zur Beschreibung der Anordnung einer (2n+1)-Spiegelgruppe dienen kann, wird insbesondere durch die Normale der ebenen Strahlteilerfläche und durch die optische Achse des vorgeordneten Objektivs am Eingang des Michelsontyp-Interferometers aufgespannt.

Eine (2n+1)-Spiegelgruppe kann voneinander isolierte Spiegelflächen aufweisen, die ohne direkte Kontaktierung zueinander senkrecht zu einer gemeinsamen Referenzebene angeordnet sein können. Alternativ oder zusätzlich kann eine (2n+1)-Spiegelgruppe auch mindestens zwei Spiegelflächen aufweisen, die einander kontaktieren oder gar in einem Stück miteinander ausgebildet sind. Dabei entsprechen die Spiegelflächen dann Spiegelflächenabschnitten, die in unterschiedlichen Ebenen senkrecht zu einer gemeinsamen Referenzebene angeordnet sind. In anderen Worten bilden in diesem Fall zwei Spiegelflächenabschnitte einen Spiegel mit einem Knick.

Der Begriff "(2n+1)-Winkelspiegelgruppe" entspricht dem Begriff "(2n+1)-fach-Winkelspiegelgruppe". Bei einer Dreifach-Winkelspiegelgruppe stehen, anders als bei einer Tripelspiegel-Anordnung, die planen Spiegelflächen alle stets senkrecht zu einer gemeinsamen Bezugsebene.

Zylinderwellen sind insbesondere Lichtwellen, die einen Ausschnit aus einer Zylinderfläche darstellen.

Die erste Bildfeld-Diskriminatoreinheit und die zweite Bildfeld-Diskriminatoreinheit liegen im Michelsontyp-Interferometer in optisch konjugierten Ebenen, wenn das Michelsontyp-Interferometer hinreichend präzise winkelmäßig justiert ist und die optischen Weglängen in den beiden Interferometerarmen gleich sind. Dabei müssen auch die gleichen optischen Materialien in den beiden Interferometerarmen eingesetzt sein.

Ein scharfes Bild ist insbesondere dadurch ausgezeichnet, dass es nahezu beugungsbegrenzt ist.

Ein unscharfes Bild ist insbesondere dadurch ausgezeichnet, dass dessen Bildpunkte die Größe eines Airy-Scheibchens um ein Mehrfaches übertreffen.

Astigmatismus umfasst insbesondere das Phänomen, dass in zwei senkrecht zueinander angeordneten Schnittebenen zwei Bildpunkte, die jeweils von Strahlenbüscheln der jeweiligen Schnittebene gebildet sind, erheblich in der Tiefenlage, also in Ausbreitungsrichtung des Lichts, signifikant separiert sind. In der Erfindung liegt so bei der Detektion einer der Bildpunkte im Wesentlichen bevorzugt im Unendlichen und der andere im Nahbereich, bevorzugt auf dem gerasterten Detektor.

Der Begriff "Tiefe" ist die Dimension in Ausbreitungsrichtung des Lichts.

Der Begriff "wellenoptische Schärfentiefe" ist durch die Relation Lichtwellenlänge dividiert durch das Quadrat des Sinus des Aperturwinkels des zugehörigen Lichtbündels gegeben.

Eine verspiegelte Fläche ist insbesondere dazu ausgelegt etwa 80 % bis etwa 100 %, insbesondere etwa 93 % bis etwa 100 % und bevorzugt etwa 97 % bis etwa 100 % des einfallenden Lichtes in zumindest einem Teil des Wellenlängenspektrums elektromagnetischer Strahlung zu reflektieren.

Eine unverspiegelte bzw. nicht verspiegelte Fläche ist insbesondere dazu ausgelegt weniger als etwa 60 %, insbesondere weniger als etwa 30 % und bevorzugt weniger als etwa 5 % des einfallenden Lichtes in zumindest einem Teil des Wellenlängenspektrums elektromagnetischer Strahlung zu reflektieren.

Ein optisches Übersprechen kann bei der Messung von biologischem Gewebe durch Streulicht auftreten, welches sich auf mehrere Detektorelemente eines gerasterten Detektors verteilt. Dieses Streulicht wird durch eine konfokale Diskriminierung weitgehend blockiert, wodurch sich das optische Übersprechen reduziert. Weiterhin kann ein optisches Übersprechen auch so verstanden werden, dass Strahlen eines nichtperfekten und/oder auch etwas defokussierten Lichtstrahlenbündels, welche einen Bildpunkt, beispielsweise auf einem Detektorelement eines gerasterten Detektor erzeugen, auch ein oder mehrere benachbarte Detektorelemente des gerasterten Detektors treffen.

### Bezugszeichenliste mit Erläuterungen

| **Bezugszeichen** | **Bezeichnung** |
|---|---|
| 1 | Rücken eines älteren Patienten unter Diagnose |
| 10 | biologisches Messobjekt |
| 101 | biologisches Messobjekt mit Fluoreszenzmarkern |
| 11 | Hautmerkmal, welches aus medizinischer Sicht sorgfältig untersucht werden muss, da es sich um ein Muttermal oder ein Melanom handeln kann. |
| 12 | Hochrisikobereich, farblich auf dem Monitor hervorgehoben Die Risikohöhe kann nach Auswertung der Spektraldaten mittels künstlicher Intelligenz ermittelt werden. |
| 13 | Bereich auf dem Rücken 1, der mittels Spektrometer-Scan erfasst wird |
| 14 | Messobjekt zur Auflichtmessung |
| 15 | teiltransparentes Messobjekt für eine Durchlichtmessung |
| 16 | heiße, strömende Abgaswolke in der Ferne als Messobjekt |
| 17 | unzugängliches organisches Messobjekt in mittlerer Entfernung, welches Wärmestrahlung abgibt |
| 18 | Triebwerksflamme als Messobjekt |
| 19 | Gewebe am offenen Körper eines Menschen unter den Bedingungen einer chirurgischen Operation |
| 20 | Fourier-Transformations-Spektrometer-System |
| 21 | Rechnersystem für die Steuerung der Komponenten wie Lichtquelle und gerasterter Detektor und mit Rechenprogramm zur Auswertung der räumlichen Interferogramme rI mit Berechnung von Spektren |
| 22 | Auswerteprogramm für errechnete Spektren, auch um Hochrisikobereiche für ein Melanom bei der medizinischen Untersuchung zu lokalisieren |
| 23 | Monitor zur Darstellung der ausgewerteten Daten |
| 25 | Steuer- und Synchronisierungsmittel für die rechnergesteuerten Treiber 26 und 27 |
| 26 | rechnergesteuerter Treiber mit Synchronisierungsmitteln für ein digitales Mikrospiegel-Array 654, das synchron zum digitalen Mikrospiegel-Array 655 arbeitet |
| 27 | rechnergesteuerter Treiber mit Synchronisierungsmitteln für das digitale Mikrospiegel-Array 655, das synchron zum digitalen Mikrospiegel-Array 654 arbeitet |
| 28 | Datenverbindung zu Datenbanken |
| 30 | mobiler Messkopf |
| 301 | miniaturisierter mobiler Messkopf am Roboterarm als hyperspektrales Diagnose-Instrument |
| 31 | Handgriff des mobilen Messkopfes 30 |
| 32 | Startknopf für die Datenaufnahme |
| 40 | gepulste Streifenlichtquelle im NIR-Bereich |
| 41 | Lichtquellentreiber |
| 42 | Datenverbindung |
| 43 | gepulste Streifenlichtquelle, durch den Rechner 21 steuerbar, deren Licht mittels Einkoppelstrahlteilerwürfel 57 koaxial in den Beleuchtungsstrahlengang eingekoppelt wird |
| 44 | gepulste UV-Lichtquelle mit integrierter Strahlformungsoptik zur Projektion eines Lichtstreifens 802 auf eine starre Blendenscheibe 794 |
| 45 | Abbildungsoptik für eine Lichtquelle zur Erzeugung strukturierten Lichts |
| 46 | Lichtquelle zur Erzeugung strukturierten Lichts mit integrierter Abbildungsoptik für eine streifenförmige Objektbeleuchtung für eine Transmissionsmessung |
| 47 | rechnergesteuerte Streifenlichtquelle mit einem OLED-Array 48, synchronisiert mit einem Flüssigkristall-Display 79 |
| 48 | OLED-Array als Lichtquelle |
| 49 | externe fasergekoppelte Kaltlichtquelle im nahinfraroten Spektralbereich mit Strahlformungsoptik für die Beleuchtung einer rotierenden Blendenscheibe 797 mit Pinholes 798 mit einem Lichtstreifen 803 |
| 491 | Strahlformungsoptik für die Beleuchtung einer rotierenden Blendenscheibe 797 mit einem Lichtstreifen 803 |
| 50 | Optik-Einheit des mobilen Messkopfes 30 mit einem Michelsontyp-Interferometer mit einem diesem nachgeordneten Objektiv am Ausgang desselben und einem gerasterten Matrix-Detektor zur Detektion räumlicher Interferogramme rI |
| 51 | dem Michelsontyp-Interferometer nachgeordnetes, anamorphotisches und weitgehend achromatisches Objektiv, um von kohärenten Bildpunkten von einem Messobjekt zueinander geneigte und interferierende Zylinderwellen auf einem gerasterten Detektor zu erzeugen. |
| 511 | rotatorische Komponente des anamorphotischen Objektivs 51 |
| 512 | Zylinder-Komponente des anamorphotischen Objektivs 51 |
| 52 | anamorphotisches Objektiv auch mit Zylinderkomponente für den MIR-CaF2-Spektralbereich, welches in der optischen Gesamtfunktion prinzipiell dem Objektiv 51 entspricht |
| 53 | anamorphotisches Spiegelobjektiv mit Zylinderspiegel für den MIR-KBr-Spektralbereich, welches in der optischen Gesamtfunktion prinzipiell dem Objektiv 51 entspricht |
| 537 | anamorphotisches Spiegelobjektiv, bestehend aus den drei spiegelnden Freiformflächen 675, 676 und 677, welches in der optischen Gesamtfunktion prinzipiell dem Objektiv 51 entspricht |
| 54 | InGaAs-Kamera für den nah-infraroten Spektralbereich (NIR) |
| 55 | CMOS-Kamera für den sichtbaren Spektralbereich (VIS) |
| 56 | Steuer- und Datenverbindung |
| 57 | Einkoppelstrahlteilerwürfel für Licht zur Beleuchtung eines Messobjekts |
| 571 | Einkoppelstrahlteilerwürfel mit Reflexionsschicht für UV-Licht und mit Transmissionseigenschaften der Teilerschicht für Fluoreszenzlicht im VIS |
| 58 | Bolometer-Matrix-Detektor |
| 59 | CMOS-Kamera zum Monitoring für den sichtbaren Spektralbereich (VIS) |
| 601 | Michelsontyp-Interferometer mit Lateral-Shear s |
| 602 | Michelsontyp-Interferometer mit Lateral-Shear s, welches hier als Glasblock mit einer Dreifach-Winkelspiegelgruppe 642 ausgebildet ist, welche als Prismenanordnung in W Form ausgebildet ist. |
| 603 | Michelsontyp-Interferometer mit Lateral-Shear s, welches hier als Glasblock mit Ausgleichsplatte 637, einer schmalen, planen Spiegelfläche 638 und mit einer Dreifach-Winkelspiegelgruppe 642 ausgebildet ist, welche als Prismenanordnung in W Form ausgebildet ist. Das Michelsontyp-Interferometer 603 weist zwei Bildfeld-Diskriminatoren in Form eines schmalen Planpiegels 634 und einer planen Spiegelfläche 638 auf. |
| 604 | Michelsontyp-Interferometer mit Lateral-Shear s, welches hier als Glasblock mit Ausgleichsplatte 632 und mit einem linienhaften planen Endspiegel 633 sowie mit einer Dreifach-Winkelspiegelgruppe 643 in Rachenform ausgebildet ist. |
| 605 | Michelsontyp-Interferometer mit Lateral-Shear s mit zwei Dreifach-Winkelspiegelgruppen 652 und 653 in Luft und mit je einem digitalen Mikrospiegel-Array 654 und 655 |
| 606 | Michelsontyp-Interferometer mit Lateral-Shear s für das FIR mit Folienstrahlteiler |
| 607 | Michelsontyp-Interferometer mit Lateral-Shear s für den MIR-Spektralbereich mit CaF2-Substraten für den Strahlteiler |
| 608 | Michelsontyp-Interferometer mit Lateral-Shear s für das MIR mit Plattenstrahlteiler aus KBr und passiven Endreflektoren 678 und 679, jedoch ohne Bildfeld-Diskriminatoren im Interferometer |
| 609 | Michelsontyp-Interferometer mit Lateral-Shear s, welches hier als Glasblock mit Ausgleichsplatte 637 - wie das Michelsontyp-Interferometer 603, jedoch ohne Bildfeld-Diskriminatoren - und mit einer Dreifach-Winkelspiegelgruppe 642 in W-Form ausgebildet ist. Anstelle des schmalen Planpiegels 634 und der schmalen, planen Spiegelfläche 638 im Michelsontyp-Interferometer 603 sind hier im Michelsontyp-Interferometer 609 anstelle dieser etwas breitere Planspiegel eingesetzt, was hier nicht extra dargestellt ist. So besteht im Michelsontyp-Interferometer 609 selbst im Wesentlichen keine Bildfeld-Diskriminierung. |
| 62 | ebene Strahlteilerschicht |
| 621 | Strahlteilerblock |
| 622 | Strahlteilerwürfel |
| 623 | Mylarfolie als Strahlteiler, welche die Strahlteilerfläche darstellt |
| 624 | Strahlteilerplatte aus CaF2 |
| 625 | ebene Strahlteilerschicht für den MIR-Bereich, speziell hier für den Wellenzahlbereich 4000cm⁻¹ bis 1200cm⁻¹ ausgebildet |
| 626 | Kompensationsplatte aus CaF2 |
| 627 | Strahlteilerplatte aus KBr |
| 628 | ebene Strahlteilerschicht für den längerwelligen MIR-Bereich, speziell hier für den Wellenzahlbereich 4000cm⁻¹ bis 400cm⁻¹ ausgebildet |
| 629 | Kompensationsplatte aus KBr |
| 632 | Spiegelplatte angepasster Dicke mit Rückseitenverspiegelung im Arm des Michelsontyp-Interferometers. Die Dicke der Spiegelplatte wird für den Abgleich der optischen Weglänge angepasst. |
| 633 | linienhafter planer Endspiegel, stellt einen ersten Bildfeld-Diskriminator BFD1 dar |
| 634 | schmaler Planspiegel in einer Dreifach-Winkelspiegelgruppe Dieser stellt einen zweiten Bildfeld-Diskriminator BFD2 dar. |
| 635 | linienhafter Spiegel in einer Dreifach-Winkelspiegelgruppe in Rachenform 643 |
| | Dieser linienhafte Spiegel stellt einen zweiten Bildfeld-Diskriminator BFD2 dar. |
| 637 | Ausgleichsplatte mit schmaler, planer Spiegelfläche 638 |
| 638 | schmale, plane Spiegelfläche Diese schmale Spiegelfläche stellt eine Endspiegelfläche sowie einen ersten Bildfeld-Diskriminator BFD1 dar. |
| 639 | Deckplatte aus Glas |
| 640 | (2n+1)-fach-Winkelspiegelgruppe, mit n=1, 2, 3, ... |
| | Eine (2n+1)-fach-Winkelspiegelgruppe besteht aus einer Anordnung von in der Summe (2n+1) planen Spiegeln oder planen Spiegelflächen in Rachen- oder W-Form oder in einer Mischform und jeweils mit Winkel zueinander, welche in der Regel senkrecht zu einer gemeinsamen Referenzebene RE ausgerichtet sind. Die Winkelspiegelgruppe stellt im Michelsontyp-Interferometer jeweils eine Endspiegel-Anordnung dar. |
| | Dabei ist die (2n+1)-fach-Winkelspiegelgruppe so ausgebildet und angeordnet, dass der Einfallswinkel des Hauptstrahls eines Teilstrahlenbündels im Michelsontyp-Interferometer auf einen der planen Spiegel oder eine der planen Spiegelflächen dabei in der Regel größer als zwei Altgrad ist. |
| 641 | Dreifach-Winkelspiegelgruppe |
| | Eine Dreifach-Winkelspiegelgruppe besteht aus einer Anordnung von in der Summe drei planen Spiegeln oder planen Spiegelflächen in Rachen- oder W-Form und jeweils mit Winkel zueinander, wobei die planen Spiegel oder planen Spiegelfläche in der Regel senkrecht zu einer gemeinsamen Referenzebene RE ausgerichtet sind. Die Dreifach-Winkelspiegelgruppe stellt im Michelsontyp-Interferometer jeweils eine Endspiegel-Anordnung dar. |
| | Dabei ist die Dreifach-Winkelspiegelgruppe so ausgebildet und angeordnet, dass der Einfallswinkel des Hauptstrahls eines Teilstrahlenbündels im Michelsontyp-Interferometer auf einen der planen Spiegel oder eine der planen Spiegelflächen dabei in der Regel größer als zwei Altgrad ist, wobei der Aperturwinkel des Teilstrahlenbündels in der Regel kleiner als der Einfallswinkel des Hauptstrahls ist und somit in keinem Fall ein senkrechter Einfall von Strahlen auf einen der planen Spiegel oder eine der planen Spiegelflächen besteht. |
| 642 | Dreifach-Winkelspiegelgruppe, ausgebildet als Prismenanordnung in W-Form gemäß der Definition für eine Dreifach-Winkelspiegelgruppe 641 |
| 643 | Dreifach-Winkelspiegelgruppe, ausgebildet als Prismenanordnung in Rachen-Form gemäß der Definition für eine Dreifach-Winkelspiegelgruppe 641 |
| 644 | Dreifach-Winkelspiegelgruppe in Metall, ausgebildet als Anordnung in Rachen-Form und in Luft gemäß der Definition für 641 |
| 645 | Metallgrundkörper mit Winkelspiegelgruppe |
| 645-1 | Metallgrundkörper im ersten Arm IA1 eines Michelsontyp-Interferometers |
| 645-2 | Metallgrundkörper im zweiten Arm IA2 eines Michelsontyp-Interferometers |
| 646 | Freimachung, welche aus fertigungstechnischen Gründen von Vorteil ist |
| 647 | erster Planspiegel |
| 648 | zweiter Planspiegel, schmal ausgebildet |
| 649 | dritter Planspiegel |
| 650 | blanke Fläche |
| 651 | blanke Fläche |
| 652 | Dreifach-Winkelspiegelgruppe in einem Metallblock 645-1, ausgebildet als Anordnung in Rachen-Form und in Luft, gemäß der Definition für 641, |
| | Eine Spiegelfläche ist mittels digitalem Mikrospiegel-Array 654 gebildet. |
| 653 | zweite Winkelspiegelgruppe in einem Metallblock 645-2, ausgebildet als Anordnung in Rachen-Form und in Luft, gemäß der Definition für 641, |
| | Eine Spiegelfläche ist mittels digitalem Mikrospiegel-Array 655 gebildet. |
| 654 | digitales Mikrospiegel-Array (engl. Digital Micro Mirror Device, DMD) |
| | Dieses digitale Mikrospiegel-Array mit den - durch Programmierung das Licht - selektierenden Mikrospiegeln stellt den ersten Bildfelddiskriminator BFD1 im Michelsontyp-Interferometer 605 dar. |
| 655 | zweites digitales Mikrospiegel-Array (DMD) |
| | Dieses digitale Mikrospiegel-Array mit den - durch Programmierung das Licht - selektierenden Mikrospiegeln stellt den zweiten Bildfelddiskriminator dar BFD2 im Michelsontyp-Interferometer 605 dar. |
| 656 | genutzter Gesamtbereich für die Bildfeld-Diskriminierung auf einem digitalen Mikrospiegel-Array 654 |
| 657 | genutzter Gesamtbereich für die Bildfeld-Diskriminierung auf dem zweiten digitalen Mikrospiegel-Array 655 |
| 658 | spiegelnder, einprogrammierter Bereich für die Bildfeld-Diskriminierung auf einem digitalen Mikrospiegel-Array 654 Dieser spiegelnde Bereich selektiert das letztlich zur Detektion kommende Licht und kann nach und nach durch Rechnersteuerung lateral verschoben werden. |
| 659 | spiegelnder, einprogrammierter Bereich für die Bildfeld-Diskriminierung auf dem zweiten digitalen Mikrospiegel-Array 655 Dieser spiegelnde Bereich selektiert das letztlich zur Detektion kommende Licht und kann nach und nach - und durch Rechnersteuerung synchronisiert zum spiegelnden Bereich 658 des digitalen Mikrospiegel-Arrays 654 - lateral verschoben werden. |
| 660 | Fixierung für das digitale Mikrospiegel-Array 655 |
| 661a, 661b | einzelne Mikrospiegel des digitalen Mikrospiegel-Arrays 655 |
| 662 | zusammengesetzte asymmetrische Dreifach-Winkelspiegelgruppe gemäß der Definition für 641, ausgebildet als Anordnung in W-Form, in Luft und mit spaltförmiger Abschattblende 666 und Spalt 667 derselben |
| 663 | erster vergoldeter Planspiegel der Dreifach-Winkelspiegelgruppe 662 |
| 664 | zweiter vergoldeter Planspiegel der Dreifach-Winkelspiegelgruppe 662 |
| 665 | dritter vergoldeter Planspiegel der Dreifach-Winkelspiegelgruppe 662 |
| 666 | spaltförmige Abschattblende |
| 667 | Spalt der Abschattblende 666 |
| 670 | Anordnung mit einer Spiegeloptik und mit einem Michelsontyp-Interferometer 606 für die Messung im Fernbereich im fern-infraroten Spektralbereich (FIR) |
| 671 | Spiegelblock mit zwei spiegelnden Freiformflächen (672 und 676) für den fern-infraroten Spektralbereich (FIR) |
| 672 | spiegelnde Freiformfläche am Spiegelblock 671 |
| 673 | Spiegelblock mit drei spiegelnden Freiformflächen (674, 675 und 677) für den fern-infraroten Spektralbereich (FIR) |
| 674 | zweite spiegelnde Freiformfläche, erste spiegelnde Fläche am Spiegelblock 673 |
| 675 | zweite spiegelnde Freiformfläche am Spiegelblock 673 |
| 676 | spiegelnde Freiformfläche in Sattelform am Spiegelblock 671 |
| 677 | dritte spiegelnde Freiformfläche am Spiegelblock 673 zur Auskopplung zwecks Detektion der räumlichen Interferogramme rI |
| 678 | zusammengesetzte symmetrische Dreifach-Winkelspiegelgruppe gemäß der Definition für 641, ausgebildet als Anordnung in W-Form und in Luft für den MIR- oder FIR-Spektralbereich mit drei vergoldeten Planspiegeln 678-1, 678-2 und 678-3 |
| 678-1 | erster vergoldeter Planspiegel für den MIR- oder FIR-Spektralbereich |
| 678-2 | schmaler zweiter vergoldeter Planspiegel für den MIR- oder FIR-Spektralbereich |
| | Dieser wirkt als Bildfeld-Diskriminator. |
| 678-3 | dritter vergoldeter Planspiegel für den MIR- oder FIR-Spektralbereich |
| 679 | vergoldeter Planspiegel für den MIR- oder FIR-Spektralbereich Dieser vergoldete Planspiegel ist der einzige Endspiegel im Interferometerarm IA1 und wirkt als Bildfeld-Diskriminator. |
| 680 | Reflektorblock für den MIR-Spektralbereich, ausgebildet mit einer Dreifach-Winkelspiegelgruppe mit den Planspiegeln 683 und 684 und einem digitalen Mikrospiegel-Array 682 |
| 681 | erstes digitales Mikrospiegel-Array mit Goldbeschichtung der Mikrospiegel für den MIR-Spektralbereich im Michelsontyp-Interferometer 607 |
| | Dieses Mikrospiegel-Array 681 stellt einen Endspiegel im Michelsontyp-Interferometer 607 und stellt mit den - durch Programmierung - das Licht selektierenden Mikrospiegeln gleichzeitig auch den ersten Bildfeld-Diskriminator BFD1 dar. |
| 682 | zweites digitales Mikrospiegel-Array mit Goldbeschichtung der Mikrospiegel für den MIR-Spektralbereich im Reflektorblock 680 im Michelsontyp-Interferometer 607 |
| | Dieses Mikrospiegel-Array 682 im Michelsontyp-Interferometer 607 stellt mit den - durch Programmierung - das Licht selektierenden Mikrospiegeln gleichzeitig auch den zweiten Bildfeld-Diskriminator BFD2 dar. |
| 683 | erster Spiegel im Reflektorblock 680 |
| 684 | dritter Spiegel im Reflektorblock 680 |
| 685 | zylindrische Wellenfronten |
| 686 | Scheitellinien zueinander geneigter zylindrischer Wellenfronten |
| 70 | vorgeordnetes Objektiv als fokussierendes Abbildungssystem mit der optischen Achse OAI am Eingang des Michelsontyp-Interferometers 601, welches fokussierte Eingangsstrahlenbündel für das Michelsontyp-Interferometer 601 erzeugt |
| | Das vorgeordnete Objektiv 70 weist eine Autofokus-Funktion auf. |
| 71 | vorgeordnetes Objektiv als fokussierendes Abbildungssystem mit der optischen Achse OAI am Eingang des Michelsontyp-Interferometers 602 und mit einer Telezentrieblende 72 in der gemeinsamen Brennebene der Einzelobjektive |
| | Das vorgeordnete Objektiv ist somit beidseitig telezentrisch und erzeugt fokussierte Eingangsstrahlenbündel für das Michelsontyp-Interferometer 602. |
| 72 | telezentrische Blende |
| 73 | vorgeordnetes Objektiv als fokussierendes Abbildungssystem mit der optischen Achse OAI für den MIR-Spektralbereich mit CaF2-Substraten, welches fokussierte Eingangsstrahlenbündel für das Michelsontyp-Interferometer erzeugt |
| 75 | zweistufiges, vorgeordnetes Objektiv als fokussierendes Abbildungssystem mit der optischen Achse OAI für den mittel-infraroten Spektralbereich (MIR), welches mit einem integrierten Bildfeld-Diskriminator ausgebildet ist Es erzeugt fokussierte Eingangsstrahlenbündel für das Michelsontyp-Interferometer. |
| 751 | konfokale Anordnung mit einem dem Michelsontyp-Interferometer 603 vorgeordneten fokussierenden Objektiv 781 mit der optischen Achse OAI, welches fokussierte Eingangsstrahlenbündel für das Michelsontyp-Interferometer erzeugt |
| 752 | vorgeordnetes Objektiv als fokussierendes Abbildungssystem, gebildet aus den Freiformflächen 672 und 674 und mit der optischen Achse OAI Diese Freiformflächen 672 und 674 erzeugen fokussierte Eingangsstrahlenbündel für das Michelsontyp-Interferometer 606 und sind diesem vorgeordnet. |
| 76 | erstes Objektiv eines vorgeordneten zweistufigen Objektivs 75 |
| 761 | erstes, zusammengesetztes Objektiv in einer konfokalen Anordnung 751 mit zugeordnetem Einkoppelstrahlteilerwürfel 57 |
| 762 | erstes Objektiv in einer konfokalen Anordnung 751, welches als Mikroskopobjektiv ausgebildet ist |
| 763 | erste, zusammengesetzte mikroskopische Objektivstufe in einer konfokalen Anordnung 751 |
| 77 | länglicher Spaltblenden-Diskriminator |
| 78 | zweites Objektiv eines vorgeordneten zweistufigen Objektivs 75, welches selbst das vorgeordnete Objektiv mit der optischen Achse OAI darstellt und fokussierte Eingangsstrahlenbündel für das Michelsontyp-Interferometer erzeugt |
| 781 | zweites, zusammengesetztes Objektiv mit der optischen Achse OAI in einer konfokalen Anordnung 751, welches fokussierte Eingangsstrahlenbündel für das Michelsontyp-Interferometer 603 erzeugt und somit ein vorgeordnetes Objektiv darstellt. |
| 79 | Flüssigkristall-Display (LCD) als steuerbarer Bildfeld-Diskriminator in der Bildebene BEI |
| 791 | in das Flüssigkristall-Display (LCD) 79 eingeschriebener/einprogrammierter schmaler Durchlassbereich |
| 792-1 | Bild des eingeschriebenen/einprogrammierten schmalen Durchlassbereiches 791 im ersten Interferometerarm IA1 |
| 792-2 | Bild des eingeschriebenen/einprogrammierten schmalen Durchlassbereiches 791 im zweiten Interferometerarm IA2 |
| 794 | starre Blendenscheibe mit einem Pinhole-Muster |
| 795 | beleuchtetes Pinhole-Muster in einer Zick-Zack-Anordnung von Pinholes |
| 796 | Bild des beleuchteten Pinhole-Musters |
| 797 | rotierende Blendenscheibe mit Pinholes |
| 798 | Pinholes auf einem Kreisring angeordnet |
| 799 | Bilder beleuchteter Pinholes auf einem Kreisring angeordnet |
| 80 | Lichtstreifen, welcher projiziert wird, und in der Höhe und Länge etwas überdimensioniert ist, damit das Messfeld in der Regel voll ausgeleuchtet ist. |
| 801 | Lichtstreifen, welcher mittels NIR-Streifenlichtquelle auf ein teiltransparentes Messobjekt 15 projiziert wird. |
| 802 | Streifen ultravioletten Lichts, projiziert auf eine starre Blendenscheibe 794 mit einem Pinhole-Muster 795 |
| 803 | Lichtstreifen, projiziert auf eine rotierende Blendenscheibe 797 mit Pinholes 798, von denen einige beleuchtet werden |
| 81 | Messfeld, welches zu einem Zeitpunkt t1 erfasst wird |
| 82 | Messfeld auf einem bewegten Messobjekt, welches von einem stationären Messkopf zu einem Zeitpunkt t1 erfasst wird, beispielsweise eine driftende Abgaswolke |
| 83 | Messfeld mit Beleuchtung durch Pinhole-Bilder, welches zu einem Zeitpunkt t1 erfasst wird |
| 90 | Transportschlitten für y-Scan der Optik-Einheit 50 des mobilen Messkopfes 30 ohne Darstellung des rechnersteuerbaren Antriebs |
| 91 | 2D-Spiegel-Scanner |
| 92 | geneigte CaF2-Platte zur Kompensation von Astigmatismus |
| 93 | Auskoppelstrahlteilerschicht für Monitoring |
| 94 | achssenkrechte CaF2-Platte zur Kompensation des Öffnungsfehlers, welcher im Vorobjektiv 92 einkorrigiert ist |
| 95 | geneigte CaF2-Platte zur Kompensation von Astigmatismus |
| 96 | 5-Achsen-Roboterarm zur präzisen Führung des Messkopfes |
| 97 | Schutzfenster, transparent für den nahinfraroten Spektralbereich (NIR) |
| 98 | Transportschlitten für y-Scan eines Messobjekts |
| 99 | Sperrfilter für ultraviolettes Licht |
| AHS | Ausgangshauptstrahl |
| alpha | Aperturwinkel, gleich dem halben Öffnungswinkel |
| b | Breite der beiden Bildfeld-Diskriminatoren BFD1 und BFD2, die zumindest näherungsweise gleich ist. |
| b' | Breite des lateralen Messobjekt-Inkrements als Rückabbildung der beiden Bildfeld-Diskriminatoren BFD1 und BFD2 |
| b" | näherungsweise Breite des lateralen Messobjekt-Inkrements als unscharfe Rückabbildung der Breite b der beiden Bildfeld-Diskriminatoren BFD1 und BFD2 |
| BEI1 | Bildebene im Interferometerarm IA1, in welcher sich der Bildfeld-Diskriminator BFD1 befindet |
| | Die Bildebene BEI1 und die Bildebene BEI2 sind zumindest näherungsweise optisch konjugiert. |
| BEI2 | Bildebene im Interferometerarm IA2, in welcher sich der Bildfeld-Diskriminator BFD2 befindet |
| BEvo | Bildebene vor dem Michelsontyp-Interferometer, in welcher eine Bildfeld-Diskriminierung durchgeführt wird |
| BFD1's | scheinbares Bild des ersten Bildfeld-Diskriminators BFD1 in der scheinbaren Bildebene SBE12 |
| BFD1 | erster Bildfeld-Diskriminator |
| | Der Bildfeld-Diskriminator BFD1 ist der Bildebene BEI1 zugeordnet. Ein Bildfeld-Diskriminator kann beispielsweise eine Spaltblende, ein Pinhole-Array, ein Durchlassbereich eines Flüssigkristall-Arrays oder ein schmaler reflektierender Bereich auf einem Spiegel oder einem Mikrospiegel-Array sein. |
| | Ein Bildfeld-Diskriminator weist einen nichtdiskriminierenden, also einen selektierenden Bereich auf, ausgebildet beispielsweise als feine Blendenöffnung zum Durchlassen von Licht oder einen reflektierenden Bereich, wobei nur das selektierte Licht letztlich zur Detektion kommt. |
| BFD2's | scheinbares Bild des zweiten Bildfeld-Diskriminators BFD2 in der scheinbaren Bildebene SBE12 |
| BFD2 | zweiter Bildfeld-Diskriminator, s. a. zu BFD1 Der Bildfeld-Diskriminator BFD2 ist der Bildebene BEI2 zugeordnet. |
| BFD1'r | reelles rückabgebildetes Bild des ersten Bildfeld-Diskriminators BFD1 auf dem Messobjekt |
| | Im justierten Zustand des Michelsontyp-Interferometers fallen die reellen Bilder BFD1 'r und BFD2'r zusammen. |
| BFD2'r | reelles rückabgebildetes Bild des zweiten Bildfeld-Diskriminators BFD2 auf dem Messobjekt |
| BTB | lateral begrenztes Teilbildbündel |
| | Dieses ist durch sowohl Strahlteilung als auch Begrenzung mittel optisch lateral begrenzender Mittel gebildet, z.B. letzteres durch eine Spaltblende, einen Streifenspiegel oder einen schmalen Spiegel. |
| C | Punkt an einem ausgewählten Pinhole einer Blendenscheibe 794 oder 797 |
| C' | reeller Bildpunkt des Punktes C auf einem Messobjekt |
| C" | reelles Bild des Bildpunktes C' auf der Blendenscheibe 794 oder 797 |
| C‴1s | scheinbarer Bildpunkt im ersten Interferometerarm IA1 nach Entfaltung des Strahlenganges |
| | Dieser scheinbare Bildpunkt C‴1e ist zu einem reellen Bildpunkt O"1 eines Messobjekts optisch konjugiert und fällt mit dem scheinbaren Bildpunkt O"1s, symbolisch dargestellt in den Figuren 19 und 20, zusammen. |
| C‴2s | scheinbarer Bildpunkt im zweiten Interferometerarm IA2 nach Entfaltung des Strahlenganges |
| | Dieser scheinbare Bildpunkt C "'2e ist zu einem reellen Bildpunkt O"2 eines Messobjekts optisch konjugiert und fällt mit dem scheinbaren Bildpunkt O"2s, symbolisch dargestellt in den Figuren 19 und 20, zusammen. |
| delta beta | Winkel zwischen den interferierenden Wellenfronten |
| sS | Summe der Lateral-Shear bei zwei Winkelspiegelgruppen |
| sD | Differenz der Lateral-Shear bei zwei Winkelspiegelgruppen |
| DE | Detektionsebene am Ausgang des Michelsontyp-Interferometers |
| EB | Eingangsstrahlenbündel, welches in der Regel ein fokussiertes Strahlenbündel ist |
| | Es bestehen am Eingang des Michelsontyp-Interferometers jedoch gleichzeitig viele Strahlenbündel, eines von jedem erfassten Objektpunkt, so dass ein Bild erzeugt werden kann. In den Figuren ist jedoch jeweils nur ein einziges Eingangsstrahlenbündel EB beispielhaft dargestellt, welches von einem einzigen Objektpunkt stammt. |
| effektive Spiegelfläche eines Michelsontyp-Interferometers | Die effektive Spiegelfläche eines Michelsontyp-Interferometers ist in einem Interferometerarm mit nur einem Endspiegel durch die reale Spiegelfläche dieses Endspiegels dargestellt, welche eine reelle Spiegelfläche RSF ist. |
| | Dagegen ist in einem Michelsontyp-Interferometer mit drei oder mehr als drei Spiegelflächen in einem Interferometerarm, welche eine Spiegelgruppe darstellen, die effektive Spiegelfläche in der Regel durch eine scheinbare Endspiegelfläche SEF dargestellt, die sich aus der Entfaltung der Spiegelgruppe ergibt. Eine scheinbare Endspiegelfläche SEF besteht nur, wenn sich in einem Arm des Michelsontyp-Interferometers mehr als ein einziger Endspiegel befindet. Dies ist bei einem Dreifach-Winkelspiegel oder bei einer (2n+1)-fach-Winkelspiegelgruppe mit n=2, 3, ... im Arm eines Michelsontyp-Interferometers in der Regel gegeben. |
| effektive Spiegelverkippung im Michelsontyp-Interferometer | Die effektive Spiegelverkippung alpha_SMI im Michelsontyp-Interferometer ergibt sich nach der Entfaltung des Michelsontyp-Interferometers als verbleibende Verkippung der beiden effektiven Endspiegelflächen, die wiederum real oder auch scheinbar sein können. Die Schwerstrahlen der beiden Teilstrahlenbündel TB1 und TB2 am Ausgang des Michelsontyp-Interferometers sind dann stets um den Winkel 2*alpha_SMI zueinander geneigt. Bei einem ideal justierten Michelsontyp-Interferometer ist alpha_SMI gleich null. |
| EHS | Eingangshauptstrahl |
| Endreflektor | Ein Endreflektor kann als ein einzelner Spiegel, bevorzugt als ein Planspiegel, ausgebildet sein, aber auch eine Anordnung mit mehreren Spiegeln darstellen. |
| F511 (F521, F531) | vorderer Brennpunkt des Objektivs 511 im nachgeordneten anamorphotischen Objektiv 51 (52, 53) in der yz-Ebene, welche die Referenzebene RE darstellt |
| F'511 (F'521, F'531) | hinterer Brennpunkt des Objektivs 511 im nachgeordneten anamorphotischen Objektiv 51 (52, 53) in der yz-Ebene, welche die Referenzebene RE darstellt |
| f'511 (f'521, f'531) | hinterere Brennweite des Objektivs 511 (521, 531, 581, 591) im nachgeordneten anamorphotischen Objektiv 51 (52, 53, 58, 59) |
| | Es ist f'511= f'51yz. |
| | (f521=f'52yz, f'531=f'53yz) |
| F5lxz (F52xz, F53xz) | vorderer Brennpunkt des anamorphotischen Objektivs 51 (52, 53) in der in der xz-Ebene |
| F'51xz (F'52xz, F'53xz) | hinterer Brennpunkt des anamorphotischen Objektivs 51 (52, 53, 58, 59) in der in der xz-Ebene |
| | Es ist F'51 1 = F'51yz. |
| | (F'521=F'52yz, F'531=F'53yz) |
| F51yz (F52yz, F53yz) | vorderer Brennpunkt des anamorphotischen Objektivs 51 (52, 53) in der yz-Ebene, welche die Referenzebene RE darstellt |
| | Es ist F511= F51yz. |
| | (F521=F52yz,F531=F53yz) |
| F'51yz (F'52yz, F'53yz) | hinterer Brennpunkt des anamorphotischen Objektivs 51 (52, 53) in der yz- Ebene, welche die Referenzebene RE darstellt |
| FFT | schnelle Fourier-Transformation, ein effektiver Algorithmus, der zur Berechnung eines Spektrums aus einem Interferogramm genutzt wird |
| FIR | fern-infraroter Spektralbereich |
| Michelsontyp-Interferometer | Bei einem Michelsontyp-Interferometer sind das hinlaufende und das rücklaufende Bündel in jedem Arm desselben zumindest näherungsweise parallel. |
| | Die reelle Spiegelfläche (RSF), dargestellt durch einen einzigen Planspiegel in einem Interferometerarm oder die scheinbare Endspiegelfläche (SEF) bei einer Mehrspiegel-Anordnung in einem Interferometerarm, stehen in der Regel zumindest näherungsweise senkrecht zur Referenzebene RE. Die Teilstrahlenbündel TB 1 und TB2 fallen bei einem Michelsontyp-Interferometer zumindest näherungsweise senkrecht auf eine Spiegelfläche (RSF) oder eine scheinbare Endspiegelfläche (SEF) ein. |
| MIR | mittel-infraroter Spektralbereich |
| NIR | nah-infraroter Spektralbereich |
| NT | Normale der Strahlteilerebene, welche mit der optischen Achse OAI die Referenzebene RE aufspannt |
| | Die Strahlteilerebene ist durch eine Strahlteilerschicht 62 oder durch eine Mylarfolie 623 oder durch ein Gitter dargestellt. |
| O | beleuchteter oder selbstleuchtender Objektpunkt auf dem Messobjekt |
| O' | reeller Bildpunkt des Objektpunkts O |
| O'1 | reeller Bildpunkt des Objektpunkts O im ersten Interferometerarm IA1 |
| O'1s | scheinbarer Bildpunkt vom Objektpunkt O nach einmaliger Abbildung O'1s ist dem ersten Arm lAldes Michelsontyp-Interferometers zugeordnet. |
| | Der scheinbare Bildpunkt O'1s liegt in der scheinbaren Bildebene SBE1 und und bei hinreichend präziser Justierung des Michelsontyp-Interferometers auch in der scheinbaren Bildebene SBE12 vor dem anamorphotischen Objektiv 51 (52, 53, 537). |
| | Bei den Tiefenpositionen von scheinbaren Bildpunkten sind hier auch Tiefenverschiebungen durch refraktive Materialien berücksichtigt, wenn vorhanden. |
| O"1s | vollständig entfalteter, also scheinbarer Bildpunkt vom Objektpunkt O vor dem anamorphotischen Objektiv 51, der aus dem ersten Arm IA1 des Michelsontyp-Interferometers stammt, bei einem zweistufigen, vorgeordneten Objektiv 74 oder einer konfokalen Anordnung 751 |
| O'1 -unscharf | unscharfer Bildfleck des Objektpunkts O im ersten Interferometerarm IA1 durch Astigmatismus |
| O'2 | reeller Bildpunkt des Objektpunkts O im zweiten Interferometerarm IA2 |
| O '2s | scheinbarer Bildpunkt vom Obj ektpunkt O nach einmaliger Abbildung, der dem zweiten Arm IA2 des Michelsontyp-Interferometers zugeordnet ist |
| | Der scheinbare Bildpunkt O'2s liegt in der scheinbaren Bildebene SBE2 und bei hinreichend präziser Justierung des Michelsontyp-Interferometers auch in der scheinbaren Bildebene SBE12 vor dem anamorphotischen Objektiv 51 (52, 53, 537). |
| | Bei den Tiefenpositionen von scheinbaren Bildpunkten sind hier auch Tiefenverschiebungen durch refraktive Materialien berücksichtigt, wenn vorhanden, beispielsweise bei Vorhandensein eines Strahlteilerwürfels im Interferometer aus optischem Glas. |
| O "2s | scheinbarer Bildpunkt vom Objektpunkt O vor dem anamorphotischen Objektiv 51, der aus dem zweiten Arm IA2 des Michelsontyp-Interferometers stammt, bei einem zweistufigen, vorgeordneten Objektiv 74 oder einer konfokalen Anordnung 751 |
| O"2-unscharf | unscharfer Bildfleck durch Astigmatismus des Objektpunkts O im zweiten Interferometerarm IA2 |
| O‴2-unscharf | unscharfer Bildfleck durch Astigmatismus des Objektpunkts O im zweiten Interferometerarm IA2 |
| O'1e | entfalteter Bildpunkt des Objektpunkts O nach Entfaltung des ersten Interferometerarms IA1 in der scheinbaren Endspiegelfläche SEF1, O"le und O"2e stellen kohärente Lichtquellenpunkte dar. |
| O'2e | entfalteter Bildpunkt des Objektpunkts O nach Entfaltung des zweiten Interferometerarms IA2 in der scheinbaren Endspiegelfläche SEF2 im Fall der Bildfeld-Diskriminierung im Michelsontyp-Interferometer |
| O" | Bildpunkt vom Objektpunkt O nach zweimaliger Abbildung |
| O"1_xz | Bildpunkt des Objektpunkts O auf dem gerasterten Detektor in der xz-Ebene |
| O"1s | scheinbarer Bildpunkt des Objektpunkts O in der scheinbaren Bildebene SBE1 nach Entfaltung des ersten Interferometerarms IA1, beispielsweise in einer konfokalen Anordnung 751 (in Figur 18) im Fall der Bildfeld-Diskriminierung vor dem Michelsontyp-Interferometer |
| O"2s | scheinbarer Bildpunkt des Objektpunkts O in der scheinbaren Bildebene SBE2 nach Entfaltung des zweiten Interferometerarms IA2, beispielsweise in einer konfokalen Anordnung 751 (in Figur 18) im Fall der Bildfeld-Diskriminierung vor dem Michelsontyp-Interferometer |
| OAI | optische Achse des vorgeordneten Objektivs 70, 71, 73, 752, 78, 781, 752, welche dem des Michelsontyp-Interferometers IA2 in der Regel zugewandt ist |
| | Im entfalteten Zustand der Anordnung fällt die optische Achse OAI mit der z-Achse zusammen. |
| P | Punkt an einem Flüssigkristall-Display (LCD) 79, beispielsweise an einem Pixel desselben |
| OE | Objektelement |
| | Die Objektelemente OE_1, OE_2 ... OE_n sind in x-Richtung angeordnet, die so zusammen ein streifenförmiges Messfeld bilden. |
| P' | Bild des Punktes P nach Abbildung durch das erste Objektiv 761 |
| P" | Bild des Punktes P nach Abbildung durch das zweite Objektiv 781 |
| P‴1s | scheinbarer Bildpunkt vom Punkt P in der scheinbaren Bildebene SBE12 nach Entfaltung des ersten Interferometerarms IA1 |
| | Der scheinbare Bildpunkt P"'1s fällt (hier in Figur 18) mit einem scheinbaren Objektpunkt O"1s zusammen. |
| P‴2s | scheinbarer Bildpunkt vom Punkt P in der scheinbaren Bildebene SBE12 nach Entfaltung des zweiten Interferometerarms IA2 |
| | Der scheinbare Bildpunkt P"'2s fällt (hier in Figur 18) mit einem scheinbaren Objektpunkt O"2s zusammen. |
| RE | Referenzebene, welche durch die optische Achse OAI des dem Michelsontyp-Interferometer vorgeordneten Objektivs (70, 71, 73, 75, 781, 752) und die Normale der ebenen Strahlteilerschicht 62, 625, 628 oder der Mylarfolie 623 aufgespannt ist. |
| | Der gerasterte Detektor steht im Standardfall senkrecht auf der Referenzebene RE. |
| rI | räumliches Interferogramm |
| RSF | reelle Spiegelfläche |
| | Die reelle Spiegelfläche ist hier in der Regel im ersten Interferometerarm IA1 dargestellt und ist eine Endspiegelfläche. Diese reelle Spiegelfläche ist deckungsgleich mit einem realen planen Endspiegel 633, 638, 679 oder auch mit dem digitalen Mikrospiegel-Array 681, wenn dieses als Endspiegel eingesetzt ist. |
| RZ | Radius einer Zylinderwelle, der auf dem gerasterten Detektor sehr klein wird, bzw. gegen null geht |
| s | Lateral-Shear nach Passieren einer (2n+1)-fach-Winkelspiegelgruppe mit n=1, 2, 3 - wie oben beschrieben - für den Fall, dass nur eine einzige (2n+1)-fach-Winkelspiegelgruppe im Interferometer angeordnet ist. Die Lateral-Shear stellt den Querversatz von kohärenten Bildpunkten dar. |
| s1 | Lateral-Shear nach Passieren einer ersten (2n+1)-fach-Winkelspiegelgruppe mit n=1, 2, 3 - wie oben beschrieben |
| s2 | Lateral-Shear nach Passieren einer zweiten (2n+1)-fach-Winkelspiegelgruppe mit n=1, 2, 3 - wie oben beschrieben |
| SBE1 | scheinbare Bildebene vor dem anamorphotischen Objektiv 51 (52, 53, 537) aus dem Interferometerarm IA1 mit dem scheinbaren Bildpunkt O'1s, beziehungsweise ggf. auch O"1s, ggf. auch C‴1s sowie ggf. auch P‴1s |
| SBE2 | scheinbare Bildebene vor dem anamorphotischen Objektiv 51 (52, 53, 537) aus dem Interferometerarm IA2 mit dem scheinbaren Bildpunkt O'2s, beziehungsweise ggf. auch O"2s, ggf. auch C'"2s sowie ggf. auch P‴2s. |
| | Die beiden scheinbaren Bildebenen SBE1 und SBE2, welche die gleiche Tiefenlage aufweisen, liegen bei hinreichend präziser Justierung des Michelsontyp-Interferometers auch in der scheinbaren Bildebene SBE12. |
| SBE12 | scheinbare Bildebene vor dem anamorphotischen Objektiv 51 (52, 53, 537) mit den scheinbaren Bildpunkten O'1s und O'2s |
| | In der scheinbaren Bildebene SBE12 liegen auch die beiden scheinbaren Bildebenen SBE1 und SBE2, welche die gleiche Tiefenlage aufweisen. Die scheinbare Bildebene SBE12 und auch die beiden scheinbaren Bildebenen SBE1 und SBE2 sind in Figur 5 und den Figuren 13 und 14 gemeinsam dargestellt. |
| | (In den übrigen Figuren wurde auf die Darstellung der beiden scheinbaren Bildebenen SBE1 und SBE2 verzichtet und nur die scheinbare Bildebene SBE12 dargestellt.) In der scheinbaren Bildebene SBE12 liegen auch stets die scheinbaren Bildpunkte von Objektpunkten, welche mit Lateral-Shear s separiert sind, hier z.B. die Bildpunkte O'1s und O'2s sowie O'Ts und O"2s. Die scheinbaren Bildpunkte (O'1s und O'2s sowie O"1s und O"2s) sind jeweils optisch kohärent, also interferenzfähig. Auch Bildpunkte (C‴1s und C‴2s in Figur 18 und 19 sowie P‴1s und P‴2s in Figur 18) von den Punkten C und P sind in der scheinbaren Bildebene SBE12 mit Lateral-Shear s separiert. |
| | Eine gemeinsame Tiefenlage der beiden scheinbaren Bildebenen SBE1 und SBE2 in der Tiefe, die das Modell einer scheinbaren Bildebene SBE12 rechtfertigt, setzt bekannterweise neben der Balancierung (Tiefenabgleich) des Interferometers jedoch auch eine hinreichend präzise winkelmäßige Justierung des Michelsontyp-Interferometers voraus. Dies bedeutet, dass die selektierten Wellenfronten direkt am Ausgang des Michelsontyp-Interferometers und vor dem anamorphotischen Objektiv (51, 52, 53, 537), die zu den beiden selektierten Teilstrahlenbündel gehören, maximal um eine halbe Wellenlänge zueinander verkippt sind. Der Wert "halbe Wellenlänge" ist auf die laterale Ausdehnung dieser Wellenfronten in einer Richtung parallel zur Referenzebene RE und quer zur Lichtausbreitungsrichtung bezogen, wodurch sich der Kippwinkel zwischen den Wellenfronten ergibt. Dabei steht die Kippachse senkrecht zur Referenzebene RE. Dies muss für Licht der kürzesten Wellenlänge eingehalten sein, das noch zur Detektion kommt und somit zur Spektrenberechnung verwendet wird. Diese präzise Justierung des Michelsontyp-Interferometers ist zwingend erforderlich, um bei der Detektion der räumlichen Interferogramme einen hohen Kontrast von mindestens 50% erreichen zu können, um letztlich vergleichsweise rauscharme Spektren errechnen zu können. Diese Zusammenhänge sind Fachleuten der Interferometrie bestens bekannt. Von diesem hinreichend präzisen Justierzustand des Michelsontyp-Interferometers wird in dieser Schrift stets ausgegangen. |
| SEF1 | scheinbare Endspiegelfläche im ersten Interferometerarm IA1 im Sinne einer Ersatzspiegelfläche |
| | Die scheinbare Endspiegelfläche SEF1 ergibt sich aus der Entfaltung einer Dreifach-Winkelspiegelgruppe, die im ersten Interferometerarm IA1 angeordnet ist. Das gilt auch für die Entfaltung einer (2n+1)-fach-Winkelspiegelgruppe, mit n=1, 2, 3, .... |
| SEF2 | scheinbare Endspiegelfläche im zweiten Interferometerarm IA2 im Sinne einer Ersatzspiegelfläche |
| | Die scheinbare Endspiegelfläche SEF2 ergibt sich aus der Entfaltung einer Dreifach-Winkelspiegelgruppe, die im zweiten Interferometerarm IA2 angeordnet ist. Das gilt auch für die Entfaltung einer (2n+1)-fach-Winkelspiegelgruppe, mit n=1, 2, 3, .... |
| Sp | Spektrum, welches aus einem räumlichem Interferogramm mittels FFT errechnet wurde |
| TB1 | erstes Teilstrahlenbündel nach Strahlteilung |
| | Es gibt jedoch gleichzeitig viele Teilstrahlenbündel, die von jeweils einem Objektpunkt stammen. Das in den Figuren dargestellte Teilstrahlenbündel TB1, resultierend aus dem Objektpunkt O, steht also nur stellvertretend für eine Vielzahl derselben. Einem Teilstrahlenbündel ist auch eine Wellenfront zugeordnet, beispielsweise am Ausgang des Interferometers. |
| TB2 | zweites Teilstrahlenbündel nach Strahlteilung |
| | Es gibt jedoch gleichzeitig viele Teilstrahlenbündel, die von jeweils einem Objektpunkt stammen. Das in den Figuren dargestellte Teilstrahlenbündel TB2, resultierend aus dem Objektpunkt O, steht also nur stellvertretend für eine Vielzahl derselben. |
| VIS | visueller Spektralbereich |
| x-Richtung | senkrecht zur Referenzebene RE |
| | Die Längsrichtung eines Streifens, bzw. Längsrichtung einer Streifenlichtquelle oder auch eines Spaltes ist parallel zur x-Richtung. |
| y-Richtung | parallel zur Referenzebene RE |
| | Die Richtung der Lateral-Shear - im entfalteten Zustand - ist parallel zur y-Richtung. |
| z-Richtung | Ausbreitungsrichtung des Interferenzlichts in Richtung Detektion Die z-Richtung ist parallel zur optischen Achse OAI. |

## Patentansprüche

1. Fourier-Transformations-Spektrometer, FT-Spektrometer, umfassend:
Michelsontyp-Interferometer (601; 602; 603; 604; 605; 606; 607; 608, 609) umfassend:
zumindest eine Strahlteilereinheit (57; 571; 621; 622; 623; 624; 627), wobei die Strahlteilereinheit (57; 571; 621; 622; 623; 624; 627) ausgelegt ist,
zum Aufspalten eines einfallenden Lichtbündels (EB) eines räumlich ausgedehnten Objekts, in ein erstes Teilstrahlenbündel (TB1) und ein zweites Teilstrahlenbündel (TB2); und
zum zumindest teilweisen Überlagern des ersten Teilstrahlenbündels (TB1) und des zweiten Teilstrahlenbündels (TB2) mit einer Lateral-Shear (s);
eine erste Strahlumlenkungseinheit (640; 641; 642; 643; 644; 652; 653; 662; 678) die dazu ausgelegt ist, das erste Teilstrahlenbündel (TB1) zumindest ein erstes Mal abzulenken;
eine zweite Strahlumlenkungseinheit (640; 641; 642; 643; 644; 652; 653; 662; 678) die dazu ausgelegt ist, das zweite Teilstrahlenbündel (TB2) zumindest ein erstes Mal abzulenken;
wobei mindestens eine aus der ersten Strahlumlenkungseinheit (640; 641; 642; 643; 644; 652; 653; 662; 678) und der zweiten Strahlumlenkungseinheit (640; 641; 642; 643; 644; 652; 653; 662; 678) eine (2n+1)-Winkelspiegelgruppe (640; 641; 642; 643; 644; 652; 653; 662; 678) mit (2n+1) Spiegelflächen (663, 664, 665) darstellt und alle (2n+1) Spiegelflächen senkrecht zu einer gemeinsamen Referenzebene angeordnet sind, um jeweils das erste Teilstrahlenbündel (TB1) und/oder das zweite Teilstrahlenbündel (TB2) (2n+1) mal abzulenken und wobei das (2n+1)-fache Ablenken die Lateral-Shear (s) zwischen dem ersten Teilstrahlenbündel (TB1) und dem zweiten Teilstrahlenbündel (TB2) erzeugt und wobei n eine natürliche Zahl ≥1 ist;
wobei das FT-Spektrometer ferner umfasst:
zumindest ein Objektiv (70; 71; 73; 752; 78; 781), das so vor der Strahlteilereinheit (57; 571; 621; 622; 623; 624; 627) angeordnet ist, dass das einfallende Lichtbündel (EB) das Objektiv zumindest teilweise vor dessen Aufspaltung an der Strahlteilereinheit (57; 571; 621; 622; 623; 624; 627) passiert und fokussiert wird und das erste Teilstrahlenbündel (TB1) und das zweite Teilstrahlenbündel (TB2) jeweils eine Vielzahl von kohärenten Bildpunkten (O'1, O'2) des räumlich ausgedehnten Objekts in einer Bildebene (BEI1, BEI2) in Lichtrichtung nach der Strahlteilereinheit (57; 571; 621; 622; 623; 624; 627) und vor einem Detektor (54) erzeugt;
den Detektor (54; 55; 58; 59) zur Erfassung einer Vielzahl von räumlichen Interferogrammen (rI1, rI2, rI3, rI4) auf Basis der räumlichen Überlagerung des ersten Teilstrahlenbündels (TB1) und des zweiten Teilstrahlenbündels (TB2), die der zumindest teilweisen Abbildung der Vielzahl von kohärenten Bildpunkten (O'1, O'2) entspricht; und
zumindest eine Recheneinheit (21) zur Fourier-Transformation der Vielzahl von räumlichen Interferogrammen (rIl, rI2, rI3, rI4), zur Erzeugung einer Vielzahl von Spektren (Sp1, Sp2, Sp3, Sp4) und darauf basierend zur Erzeugung zumindest eines Teilbereiches mindestens eines hyperspektralen Bildes des räumlich ausgedehnten Objekts,
zumindest eine der Strahlteilereinheit (57; 571; 621; 622; 623; 624; 627) in Lichtrichtung nachgeordnete Bildfeld-Diskriminatoreinheit (BFD1, BFD2, 633, 634, 635, 638, 654, 655, 666, 678-2) umfasst, welche derart angeordnet ist, dass das erste Teilstrahlenbündel (TB1) und/oder das zweite Teilstrahlenbündel (TB2) räumlich selektiert wird; und
ein anamorphotisches Objektiv (51, 52, 53, 537) zur Erzeugung der räumlichen Interferogramme (rI1, rI2, rI3, rI4) aus dem selektierten Bildfeld.

2. FT-Spektrometer nach Anspruch 1, wobei die Referenzebene durch die Normale der Strahlteilereinheit (57; 571; 621; 622; 623; 624; 627) und durch die optische Achse (OAI) des vorgeordneten Objektivs (70; 71; 73; 752; 78; 781 aufgespannt ist.

3. FT-Spektrometer nach Anspruch 1 oder 2, wobei die (2n+1)-Winkelspiegelgruppe einer Rachen- oder einer W-Form entspricht.

4. FT-Spektrometer nach einem der vorangehenden Ansprüche, wobei jede der Spiegelflächen dazu angeordnet ist, das erste Teilstrahlenbündel (TB1) oder das zweite Teilstrahlenbündel (TB2) ein einziges Mal zu reflektieren.

5. FT-Spektrometer nach einem der vorangehenden Ansprüche,
wobei mindestens eine aus der ersten Strahlumlenkungseinheit (640; 641; 642; 643; 644; 652; 653; 662; 678) und der zweiten Strahlumlenkungseinheit (640; 641; 642; 643; 644; 652; 653; 662; 678) dazu ausgelegt ist, das erste Teilstrahlenbündel (TB1) oder das zweite Teilstrahlenbündel (TB2) ein einziges Mal mittels einmaliger Reflexion an einer Spiegelfläche der entsprechenden Strahlumlenkungseinheit (640; 641; 642; 643; 644; 652; 653; 662; 678) abzulenken; und/oder
wobei mindestens eine aus der ersten Strahlumlenkungseinheit (640; 641; 642; 643; 644; 652; 653; 662; 678) und der zweiten Strahlumlenkungseinheit (640; 641; 642; 643; 644; 652; 653; 662; 678) dazu ausgelegt ist, das erste Teilstrahlenbündel (TB1) und/oder das zweite Teilstrahlenbündel (TB2) drei Mal mittels dreimaliger Reflexion an drei Spiegelflächen einer Dreifach-Winkelspiegelgruppe der entsprechenden Strahlumlenkungseinheit (640; 641; 642; 643; 644; 652; 653; 662; 678) abzulenken.

6. FT-Spektrometer nach einem der vorangehenden Ansprüche, wobei die erste Strahlumlenkungseinheit (640; 641; 642; 643; 644; 652; 653; 662; 678) und die zweite Strahlumlenkungseinheit (640; 641; 642; 643; 644; 652; 653; 662; 678) zusammen eine Anzahl von Spiegelflächen aufweist, die entweder (2n+1+1) oder (2n₁+1+2n₂+1) entspricht, wobei n eine natürliche Zahl ≥1, n₁ eine natürliche Zahl ≥1 und n₂ eine natürliche Zahl ≥1 ist.

7. FT-Spektrometer nach einem der vorangehenden Ansprüche,
wobei zwischen mindestens zwei der (2n+1) Spiegelflächen der (2n+1)-Winkelspiegelgruppe der zumindest eine Strahlumlenkungseinheit (640; 641; 642; 643; 644; 652; 653; 662; 678) zumindest eine Bildfeld-Diskriminatoreinheit (BFD1, BFD2, 633, 634, 635, 638, 654, 655) derart angeordnet ist, dass das erste Teilstrahlenbündel (TB1) und/oder das zweite Teilstrahlenbündel (TB2) räumlich selektiert wird.

8. FT-Spektrometer nach Anspruch 7, wobei zumindest eine Bildfeld-Diskriminatoreinheit in einer der Spiegelflächen der ersten Strahlumlenkungseinheit (640; 641; 642; 643; 644; 652; 653; 662; 678) und/oder der zweiten Strahlumlenkungseinheit (640; 641; 642; 643; 644; 652; 653; 662; 678) integriert ist.

9. FT-Spektrometer nach Anspruch 7 oder 8, wobei das Michelsontyp-Interferometer (601, 602, 603, 604, 605, 606, 607) ferner zumindest eine der Strahlteilereinheit (57; 571; 621; 622; 623; 624; 627) in Lichtrichtung nachgeordnete erste und eine zweite Bildfeld-Diskriminatoreinheit (BFD1, BFD2, 633, 634, 635, 638, 654, 655) umfasst, welche derart angeordnet sind, dass das erste Teilstrahlenbündel (TB1) und das zweite Teilstrahlenbündel (TB2) räumlich selektiert werden und dass die erste Bildfeld-Diskriminatoreinheit (BFD1, BFD2, 633, 634, 635, 638, 654, 655) optisch konjugiert zur zweiten Bildfeld-Diskriminatoreinheit (BFD1, BFD2, 633, 634, 635, 638, 654, 655) ist.

10. FT-Spektrometer nach einem der Ansprüche 7 bis 9, wobei die Bildfeld-Diskriminatoreinheit zumindest eines der folgenden aufweist:
eine spaltförmige Abschattblende (666),
ein Mikrospalt-Abschattblenden-Array,
eine Pinhole-Abschattblende (794, 797),
ein eindimensionales oder ein zweidimensionales Pinhole-Abschattblenden-Array (795) in Form einer Blendenscheibe,
ein Mikrospalten-Abschattblenden-Array mit einer Vielzahl von Mikrospalten in lateral versetzter Anordnung,
ein Mikrospalten-Abschattblenden-Array mit mechanisch beweglichen Elementen,
ein reflektiver Bereich spaltförmiger Bereich, der einen Teil der ersten und/oder der zweiten Strahlumlenkungseinheit (640; 641; 642; 643; 644; 652; 653; 662; 678) darstellt.

11. FT-Spektrometer nach einem der vorangehenden Ansprüche, wobei zumindest eine aus der ersten Strahlumlenkungseinheit (640; 641; 642; 643; 644; 652; 653; 662; 678) und der zweiten Strahlumlenkungseinheit (640; 641; 642; 643; 644; 652; 653; 662; 678) ein Prisma mit zumindest einer Reflexionsfläche aufweist, die dazu ausgelegt ist, das erste Teilstrahlenbündel (TB1) und/oder das zweite Teilstrahlenbündel (TB2) zumindest einmal zu reflektieren.

12. FT-Spektrometer nach einem der vorangehenden Ansprüche, ferner umfassend eine dem Michelsontyp-Interferometer (609) vorgeordnete konfokale Anordnung (751),
wobei optional die konfokale Anordnung (751) eine starre Blendenscheibe (794) oder eine rotierende Blendenscheibe (797) und/oder einen räumlichen Lichtmodulator in Reflexion oder Transmission aufweist.

13. FT-Spektrometer nach einem der vorangehenden Ansprüche, wobei die Strahlteilereinheit (57; 571; 621; 622; 623; 624; 627) einen Amplituden-Strahlteiler darstellt und eine ebene Strahlteilerschicht (62, 625, 628), eine Mylarfolie (623) oder ein Gitter aufweist.

14. Verfahren zur interferometrischen Messung mittels eines FT-Spektrometers mit Michelsontyp-Interferometer (601, 602, 603, 604, 605, 606, 607, 608, 609) umfassend:
Aufspalten eines einfallenden Lichtbündels, welcher von einem räumlich ausgedehnten Objekt ausgesendet wird, in einen ersten Teilstrahlenbündel und einen zweiten Teilstrahlenbündel mittels einer Strahlteilereinheit (57; 571; 621; 622; 623; 624; 627);
zumindest einmaliges Ablenken des ersten Teilstrahlenbündels mittels einer ersten Strahlumlenkungseinheit (640; 641; 642; 643; 644; 652; 653; 662; 678);
zumindest einmaliges Ablenken des zweiten Teilstrahlenbündels mittels einer zweiten Strahlumlenkungseinheit (640; 641; 642; 643; 644; 652; 653; 662; 678); wobei an mindestens einer aus der ersten Strahlumlenkungseinheit (640; 641; 642; 643; 644; 652; 653; 662; 678) und der zweiten Strahlumlenkungseinheit (640; 641; 642; 643; 644; 652; 653; 662; 678) das erste Teilstrahlenbündel (TB1) und/oder das zweite Teilstrahlenbündel (TB2) (2n+1) mal abgelenkt wird mittels einer (2n+1)-Winkelspiegelgruppe mit (2n+1) Spiegelflächen, um eine Lateral-Shear (s) zwischen dem ersten Teilstrahlenbündel (TB1) und dem zweiten Teilstrahlenbündel (TB2) zu erzeugen und wobei n eine natürliche Zahl ≥1 ist;
Senden des einfallenden Lichtbündels durch ein Objektiv vor dem Aufspalten, zur Erzeugung einer Vielzahl von kohärenten Bildpunkten des räumlich ausgedehnten Objekts in einer Bildebene (BEI1, BEI2) zwischen der Strahlteilereinheit (57; 571; 621; 622; 623; 624; 627) und einem Detektor;
räumliches zumindest teilweises Überlagern des ersten Teilstrahlenbündels und des zweiten Teilstrahlenbündels mittels der Strahlteilereinheit (57; 571; 621; 622; 623; 624; 627);
zumindest teilweises Abbilden der Vielzahl von kohärenten Bildpunkten bei gleichzeitigem Erzeugen einer Vielzahl von räumlichen Interferogrammen auf einem Detektorfeld des Detektors auf Basis der räumlichen Überlagerung;
Erfassen der Vielzahl von räumlichen Interferogrammen mittels des Detektors; und
Fourier-Transformation der Vielzahl von räumlichen Interferogrammen zur Erzeugung einer Vielzahl von Spektren und darauf basierend Erzeugung eines hyperspektralen Bildes zumindest eines Ausschnitts des räumlich ausgedehnten Objekts, wobei das Verfahren ferner umfasst:
räumliches Selektieren des ersten Teilstrahlenbündels (TB1) und/oder des zweiten Teilstrahlenbündels (TB2) mittels zumindest einer der Strahlteilereinheit (57; 571; 621; 622; 623; 624; 627) in Lichtrichtung nachgeordneten Bildfeld-Diskriminatoreinheit (BFD1, BFD2, 633, 634, 635, 638, 654, 655, 666, 678-2) oder mittels zumindest einer dem Michelsontyp-Interferometer vorgeordneter Bildfeld-Diskriminatoreinheit; und
Erzeugung der räumlichen Interferogramme (rI1, rI2, rI3, rI4) aus dem selektierten Bildfeld mittels eines anamorphotischen Objektivs (51, 52, 53, 537).

15. Verfahren zur interferometrischen Messung nach Anspruch 14, ferner umfassend die Schritte, die ausgelöst und zumindest teilweise ausgeführt werden mittels zumindest einer Recheneinheit (21):
mehrmaliges gleichzeitiges Erfassen der Vielzahl von räumlichen Interferogrammen zu jeweils unterschiedlichen Zeitpunkten;
Fourier-Transformierung der zu jeweils unterschiedlichen Zeitpunkten erfassten Vielzahl von räumlichen Interferogrammen zur Erzeugung einer Vielzahl von Spektren; und
Erzeugung mindestens eines hyperspektralen Bildes des räumlich ausgedehnten Objekts.

## Claims

1. Fourier transformation spectrometer (FT spectrometer) comprising:
a Michelson-type interferometer (601; 602; 603; 604; 605; 606; 607; 608, 609) comprising:
at least one beam splitter unit (57; 571; 621; 622; 623; 624; 627), wherein the beam splitter unit (57; 571; 621; 622; 623; 624; 627) is designed to
split an incident light beam (EB) of a spatially expanded object into a first partial beam (TB1) and a second partial beam (TB2); and
at least partially overlay the first partial beam (TB 1) and the second partial beam (TB2) with a lateral shear (s);
a first beam deflection unit (640; 641; 642; 643; 644; 652; 653; 662; 678) designed to deflect the first partial beam (TB1) at least a first time;
a second beam deflection unit (640; 641; 642; 643; 644; 652; 653; 662; 678) designed to deflect the second partial beam (TB2) at least a first time;
wherein at least one among the first beam deflection unit (640; 641; 642; 643; 644; 652; 653; 662; 678) and the second beam deflection unit (640; 641; 642; 643; 644; 652; 653; 662; 678) represents a (2n+1) periscope group (640; 641; 642; 643; 644; 652; 653; 662; 678) with (2n+1) mirror surfaces (663, 664, 665) and all (2n+1) mirror surfaces are arranged vertically in relation to a common reference plane, in order to respectively deflect the first partial beam (TB1) and/or the second partial beam (TB2) (2n+1) times, and wherein the (2n+1)-fold deflection generates the lateral shear (s) between the first partial beam (TB1) and the second partial beam (TB2), and wherein n is a natural number ≥1 ;
wherein the FT spectrometer further comprises:
at least one lens (70; 71; 73; 752; 78; 781) arranged opposite the beam splitter unit (57; 571; 621; 622; 623; 624; 627) such that the incident light beam (EB) passes the lens at least partially before the light beam is split on the beam splitter unit (57; 571; 621; 622; 623; 624; 627) and the first partial beam (TB1) and the second partial beam (TB2) respectively generate a plurality of coherent image points (O'1, O'2) of the spatially expanded object in an image plane (BEI1, BEI2) in the light direction downstream of the beam splitter unit (57; 571; 621; 622; 623; 624; 627) and upstream of a detector (54);
the detector (54; 55; 58; 59) to record a plurality of spatial interferograms (rI1, rI2, rI3, rI4) on the basis of the spatial overlay of the first partial beam (TB1) and the second partial beam (TB2), which corresponds at least to the partial imaging of the plurality of coherent image points (O'1, O'2); and
at least one computing unit (21) for the Fourier transformation of the plurality of spatial interferograms (rI1, rI2, rI3, rI4) to generate a plurality of spectra (Sp1, Sp2, Sp3, Sp4), and based thereon, to generate at least a partial area of at least one hyperspectral image of the spatially expanded object,
at least one field of view discriminator unit (BFD1, BFD2, 633,634, 635, 638, 654, 655, 666, 678-2) arranged downstream of the beam splitter unit (57; 571; 621; 622; 623; 624; 627) in the light direction, which field of view discriminator unit is arranged such that the first partial beam (TB 1) and/or the second partial beam (TB2) is spatially selected, and
an anamorphic lens (51, 52, 53, 537) for generating the spatial interferograms (rI1, rI2, rI3, rI4) from the selected field of view.

2. FT spectrometer according to claim 1, wherein the reference plane is spanned by the normal of the beam splitter unit (57; 571; 621; 622; 623; 624; 627) and by an optical axis (OA1) of the upstream lens (70; 71; 73; 752; 78; 781).

3. FT spectrometer according to claim 1 or 2, wherein the (2n+1) periscope group corresponds to a throat or a W shape.

4. FT spectrometer according to any one of the preceding claims, wherein each of the mirror surfaces is arranged to reflect the first partial beam (TB1) or the second partial beam (TB2) once.

5. FT spectrometer according to any one of the preceding claims,
wherein at least one of the first beam deflection unit (640; 641; 642; 643; 644; 652; 653; 662; 678) and the second beam deflection unit (640; 641; 642; 643; 644; 652; 653; 662; 678) is designed to deflect the first partial beam (TB 1) or the second partial beam (TB2) once by means of single reflection on a mirror surface of the corresponding beam deflection unit (640; 641; 642; 643; 644; 652; 653; 662; 678); and/or
wherein at least one of the first beam deflection unit (640; 641; 642; 643; 644; 652; 653; 662; 678) and the second beam deflection unit (640; 641; 642; 643; 644; 652; 653; 662; 678) is designed to deflect the first partial beam (TB 1) and/or the second partial beam (TB2) three times by means of triple reflection on three mirror surfaces of a triple periscope group of the corresponding beam deflection unit (640; 641; 642; 643; 644; 652; 653; 662; 678).

6. FT spectrometer according to any one of the preceding claims, wherein the first beam deflection unit (640; 641; 642; 643; 644; 652; 653; 662; 678) and the second beam deflection unit (640; 641; 642; 643; 644; 652; 653; 662; 678) together have a number of mirror surfaces that either corresponds to (2n+1+1) or (2n₁+1+2n₂+1), and wherein n is a natural number ≥1, n₁ is a natural number ≥1 and n₂ is a natural number ≥1.

7. FT spectrometer according to any one of the preceding claims,
wherein at least one field of view discriminator unit (BFD1, BFD2, 633, 634, 635,638,654, 655) is arranged between at least two of the (2n+1) mirror surfaces of the (2n+1) periscope group of the at least one beam deflection unit (640; 641; 642; 643; 644; 652; 653; 662; 678) such that the first partial beam (TB1) and/or the second partial beam (TB2) is spatially selected.

8. FT spectrometer according to claim 7, wherein at least one field of view discriminator unit is integrated into one of the mirror surfaces of the first beam deflection unit (640; 641; 642; 643; 644; 652; 653; 662; 678) and/or the second beam deflection unit (640; 641; 642; 643; 644; 652; 653; 662; 678).

9. FT spectrometer according to claim 7 or 8, wherein the Michelson-type interferometer (601, 602, 603, 604, 605, 606, 607) further comprises at least one first and one second field of view discriminator unit (BFD1, BFD2, 633, 634, 635, 638, 654, 655) arranged downstream of the beam splitter unit (57; 571; 621; 622; 623; 624; 627) in the light direction downstream of the beam splitter unit (57; 571; 621; 622; 623; 624; 627) and a second field of view discriminator unit (BFD1, BFD2, 633, 634, 635, 638, 654, 655), arranged such that the first partial beam (TB 1) and the second partial beam (TB2) are spatially selected and that the first field of view discriminator unit (BFD1, BFD2, 633, 634, 635, 638, 654, 655) is optically conjugated in relation to the second field of view discriminator unit (BFD1, BFD2, 633, 634, 635, 638, 654, 655).

10. FT spectrometer according to any one of claims 7 to 9, wherein the field of view discriminator unit has at least one of the following:
a gap-shaped shading aperture (666),
a micro-gap shading aperture array,
a pinhole shading aperture (794, 797),
a one-dimensional or a two-dimensional pinhole shading aperture array (795) in the form of an aperture disk,
a micro-gap shading aperture array with a plurality of micro gaps in a laterally shifted arrangement,
a micro-gap shading aperture array with mechanically movable elements,
a reflective gap-shaped region that represents a part of the first and/or second beam deflection unit (640; 641; 642; 643; 644; 652; 653; 662; 678).

11. FT spectrometer according to any one of the preceding claims, wherein at least one of the first beam deflection unit (640; 641; 642; 643; 644; 652; 653; 662; 678) and the second beam deflection unit (640; 641; 642; 643; 644; 652; 653; 662; 678) has a prism with at least one reflection surface designed to reflect the first partial beam (TB 1) and/or the second partial beam (TB2) at least once.

12. FT spectrometer according to any one of the preceding claims, further comprising a confocal arrangement (751) arranged upstream of the Michelson-type interferometer (609),
wherein the confocal arrangement (751) has a rigid aperture disk (794) or a rotating aperture disk (797) and/or a spatial light modulator in reflection or transmission.

13. FT spectrometer according to any one of the preceding claims, wherein the beam splitter unit (57; 571; 621; 622; 623; 624; 627) represents an amplitude beam splitter, and has a planar beam splitter layer (62, 625, 628), a mylar foil (623), or a lattice.

14. Method for interferometric measurement using an FT spectrometer with a Michelson-type interferometer (601,602,603,604,605,606,607,608,609), comprising:
splitting an incident light beam transmitted from a spatially expanded object into a first partial beam and a second partial beam using a beam splitter unit (57; 571; 621; 622; 623; 624; 627);
at least a one-time deflection of the first partial beam using a first beam deflection unit (640; 641; 642; 643; 644; 652; 653; 662; 678);
at least a one-time deflection of the second partial beam using a second beam deflection unit (640; 641; 642; 643; 644; 652; 653; 662; 678); wherein the first partial beam (TB1) and/or the second partial beam (TB2) is deflected (2n+1) times on at least either of the first beam deflection unit (640; 641; 642; 643; 644; 652; 653; 662; 678) and the second beam deflection unit (640; 641; 642; 643; 644; 652; 653; 662; 678), using a (2n+1) periscope group with (2n+1) mirror surfaces in order to generate a lateral shear (s) between the first partial beam (TB 1) and the second partial beam (TB2), and wherein n is a natural number ≥ 1;
sending the incident light beam through a lens prior to the splitting to generate a plurality of coherent image points of the spatially expanded object in an image plane (BEI1, BEI2) between the beam splitter unit (57; 571; 621; 622; 623; 624; 627) and a detector;
spatially at least partially overlaying the first partial beam and the second partial beam using the beam splitter unit (57; 571; 621; 622; 623; 624; 627);
at least partially rendering the plurality of coherent image points while at the same time generating a plurality of spatial interferograms on a detector field of the detector on the basis of the spatial overlay;
recording the plurality of spatial interferograms using the detector; and
Fourier transforming the plurality of spatial interferograms to generate a plurality of spectra, and based thereon generating a hyperspectral image of at least a section of the spatially expanded object, wherein the method further comprises:
spatially selecting the first partial light beam (TB 1) and/or the second partial light beam (TB2) using at least one field of view discriminator unit (BFD1, BFD2, 633, 634, 635, 638, 654, 655, 666, 678-2) downstream of the beam splitter unit (57; 571; 621; 622; 623; 624; 627) in the light direction or using at least one field of view discriminator unit arranged upstream of the Michelson-type interferometer;
generating the spatial interferograms (rI1, rI2, rI3, rI4) from the selected field of view using an anamorphic lens (51, 52, 53, 537).

15. Method for interferometric measurement according to claim 14, further comprising the steps initiated and at least partially executed by at least one computer unit (21):
multiple simultaneous recording of the plurality of spatial interferograms at respectively different points in time;
Fourier transforming the plurality of spatial interferograms recorded at respectively different points in time to generate a plurality of spectra; and
generating at least one hyperspectral image of the spatially expanded object.

## Revendications

1. Spectromètre à transformée de Fourier, spectromètre FT, comprenant :
un interféromètre de type Michelson (601 ; 602 ; 603 ; 604 ; 605 ; 606 ; 607 ; 608 ; 609) comprenant :
au moins une unité séparatrice de faisceau (57 ; 571 ; 621 ; 622 ; 623 ; 624 ; 627), l'unité séparatrice de faisceau (57 ; 571 ; 621 ; 622 ; 623 ; 624 ; 627) étant conçue pour
diviser un faisceau lumineux (EB) incident d'un objet s'étendant dans l'espace en un premier faisceau partiel de rayons (TB 1) et un second faisceau partiel de rayons (TB2) ; et
au moins en partie superposer au premier faisceau partiel de rayons (TB1) et au second faisceau partiel de rayons (TB2) un décalage latéral (s) ;
une première unité de déviation de faisceau (640 ; 641 ; 642 ; 643 ; 644 ; 652 ; 653 ; 662 ; 678) qui est conçue pour dévier au moins une première fois le premier faisceau partiel de rayons (TB1) ;
une seconde unité de déviation de faisceau (640 ; 641 ; 642 ; 643 ; 644 ; 652 ; 653 ; 662 ; 678) qui est conçue pour dévier au moins une première fois le second faisceau partiel de rayons (TB2) ;
dans lequel au moins une de la première unité de déviation de faisceau (640 ; 641 ; 642 ; 643 ; 644 ; 652 ; 653 ; 662 ; 678) et de la seconde unité de déviation de faisceau (640 ; 641 ; 642 ; 643 ; 644 ; 652 ; 653 ; 662 ; 678) représente un groupe goniométrique à miroirs (2n+1) (640 ; 641 ; 642 ; 643 ; 644 ; 652 ; 653 ; 662 ; 678) avec (2n+1) surfaces-miroirs (663, 664, 665) et toutes les (2n+1) surfaces-miroirs sont disposées perpendiculairement à un plan de référence commun pour dévier respectivement (2n+1) fois le premier faisceau partiel de rayons (TB1) et/ou le second faisceau partiel de rayons (TB2) et dans lequel la déviation (2n+1) fois génère le décalage latéral (s) entre le premier faisceau partiel de rayons (TB1) et le second faisceau partiel de rayons (TB2) et dans lequel n est un entier naturel ≥ 1 ;
dans lequel le spectromètre FT comprend en outre :
au moins un objectif (70 ; 71 ; 73 ; 752 ; 78 ; 781), qui est disposé devant l'unité séparatrice de faisceau (57 ; 571 ; 621 ; 622 ; 623 ; 624 ; 627) de telle manière que le faisceau lumineux (EB) incident passe par l'objectif et est focalisé au moins partiellement avant sa division au niveau de l'unité séparatrice de faisceau (57 ; 571 ; 621 ; 622 ; 623 ; 624 ; 627) et le premier faisceau partiel de rayons (TB1) et le second faisceau partiel de rayons (TB2) génèrent respectivement une pluralité de points d'image cohérents (O'1, O'2) de l'objet s'étendant dans l'espace dans un plan d'image (BEI1, BEI2) situé, dans la direction de la lumière, après l'unité séparatrice de faisceau (57 ; 571 ; 621 ; 622 ; 623 ; 624 ; 627) et avant un détecteur (54) ;
le détecteur (54 ; 55 ; 58 ; 59) destiné à détecter une pluralité d'interférogrammes spatiaux (rI1, rI2, rI3, rI4) sur la base de la superposition spatiale du premier faisceau partiel de rayons (TB1) et du second faisceau partiel de rayons (TB2) qui correspond à la reproduction au moins partielle de la pluralité de points d'image cohérents (O'1, O'2) ; et
au moins une unité de calcul (21) pour la transformation de Fourier de la pluralité d'interférogrammes spatiaux (rI1, rI2, rI3, rI4), destinée à générer une pluralité de spectres (Sp1, Sp2, Sp3, Sp4) et, sur la base de ceux-ci, à générer au moins une partie d'au moins une image hyperspectrale de l'objet s'étendant dans l'espace,
au moins une unité discriminatrice de champ d'image (BFD1, BFD2, 633, 634, 635, 638, 654, 655, 666, 678-2) disposée, dans la direction de la lumière, en aval de l'unité séparatrice de faisceau (57 ; 571 ; 621 ; 622 ; 623 ; 624 ; 627), qui est disposée de telle manière que le premier faisceau partiel de rayons (TB1) et/ou le second faisceau partiel de rayons (TB2) sont sélectionnés dans l'espace ; et
un objectif anamorphotique (51, 52, 53, 537) destiné à générer les interférogrammes spatiaux (rI1, rI2, rI3, rI4) à partir du champ d'image sélectionné.

2. Spectromètre FT selon la revendication 1, dans lequel le plan de référence s'étend par la normale de l'unité séparatrice de faisceau (57 ; 571 ; 621 ; 622 ; 623 ; 624 ; 627) et par l'axe optique (OAI) de l'objectif (70 ; 71 ; 73 ; 752 ; 78 ; 781) situé en amont.

3. Spectromètre FT selon la revendication 1 ou 2, dans lequel le groupe goniométrique à miroirs (2n+1) présente une forme de mâchoire ou de W.

4. Spectromètre FT selon l'une des revendications précédentes, dans lequel chacune des surfaces-miroirs est disposée de façon à réfléchir une seule fois le premier faisceau partiel de rayons (TB 1) ou le second faisceau partiel de rayons (TB2).

5. Spectromètre FT selon l'une des revendications précédentes,
dans lequel au moins une de la première unité de déviation de faisceau (640 ; 641 ; 642 ; 643 ; 644 ; 652 ; 653 ; 662 ; 678) et de la seconde unité de déviation de faisceau (640 ; 641 ; 642 ; 643 ; 644 ; 652 ; 653 ; 662 ; 678) est conçue pour dévier une seule fois le premier faisceau partiel de rayons (TB1) ou le second faisceau partiel de rayons (TB2) par réflexion unique sur une surface-miroir de l'unité de déviation de faisceau (640 ; 641 ; 642 ; 643 ; 644 ; 652 ; 653 ; 662 ; 678) correspondante ; et/ou
dans lequel au moins une de la première unité de déviation de faisceau (640 ; 641 ; 642 ; 643 ; 644 ; 652 ; 653 ; 662 ; 678) et de la seconde unité de déviation de faisceau (640 ; 641 ; 642 ; 643 ; 644 ; 652 ; 653 ; 662 ; 678) est conçue pour dévier trois fois le premier faisceau partiel de rayons (TB 1) et/ou le second faisceau partiel de rayons (TB2) par triple réflexion sur trois surfaces-miroirs d'un groupe goniométrique à miroir triple de l'unité de déviation de faisceau (640 ; 641 ; 642 ; 643 ; 644 ; 652 ; 653 ; 662 ; 678) correspondante.

6. Spectromètre FT selon l'une des revendications précédentes, dans lequel la première unité de déviation de faisceau (640 ; 641 ; 642 ; 643 ; 644 ; 652 ; 653 ; 662 ; 678) et la seconde unité de déviation de faisceau (640 ; 641 ; 642 ; 643 ; 644 ; 652 ; 653 ; 662 ; 678) présentent ensemble un nombre de surfaces-miroirs qui correspond soit à (2n+1+1), soit à (2n₁+1+2n₂+1), où n est un entier naturel ≥ 1, n₁ est un entier naturel ≥ 1 et n₂ est un entier naturel ≥ 1.

7. Spectromètre FT selon l'une des revendications précédentes,
dans lequel, entre au moins deux des (2n+1) surfaces-miroirs du groupe goniométrique à miroirs (2n+1) de l'au moins une unité de déviation de faisceau (640 ; 641 ; 642 ; 643 ; 644 ; 652 ; 653 ; 662 ; 678), au moins une unité discriminatrice de champ d'image (BFD1, BFD2, 633, 634, 635, 638, 654, 655) est disposée de telle manière que le premier faisceau partiel de rayons (TB1) et/ou le second faisceau partiel de rayons (TB2) sont sélectionnés dans l'espace.

8. Spectromètre FT selon la revendication 7, dans lequel au moins une unité discriminatrice de champ d'image est intégrée dans une des surfaces-miroirs de la première unité de déviation de faisceau (640 ; 641 ; 642 ; 643 ; 644 ; 652 ; 653 ; 662 ; 678) et/ou de la seconde unité de déviation de faisceau (640 ; 641 ; 642 ; 643 ; 644 ; 652 ; 653 ; 662 ; 678).

9. Spectromètre FT selon la revendication 7 ou 8, dans lequel l'interféromètre de type Michelson (601, 602, 603, 604, 605, 606, 607) comprend en outre au moins une première et une seconde unité discriminatrice de champ d'image (BFD1, BFD2, 633, 634, 635, 638, 654, 655) disposées, dans la direction de la lumière, en aval de l'unité séparatrice de faisceau (57 ; 571 ; 621 ; 622 ; 623 ; 624 ; 627), qui sont disposées de telle manière que le premier faisceau partiel de rayons (TB1) et le second faisceau partiel de rayons (TB2) sont sélectionnés dans l'espace et en ce que la première unité discriminatrice de champ d'image (BFD1, BFD2, 633, 634, 635, 638, 654, 655) est optiquement conjuguée à la seconde unité discriminatrice de champ d'image (BFD1, BFD2, 633, 634, 635, 638, 654, 655).

10. Spectromètre FT selon l'une des revendications 7 à 9, dans lequel l'unité discriminatrice de champ d'image présente au moins un des éléments suivants :
un diaphragme occultant (666) en forme de fente,
un ensemble de diaphragmes occultants à microfentes,
un diaphragme occultant à sténopé (794, 797),
un ensemble de diaphragmes occultants à sténopé (795) unidimensionnel ou
bidimensionnel sous la forme d'un diaphragme circulaire,
un ensemble de diaphragmes occultants à microfentes avec une pluralité de microfentes dans une disposition décalée latéralement,
un ensemble de diaphragmes occultants à microfentes avec des éléments mobiles mécaniquement,
une zone de réflexion zone en forme de fente, qui représente une partie de la première et/ou de la seconde unité de déviation de faisceau (640 ; 641 ; 642 ; 643 ; 644 ; 652 ; 653 ; 662 ; 678).

11. Spectromère FT selon l'une des revendications précédentes, dans lequel au moins une de la première unité de déviation de faisceau (640 ; 641 ; 642 ; 643 ; 644 ; 652 ; 653 ; 662 ; 678) et de la seconde unité de déviation de faisceau (640 ; 641 ; 642 ; 643 ; 644 ; 652 ; 653 ; 662 ; 678) présente un prisme avec au moins une surface réfléchissante, qui est conçue pour réfléchir au moins une fois le premier faisceau partiel de rayons (TB1) et/ou le second faisceau partiel de rayons (TB2).

12. Spectromètre FT selon l'une des revendications précédentes, comprenant en outre un agencement confocal (751) situé en amont de l'interféromètre de type Michelson (609),
dans lequel, en option, l'agencement confocal (751) présente un diaphragme circulaire rigide (794) ou un diaphragme circulaire rotatif (797) et/ou un modulateur spatial de lumière en réflexion ou en transmission.

13. Spectromètre FT selon l'une des revendications précédentes, dans lequel l'unité séparatrice de faisceau (57 ; 571 ; 621 ; 622 ; 623 ; 624 ; 627) représente un séparateur de faisceau à division d'amplitude et présente une couche séparatrice de faisceau (62, 625, 628) plane, un film Mylar (623) ou une grille.

14. Procédé de mesure interférométrique au moyen d'un spectromètre FT doté d'un interféromètre de type Michelson (601, 602, 603, 604, 605, 606, 607, 608, 609) comprenant :
la division d'un faisceau lumineux incident, qui est émis par un objet s'étendant dans l'espace, en un premier faisceau partiel de rayons et un second faisceau partiel de rayons au moyen d'une unité séparatrice de faisceau (57 ; 571 ; 621 ; 622 ; 623 ; 624 ; 627) ;
la déviation, au moins une fois, d'un faisceau partiel de rayons au moyen d'une première unité de déviation de faisceau (640 ; 641 ; 642 ; 643 ; 644 ; 652 ; 653 ; 662 ; 678) :
la déviation, au moins une fois, du second faisceau partiel de rayons au moyen d'une seconde unité de déviation de faisceau (640 ; 641 ; 642 ; 643 ; 644 ; 652 ; 653 ; 662 ; 678) ; dans lequel le premier faisceau partiel de rayons (TB1) et/ou le second faisceau partiel de rayons (TB2) est dévié (2n+1) fois sur au moins une de la première unité de déviation de faisceau (640 ; 641 ; 642 ; 643 ; 644 ; 652 ; 653 ; 662 ; 678) et de la seconde unité de déviation de faisceau (640 ; 641 ; 642 ; 643 ; 644 ; 652 ; 653 ; 662 ; 678) au moyen d'un groupe goniométrique à miroirs (2n+1) avec (2n+1) surfaces-miroirs, afin de générer un décalage latéral (s) entre le premier faisceau partiel de rayons (TB1) et le second faisceau partiel de rayons (TB2) et dans lequel n est un entier naturel ≥ 1 ;
émission du faisceau lumineux incident à travers un objectif avant la division, pour générer une pluralité de points d'image cohérents de l'objet s'étendant dans l'espace dans un plan d'image (BEI1, BEI2) situé entre l'unité séparatrice de faisceau (57 ; 571 ; 621 ; 622 ; 623 ; 624 ; 627) et un détecteur ;
superposition spatiale, au moins partielle, du premier faisceau partiel de rayons et du second faisceau partiel de rayons au moyen de l'unité séparatrice de faisceau (57 ; 571 ; 621 ; 622 ; 623 ; 624 ; 627) ;
reproduction au moins partielle de la pluralité de points d'image cohérents avec génération simultanée d'une pluralité d'interférogrammes spatiaux sur un champ de détection du détecteur sur la base de la superposition spatiale ;
détection de la pluralité d'interférogrammes spatiaux au moyen du détecteur ; et transformation de Fourier de la pluralité d'interférogrammes spatiaux pour générer une pluralité de spectres et pour, sur la base de ceux-ci, générer une image hyperspectrale d'au moins un détail de l'objet s'étendant dans l'espace, le procédé comprenant en outre :
la sélection dans l'espace du premier faisceau partiel de rayons (TB1) et/ou du second faisceau partiel de rayons (TB2) au moyen d'au moins une unité discriminatrice de champ d'image (BFD1, BFD2, 633, 634, 635, 638, 654, 655, 666, 678-2) disposée, dans la direction de la lumière, en aval de l'unité séparatrice de faisceau (57 ; 571 ; 621 ; 622 ; 623 ; 624 ; 627) ou au moyen d'au moins une unité discriminatrice de champ d'image disposée en amont de l'interféromètre de type Michelson ; et
génération des interférogrammes spatiaux (rI1, rI2, rI3, rI4) à partir du champ d'image sélectionné au moyen d'un objectif anamorphotique (51, 52, 53, 537).

15. Procédé de mesure interférométrique selon la revendication 14, comprenant en outre les étapes, déclenchées et au moins en partie exécutées au moyen d'au moins une unité de calcul (21) :
détection simultanée à plusieurs reprises de la pluralité d'interférogrammes spatiaux à des moments respectivement différents ;
transformation de Fourier de la pluralité d'interférogrammes spatiaux détectés à des moments respectivement différents pour générer une pluralité de spectres ; et
génération d'au moins une image hyperspectrale de l'objet s'étendant dans l'espace.
